(19) Europäisches Patentamt
European Patent Office
Office européen des brevets

(11) **EP 4 521 334 A1**

(12) **EUROPEAN PATENT APPLICATION**
published in accordance with Art. 153(4) EPC

(43) Date of publication:
**12.03.2025 Bulletin 2025/11**

(21) Application number: **23819430.2**

(22) Date of filing: **09.02.2023**

(51) International Patent Classification (IPC):
*G06Q 50/10* (2012.01)

(52) Cooperative Patent Classification (CPC):
**G06Q 10/30; B09B 3/65; C02F 11/04; C12M 21/04;
C12M 41/48; G06Q 10/0631; G06Q 50/06;
G06Q 50/10; G06Q 50/26;** B09B 2101/70;
C12P 5/023; Y02E 50/30

(86) International application number:
**PCT/JP2023/004304**

(87) International publication number:
**WO 2023/238442 (14.12.2023 Gazette 2023/50)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **TBM Co., Ltd.**
**Tokorozawa-shi Saitama, 359-1164 (JP)**

(72) Inventors:
• **SAHARA, Kunihiro**
**Kodaira-shi, Tokyo 187-0003 (JP)**
• **SAHARA, Hiroki**
**Tokorozawa-shi, Saitama 359-1164 (JP)**

(74) Representative: **Cabinet Chaillot**
**16/20, avenue de l'Agent Sarre**
**B.P. 74**
**92703 Colombes Cedex (FR)**

(54) **BIOGAS MATERIAL EVALUATION VALUE COMPUTATION DEVICE AND PROGRAM USED FOR BIOGAS MATERIAL EVALUATION VALUE COMPUTATION DEVICE**

(57) This invention pertains to an evaluation value calculation device (1) for biogas materials, comprising: an input unit (111) configured to receive input regarding a machine learning library (1L) related to biogas materials or property data pertaining to biogas materials; a machine learning model (112) that receives the machine learning library (1L) or the property data from the input unit (111); a learning process execution unit (101) that trains the machine learning model (112) using the machine learning library (1L); and an evaluation value calculation execution unit (102) that estimates evaluation values related to resource recovery of biogas materials by performing estimation processes based on the property data using the machine learning model (112). This configuration enables optimal matching of food waste sources, such as residual sludge and food loss, with biogas plants that conduct methane gasification using food waste, thereby improving the operational efficiency of biogas plants.

Fig.11

**Description**

**Field of the Invention**

This invention relates to an evaluation value calculation device for biogas materials, wherein the device is to match a source of food waste such as restaurants and food factories (biogas materials) with a biogas plant that performs methane fermentation and methane gasification using food waste.

**Background Art**

Recently the problem of Global warming has become more and more serious. Efforts have been made to reduce the emission of greenhouse gas such as carbon dioxide gas and methane gas. The Japanese Ministry of the Environment has set a goal of achieving carbon neutrality, where CO2 (carbon dioxide) emissions will be reduced to practically zero by 2050, in order to achieve a decarbonized society (for example, refer to Non-Patent Document 1).

There are various ways to reduce CO2, such as the introduction of renewable energy equipment like solar and wind power generation, the introduction of energy-saving products such as heat pumps, and forest management activities. Recycling biomass (recycling food waste) is also thought to be one of them.

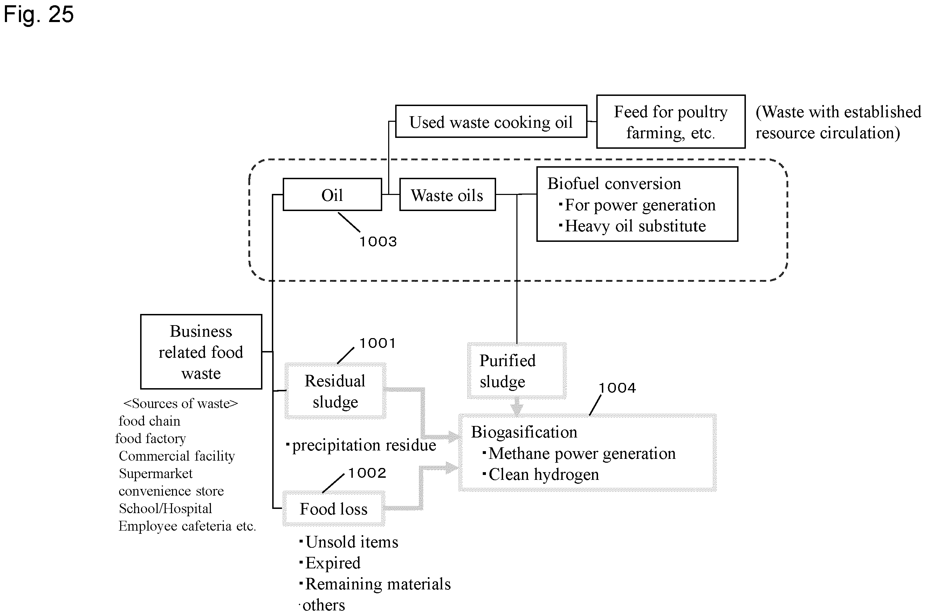

The primary sources of food waste are households and businesses. Food waste generated by businesses is classified as business waste and treated as industrial waste. Figure 25 illustrates the classification of business-related food waste into three categories: oil (1001), residual sludge (1002), and food loss (1003). Among these, "oil (1001)"-also referred to as "wastewater oil"-is a biomass resource discharged from sources such as restaurants and food factories. In Japan alone, this waste exceeds 300,000 tons annually, with significantly larger quantities observed on a global scale.

A biomass generation system has been developed to convert waste oil, previously treated solely as industrial sludge, into original biomass fuels using a diesel generator. This system represents a significant advancement by enabling CO2 reduction, recycling, and water purification. Relevant technologies and applications are detailed in Patent Documents 1 to 3.

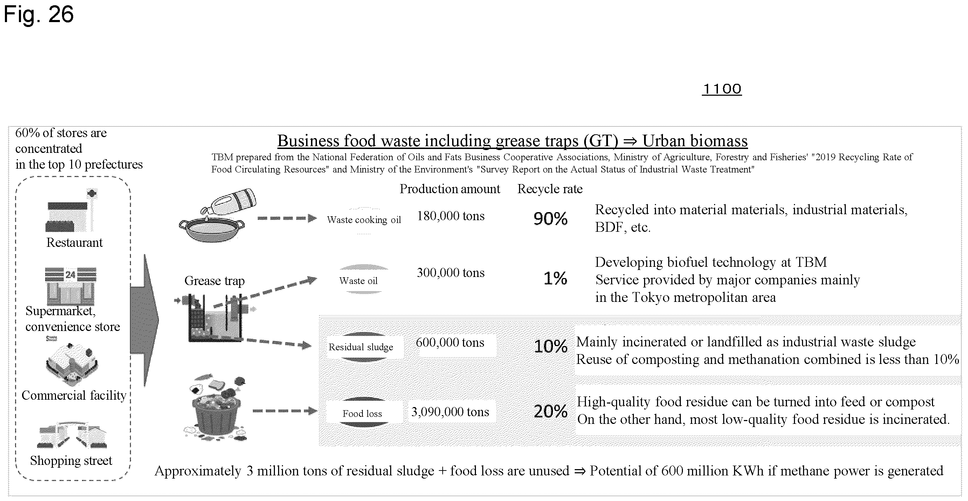

On the other hand, challenges persist in promoting the recycling of "residual sludge (1002)" and "food loss (1003)" among business-related food waste. Table 1100 in FIG. 26 highlights that approximately 3 million tons of residual sludge and food loss remain unutilized annually across Japan. However, these materials hold significant potential; if used for methane-based power generation, they could produce approximately 600 million kWh of electricity per year.

Currently, major food and beverage chains such as McDonald's® and Kentucky Fried Chicken® dispose of these food wastes as industrial waste, leaving them untapped as resources for energy generation.

**Citation List**

**Patent Document**

[Patent document 1] Japanese Unexamined Patent Application Publication No. 2014-217804
[Patent document 2] Publication of Japanese Patent Registration No.5452814
[Patent document 3] Publication of Japanese Utility Model Registration No.3216173

[Non-patent document]

[Non-patent document 1]" Achieving carbon neutrality toward 2050" [Searched on May 25, 2022], <URL: https://www.env.go.jp/earth/2050carbon_neutral.html>.

**BRIEF SUMMARY OF THE INVENTION**

**Problems that Invention is to Solve**

The initial process in converting these unused food wastes and sludge into valuable resources involves processing them into biogas (e.g., methane for power generation or green hydrogen) at a biogas plant utilizing methane fermentation. Notably, in the context of methane power generation, the amount of biogas produced per ton of raw material from business-related food waste (including residual sludge) is 5 to 10 times greater than that produced from livestock manure or sewage sludge, which have traditionally served as the primary raw materials for conventional methane gas production. Consequently, methane power generation through the treatment of food residues, either alone or in combination with livestock manure or other materials, is strongly recommended.

However, due to the unstable properties of food waste, the operational efficiency of biogas plants remains low, ranging from 38% to 49%. While there is a recognized need to recycle food waste as a resource, progress in methane utilization has been constrained by specific challenges, detailed below.

(A) First, pre-diagnosing the unstable properties of food waste is a critical step before processing it at a biogas plant. However, the cost of current services for estimating gas generation is prohibitively high, ranging from 200,000 to 500,000 yen per sample. Additionally, seasonal variations in the volume of discharged food waste and the difficulties associated with diagnosing small-lot food waste in advance present significant operational challenges. Due to these factors, biogas plants face difficulties in accurately predicting gas production. As a result, they prioritize avoiding fermentation defects and maintaining stable operations over incorporating new raw materials. This dependency on a limited range of raw materials has contributed to the low utilization rates of biogas plants.

(B) Second, conducting advanced evaluations of gas generation potential and the operational behavior of biogas plants, particularly when existing primary raw materials such as livestock manure are combined with new commercial food waste (mixed biogas materials), could significantly enhance operation management. However, the accuracy of current preliminary evaluations for mixed biogas materials remains insufficient. This shortcoming directly limits the recycling of residual sludge and food loss, thereby obstructing their effective utilization as valuable resources.

(C) Third, insufficient or imbalanced levels of nutrients (such as sulfur, nitrogen, and oil) required for methane bacteria to ferment, or a deficiency in lipase, an enzyme that facilitates the decomposition of cellulose, can result in inadequate fermentation. Consequently, the amount of gas generated may fall below expectations.

Currently, biogas plants lack the capability to determine optimal methods for promoting gasification to enhance gas generation. They are also unable to accurately identify and apply the appropriate type and quantity of gasification accelerators. This limitation contributes to reduced gas generation and, ultimately, a low capacity utilization rate.

The present application addresses the aforementioned challenges and aims to provide an evaluation value calculation device for biogas materials that promotes the active utilization of biogas resources by presenting specific evaluation values. As a result, this application resolves (A) the lack of a prior diagnostic mechanism for biogas materials and (B) and (C) the absence of evaluation values necessary for the stable operation of power plants when biogas materials are introduced, which have been obstacles to the utilization of food waste and other materials as biogas feedstocks.

**Means to Solve the Problems**

To address the aforementioned issues, the present application provides an evaluation value calculation device for biogas materials that calculates an evaluation value related to the resource recovery of biogas materials. The device includes an input unit that accepts inputs regarding a machine learning library related to biogas materials or property data concerning biogas materials, and a machine learning model that receives the machine learning library or property data from the input unit. It further comprises a learning process execution unit that trains the machine learning model based on the machine learning library and an evaluation value calculation execution unit that estimates an evaluation value related to the resource recovery of biogas materials by performing an estimation process based on the property data while utilizing the machine learning model. The property data includes at least one of the following attributes: type, collection location, property, and quantity of biomass materials.

In the evaluation value calculation device, it is preferable that data regarding the operational status of a biogas plant is input via the input unit. The data regarding the operational status of the biogas plant is then further provided to the machine learning model. The evaluation value calculation execution unit estimates an evaluation value related to the resource recovery of biogas materials by performing an estimation process based on both the property data and the data regarding the operational status of the biogas plant, utilizing the machine learning model. The data regarding the operational status of the biogas plant includes at least one of the following: the type of biogas material used at the plant, the fermentation method, the presence and amount of additives such as coagulants, the VS (Volatile Solids) volume load per 1 $m^3$ of the methane fermentation tank, the organic decomposition rate in the methane fermentation tank, the methane gas generation amount in the gas holder, and the measured methane yield (BMP).

In the evaluation value calculation device, it is preferable that the evaluation value estimated by the evaluation value calculation execution unit includes at least one of the following parameters related to mixed biogas materials, where an individual biogas material or a plurality of individual materials are combined:

(a) the C/N ratio, which represents the ratio of carbon concentration to nitrogen concentration,
(b) the lipid/VS ratio, which represents the ratio of lipid concentration to VS (Volatile Solids) concentration,
(c) the lipid/carbohydrate ratio,
(d) the protein/carbohydrate ratio,

(e) micronutrient concentrations, including iron (mg/kg-COD feedstock), cobalt (mg/g-COD feedstock), and nickel (mg/g-COD feedstock),

(f) the Na concentration, and

(g) the theoretical methane yield (ThBMP: ml/g-VS).

In the evaluation value calculation device, it is preferable that the evaluation value calculation execution unit estimates at least one of the following parameters regarding mixed biogas materials:

(a) whether the C/N ratio is 25 or more,

(b) whether the lipid/VS ratio is 35 or less,

(c) whether the lipid/carbohydrate ratio is 13 or more,

(d) whether the protein/carbohydrate ratio is 26 or more,

(e) among micronutrients, whether iron is 165 mg/kg-COD raw material or more, cobalt is 14 mg/g-COD raw material or more, and nickel is 5 mg/g-COD raw material or more,

(f) whether the Na concentration is less than 5.6 g/L, and

(g) methane yield during mixed fermentation.

In the evaluation value calculation device, it is preferable that the evaluation value calculation execution unit determines that the compatibility of mixed fermentation for mixed biogas materials improves as the methane yield in parameter (g) increases, provided that all conditions in parameters (a) to (f) are satisfied.

In the evaluation value calculation device, it is preferable that the evaluation value calculation execution unit determines that a combination is unsuitable for mixed fermentation of biogas materials if the methane yield is less than 100 mL/g-VS.

In the evaluation value calculation device, it is preferable for the evaluation value calculation execution unit to additionally determine the following conditions:

(a) a C/N ratio between 25 and 27;

(b) a lipid/VS ratio of 35 or less, while simultaneously meeting

(c) a lipid/carbohydrate ratio of 13 or more; and

(d) a protein/carbohydrate ratio of 26 or higher.

n the evaluation value calculation device, it is preferable that the evaluation value calculation execution unit further predicts and analyzes the appropriate types and quantities of gasification promoters, nutritional supplements, and auxiliary raw materials.

In the evaluation value calculation device, it is preferable that the evaluation value calculation execution unit further estimates the following as the evaluation value: whether pretreatment of each biogas material is necessary; the organic substance composition of each methane material; fermentation temperature and duration; organic substance concentration (VS) of each methane material; solid substance amount (TS); organic substance ratio (VS/TS); ash concentration; chemical oxygen demand (COD); theoretical oxygen demand (ThOD); COD/VS value; organic substance constituent element concentrations of each methane material, including the content of carbon (C:%VS), nitrogen (N:%VS), oxygen (O:%VS), and hydrogen (H:%VS); and the hemicellulose, cellulose, and lignin concentrations (%VS) of each methane material.

In the evaluation value calculation device, it is preferable that the machine learning library includes at least one of the following: whether pretreatment of individual biogas materials and mixed biogas materials is necessary; the organic substance composition of each methane material; fermentation temperature and duration; organic substance concentration (VS); solid substance amount (TS); organic substance ratio (VS/TS); ash concentration; chemical oxygen demand (COD); theoretical oxygen demand (ThOD); COD/VS value; the concentrations of organic substance constituent elements in each methane material, including carbon (C:%VS), nitrogen (N:%VS), oxygen (O:%VS), and hydrogen (H:%VS); the concentrations of nutrients such as carbohydrates, lipids, proteins, and ash in each methane material; the concentrations of hemicellulose, cellulose, and lignin (%VS) in each methane material; the C/N ratio (ratio of carbon concentration to nitrogen concentration in each methane material); the lipid/VS ratio (ratio of lipid concentration to VS concentration in each methane material); Na concentration; the concentrations of micronutrients, including iron (mg/kg-COD raw material), cobalt (mg/g-COD raw material), and nickel (mg/g-COD raw material) when materials are mixed; theoretical methane yield (ThBMP: ml/g-VS); actual methane yield (BMP: ml/g-VS); predicted values of biodegradation rate, VS decomposition rate, and energy yield.

In the evaluation value calculation device, it is preferable to further include a communication unit that enables a connection to a communication network, wherein the evaluation value related to the resource recovery of biogas materials, estimated by the machine learning library and the evaluation value calculation execution unit, is transmitted via the communication unit.

To address the aforementioned challenges, a program for an evaluation value calculation device is provided, which calculates an evaluation value related to the resource recovery of biogas materials. The program includes an input step, which involves receiving inputs regarding a machine learning library related to biogas materials or property data concerning biogas materials and inputting the machine learning library or property data into a machine learning model. It also includes a learning process execution step, which trains the machine learning model using the machine learning library to determine setting values. Furthermore, the program includes an evaluation value calculation execution step, which estimates an evaluation value related to the resource recovery of biogas materials by performing an estimation process based on the property data while utilizing the machine learning model. The property data includes at least one of the following attributes: type, collection location, property, and quantity of biomass materials.

In this program, it is preferable that data regarding the operational status of a biogas plant is input during the input step, and that the data regarding the operational status of the biogas plant is provided to the machine learning model. The evaluation value related to the resource recovery of biogas materials is then estimated by performing an estimation process based on the property data and the operational status data of the biogas plant, utilizing the machine learning model during the evaluation value calculation execution step. The data regarding the operational status of the biogas plant includes at least one of the following: the type of biogas material used at the plant, the fermentation method, the presence and amount of additives such as coagulants, the VS volume load per 1 $m^3$ of the methane fermentation tank, the organic decomposition rate in the methane fermentation tank, the methane gas generation amount in a gas holder, and the measured methane yield (BMP).

To address the aforementioned challenges, an evaluation value calculation method is provided for calculating an evaluation value related to the resource recovery of biogas materials. The method includes an input step for receiving inputs regarding a machine learning library related to biogas materials or property data concerning biogas materials and for inputting the machine learning library or property data into a machine learning model. It also includes a learning process execution step for training the machine learning model based on the machine learning library to determine setting values, and an evaluation value calculation execution step for estimating an evaluation value related to the resource recovery of biogas materials by performing an estimation process based on the property data while utilizing the machine learning model. The property data includes at least one of the following: type, collection location, property, and quantity of biomass materials.

In the evaluation value calculation method, it is preferable that data regarding the operational status of a biogas plant is input during the input step and provided to the machine learning model. An evaluation value related to the resource recovery of biogas materials is then estimated during the evaluation value calculation execution step by performing an estimation process based on both the property data and the operational status data of the biogas plant, utilizing the machine learning model. The data regarding the operational status of the biogas plant includes at least one of the following: the type of biogas material used at the plant, the fermentation method, the presence and amount of additives such as coagulants, the VS volume load per 1 $m^3$ of a methane fermentation tank, the organic decomposition rate in the methane fermentation tank, the methane gas generation amount in a gas holder, and the measured methane yield (BMP).

## Effects of the Invention

This application pertains to an evaluation value calculation device that calculates evaluation values for biogas materials. The device includes an input unit that receives inputs related to a machine learning library concerning biogas materials or property data regarding biogas materials, and a machine learning model that processes the machine learning library or property data received from the input unit. It also comprises a learning process execution unit that trains the machine learning model based on the machine learning library to determine setting values, and an evaluation value calculation execution unit that estimates evaluation values related to the resource recovery of biogas materials by performing estimation processes based on the property data while utilizing the machine learning model. The property data includes at least one of the following attributes: type, collection location, property, and quantity of biomass materials. With this configuration, the evaluation value calculation device for biogas materials enables the optimal matching of food waste sources, such as residual sludge and food loss, with biogas plants that perform methane gasification using food waste. This improves the operational efficiency of biogas plants and contributes to the realization of a decarbonized society in the long term.

## Brief description of Drawings

FIG.1: An overall process diagram illustrating a core system, including a methane resource matching system, according to an embodiment of the present invention.

FIG.2: A comprehensive configuration diagram of the methane resource matching system.

FIG.3: An explanatory diagram of a program utilized in the methane resource matching system.

FIG.4: A diagram illustrating an actual example of biogasification promotion employing a gasification promoter.

FIG.5: A flowchart depicting the overall process flow of the program used by the evaluation value calculation device.

FIG.6: A diagram explaining the operational flow of the methane resource matching system and the evaluation value calculation device.

FIG.7: A diagram explaining the overall process flow of the methane resource recovery evaluation AI within the AI methane resource recovery program used by the evaluation value calculation device.

FIG.8: A continuation diagram detailing the overall process flow of the methane resource recovery evaluation AI within the AI methane resource recovery program used by the evaluation value calculation device.

FIG.9: A diagram illustrating the overall flow of the methane yield promotion prediction AI in the AI gasification promotion program employed by the evaluation value calculation device.

FIG.10: A functional block diagram of the evaluation value calculation device.

FIG.11: A second functional block diagram of the evaluation value calculation device.

FIG.12: A flowchart outlining the operational procedures during the learning phase of the evaluation value calculation device.

FIG.13: A flowchart outlining the operational procedures during the estimation phase of the evaluation value calculation device.

FIG.14: A diagram showing a machine learning library associated with an experimental example of the present invention.

FIG.15: A continuation diagram showing a machine learning library associated with the experimental example.

FIG.16: A diagram presenting the estimated results of the evaluation value derived from a machine learning model for an individual biogas material in the experimental example.

FIG.17: A diagram presenting additional estimated results of the evaluation value in the experimental example.

FIG.18a: A diagram presenting the estimated results of the evaluation value.

FIG.18b: A diagram presenting measured values of the evaluation value.

FIG.19: A diagram presenting the estimated results of the evaluation value derived from a machine learning model for mixed biogas materials in the experimental example.

FIG.20: A diagram presenting further estimated results of the evaluation value.

FIG.21: A diagram presenting additional estimated results of the evaluation value.

FIG.22: A diagram presenting the estimated results of the evaluation value derived from a machine learning model for mixed biogas materials and auxiliary agents in the experimental example.

FIG.23: A diagram presenting further estimated results of the evaluation value.

FIG.24: A diagram presenting additional estimated results of the evaluation value.

FIG.25: A diagram classifying business-related food waste.

FIG.26: A diagram highlighting current issues related to residual sludge and food loss.

**Best Mode for Carrying Out the Invention**

(Embodiment)

A methane resource matching system, incorporating an evaluation value calculation device for biogas materials according to an embodiment of the present application, is described with reference to the accompanying figures. This methane resource matching system forms part of a decarbonization resource recovery service that supplies optimal biogas materials, such as food waste, from sources including restaurant chains and other contributors, based on the operational status of biogas plant operators.

In this embodiment, biogas materials include food waste, residual sludge (including refined sludge), food loss, and livestock waste, but exclude wastewater oil. Sources of biogas materials include, but are not limited to, restaurant chains, food factories, commercial facilities, supermarkets, convenience stores, hotels, schools, hospitals, and employee cafeterias.

<Overall process figure>

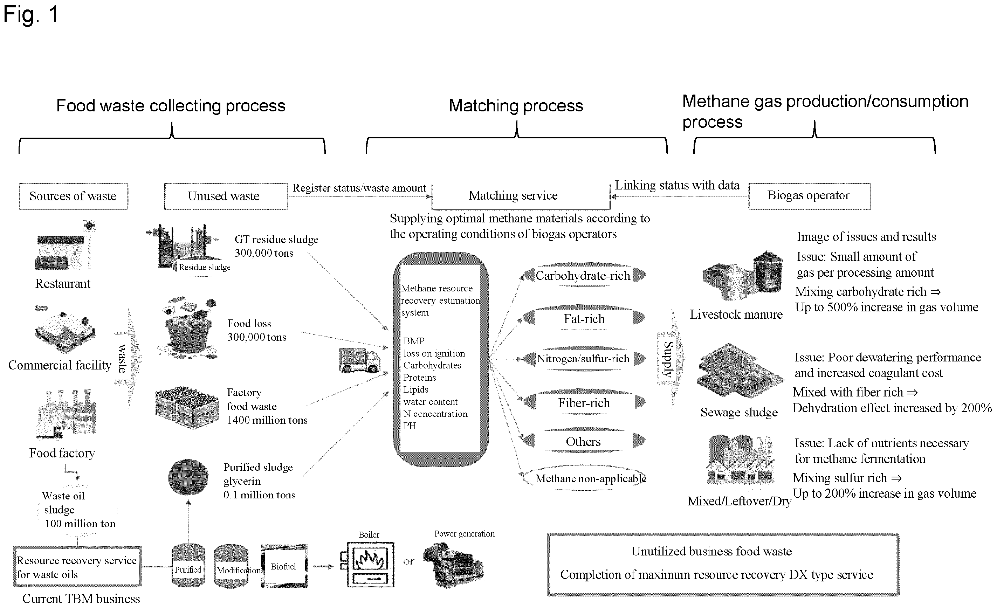

First, an overview of the overall process for a methane resource recovery service, which includes the methane resource matching system (hereinafter referred to as the "matching system"), is provided with reference to FIG. 1. As illustrated in FIG. 1, the methane resource recovery service primarily consists of a methane material collection process, a matching process, and a methane gas production and consumption process.

As the first step, in the methane material collection process, residual sludge (sedimentation residue), food loss, food waste (including lipid-based waste), and refined sludge are collected from biomass discharged by sources such as restaurants and food factories. Here, biomass refers to "renewable organic resources derived from living organisms, excluding fossil resources." Among biomass, waste biomass includes discarded paper, livestock manure, food waste, and sewage sludge.

The $CO_2$ emitted during the combustion of biomass is equivalent to the $CO_2$ absorbed from the atmosphere through photosynthesis during the growth of living organisms. Therefore, replacing fossil resource-based energy and products with biomass significantly contributes to reducing $CO_2$ emissions, where $CO_2$ is one of the greenhouse gases responsible for global warming.

In general, significant amounts of food loss, including unsold products, expired items, and leftover ingredients, are generated daily from food waste sources such as food service establishments, fast food restaurants, restaurants, hotels, and food processing plants. These materials are collected as methane resources.

Additionally, wastewater from food waste sources contains various water pollutants. If such wastewater is discharged without proper treatment, substances such as oil in the wastewater can adhere to and solidify within drain pipes, causing blockages. This not only makes water purification in combined treatment tanks and sewage treatment plants more difficult but also negatively impacts the environment.

Therefore, facilities for dumping wastewater, including solid substances such as floating substances, comprise processing units (grease traps), oil/water separation tanks, and raw water tanks in order to pool wastewater for the time being or to deposit solid substances, or to float and separate oil, or to remove these temporarily for physical removal.

In the methane material collecting process, a field agent, which is, for example, a working company, manages the cleaning of grease traps at restaurants, etc., and collects waste oil and residual sludge from the grease traps. Herein, the device disclosed in patent No.4401007, the device disclosed in patent No.4420750, and the device disclosed in utility patent No.3216173, are applicable as a technology to collect biomass from a grease trap. The applicant owns these patents.

Other field agents are, for example, agents that manage oil/water separation tanks at food factories that produce box lunches, frozen foods, and meat products, and collect biomass from the oil/water separation tanks. Specifically, oil sludge obtained from the oil/water separation tank at a food factory is separated into oil, water, and sludge using an oil sludge separation apparatus, and the sludge (residual sludge) is collected. The field agent may also manage the oil sludge separation apparatus.

Next, in the matching process, food waste (biomass materials such as food loss and so on) collected from restaurants, food factories, etc. in the methane material collecting process, and a biogas plant that uses this food waste to produce methane gas, are optimally connected using IoT (Internet of Things) and AI (Artificial Intelligence) in order to improve equipment utilization rate and productivity at biogas plants.

In this matching process, for example, food waste that has been treated as waste, presents the types, mixing ratios, and quantities of food waste that can serve as an appropriate methane resource according to conditions such as requests and operational status (for example, methane resources used and power generation status) from biomass plants. Collection and supply services can be provided. This will provide food-related businesses with various values such as waste reduction, decarbonization, and cost reductions. In addition, by supplying the optimal methane materials to individual biogas plants, values such as stabilizing gas volume and fermentation, and reducing operating costs will be created. The amount of electricity generated will increase in proportion to the increase in gas production, which can contribute to a decarbonization.

Next, in the methane gas production/consumption process, the biogas plant operator produces biogas (methane gas) from the biomass (livestock manure, food waste, oil residue (including purified sludge), food loss, and so on) brought into the biogas plant after the above matching process. Said biogas is generated by fermenting livestock manure, food loss, and residual sludge in a fermentation tank, and is generally stored in a gas holder and then used for power generation. In the future, it is expected that this methane gas will be reformed and used as green hydrogen based on non-fossil fuels.

Herein, businesses, that have introduced a biofuel (methane gas) produced from food waste, are entitled to receive carbon credits. In a case when a business has a $CO_2$ reduction target, the amount more than the target will become $CO_2$ reduction credits. In a case when a small business entity does not have any $CO_2$ reduction target, all amounts will become saved as carbon credits. In Japan, said carbon credit can be sold via a J-credit system and can be sold overseas using the carbon credit trading system established by each country.

<Whole System Overview>

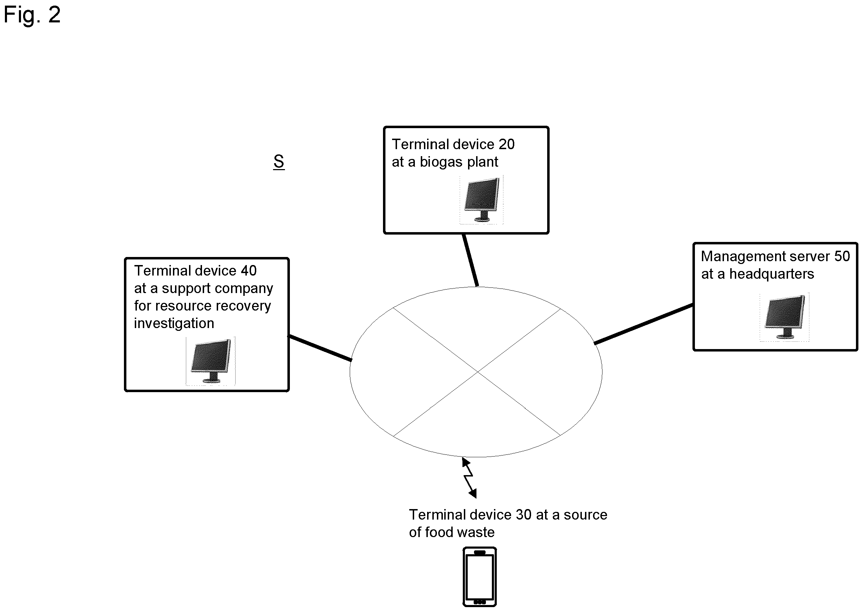

Next, the overall system of the matching system S, according to this embodiment, will be described with reference to Fig. 2. The matching system S consists of a management server (50) located at a general headquarters, a terminal device (second terminal device, 20) at a biogas plant, a terminal device (first terminal device, 30) located at sources of food waste such as restaurant chains, food factories, hotels, or commercial facilities, and a terminal device (third terminal device, 40) located at a resource investigation support company. These components are interconnected via a wide area network, such as the Internet.

The terminal device (20) is a portable device, such as a personal computer or smartphone, used at a biogas plant to process food waste into biogas (also referred to as methane gasification or methane resource recovery). The terminal device (20) is equipped with a specialized application designed for field investigation and matching purposes, allowing users to input basic data related to methane gasification. The results of the input and analysis are displayed on the device's screen.

The terminal device (20) establishes a communication session with the management server (50) using a specified protocol and facilitates the transmission and reception of data, files, and other information related to the management of methane materials, methane gas production, and methane power generation at the biogas plant. Additionally, the terminal device (20) can send requests for specific methane materials to the management server (50) in formats such as HTTP requests.

The terminal device (30) is a device, such as a smartphone, used at the site of the food waste source or at the head office of the source to input information regarding the food waste to be discharged. The terminal device (30) is equipped with a specialized application designed for source investigation and matching purposes. This application allows users to input basic matching data for methane resource recovery and displays the results on the device's screen. The specialized application may also include a web-based visualization tool for managing the collection of food waste to be discharged.

The terminal device (30) establishes a communication session with the terminal device (40) at the support company and the management server (50) using a predetermined protocol. It facilitates the transmission and reception of photos of food waste, as well as data and files related to the management of food waste and wastewater oils and fats.

The terminal device (40) is utilized at a support company responsible for investigating and collecting food waste. It is a device, such as a smartphone or personal computer, used to input information regarding food waste at its source, the raw materials used, and the operating status of biogas plants. The terminal device (40) runs a specialized application and displays the results on its screen.

Using a predetermined protocol, the terminal device (40) facilitates the transmission and reception of data and files between the terminal devices (20 and 30) and the management server (50). Additionally, the terminal device (40) acquires information about newly discharged food waste from the terminal device (30) and information requested by the biogas plant from the terminal device (20), often in the form of HTTP requests or similar formats. Based on optimal methanation material matching, the terminal device (40) efficiently matches the food waste source with the biogas plant.

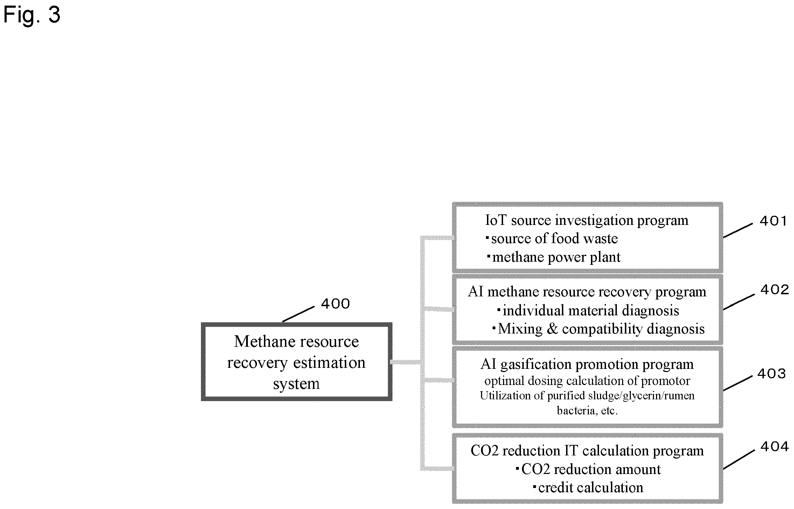

The management server (50) is located at the general headquarters responsible for overseeing the bio-gasification of food waste. It manages the bio-gasification processes at both the sources of food waste and the biogas plants. Specifically, the management server (50) obtains information about raw materials used and operating conditions at biogas plants from terminal devices (20 or 30) and about food waste discharged from terminal devices (30 or 40). Utilizing this information, the management server (50) efficiently facilitates methane resource recovery. In this process, the management server (50) centrally manages the initial matching service, resource recovery service, and CO2 reduction management service. These services are primarily executed through the four programs (401 to 404) shown in FIG. 3.

(1) IoT Source Investigation Program

The IoT source investigation program (401) identifies the type, quantity, and properties of methane materials managed by sources of food waste based on input data and photographs sent via a smartphone application or similar means from terminal devices (30 or 40). The IoT source investigation program (401) also determines the type and quantity of methane materials required by the methane gas plant based on gas production levels, power generation status, and requests sent from the biogas plant's terminal devices (20 or 30).

In other words, the management server (50), using the IoT source investigation program (401), collects and integrates data from terminal devices (20, 30, and 40) regarding source investigations, gas production levels, and power generation status at biogas plants. By consolidating data that was previously dispersed, such as food waste data from sources including food factories and gas production or power generation data from biogas plants, the management server (50) enhances the efficiency and productivity of methane gas generation and methane power generation.

Specifically, mobile devices such as smartphones (terminal devices 30, 20, and 40) are utilized at food waste sources, biogas plants, and support companies, respectively. Data from these devices can be instantly uploaded to the cloud, enabling the use of big data for further analysis without requiring physical visits to the sources.

The management server (50), using the IoT source investigation program (401), monitors and evaluates the status of food waste sources and biogas plants. As a result, the time required for processing data related to source investigations, which previously took 30 minutes per site, can be reduced to just 1 minute per site with the implementation of this program.

(2) AI Methane Resource Recovery Program

The AI methane resource recovery program (402) is used by the management server (50) and related systems to predict methane yield for individual materials and diagnose inhibition effects when multiple materials are mixed with existing raw materials. These predictions are based on past research data, newly collected analytical data, actual operational data, and other sources.

In this context, methane yield refers to the estimated amount of methane produced per unit of decomposed volatile solids (VS), which represent the organic content of food waste as a raw material. Additionally, synergy prediction refers to forecasting the synergistic effects on methane yield when different types of methane materials are mixed.

With the use of the AI methane resource recovery program (402), the management server (50) constructs a highly accurate prediction model and applies precise methane yield estimation formulas tailored for individual materials. Additionally, estimation formulas incorporating new analytical methods, nutrient factors, inhibition caused by oils and fats, and synergies resulting from raw material mixtures are developed and implemented.

This centralized approach allows the management server (50) at headquarters to efficiently manage the evaluation of energy conversion equipment and raw materials used in methane fermentation.

Specifically, in determining individual materials, methane yield and other fundamental data (actual data) are stored in a learning database for each raw material, such as food waste (including lipid-based waste), residual sludge (including refined sludge), food loss, and livestock waste used for methane fermentation. Machine learning is then performed through mathematical calculations and corrections to actual measured values, resulting in the creation of big data.

The implementation of the AI methane resource recovery program (402) enables highly accurate estimation of the potential for methane resource recovery for both individual materials and mixed materials. As a result, the basic determination of methane resource recovery potential, which previously required 180 days per raw material, can now be completed in just 2 seconds per raw material using the AI methane resource recovery program (402). Details regarding the AI methane resource recovery program (402) will be provided later in this document.

(3) AI Gasification Promotion Program

The management server (50), through the implementation of the AI gasification promotion program (403), identifies the optimal gasification promotion method and forecasts the appropriate input amounts and expected effects of gasification promoters, including nitrogen, sulfur, oils and fats, and lipase. These processes aim to resolve estimated inhibiting factors and optimize the utilization of materials with low methane yield.

Specifically, the AI gasification promotion program (403) is utilized to identify the optimal gasification promotion method based on the type, quantity, and properties of food waste. The program calculates the raw material mixing ratio and determines the optimal dosage of accelerators, such as purified sludge, glycerin, and rumen bacteria. Test data for each mixing ratio, fundamental data for each gasification accelerator candidate, and additional data from administration tests are stored in the AI learning database.

Machine learning and deep learning are then performed, incorporating mathematical calculations and corrections to actual measured values, while simultaneously generating big data. As a result, the optimal gasification promotion method, raw material mixing ratio, and dosage for each gasification accelerator can be determined with high precision. Previously, these processes required 180 days, but with the implementation of AI, they can now be completed in just 10 seconds per raw material.

Gasification promoters primarily consist of auxiliary raw materials, nutritional supplements, and accelerators. Among these, auxiliary raw materials do not promote the decomposition of other raw materials. However, because auxiliary raw materials exhibit a high methane yield per unit weight, mixing them with other raw materials can increase the apparent methane yield per unit of input material.

It should be noted, however, that auxiliary raw materials are generally difficult to process through methane fermentation when used as a sole raw material. Examples of auxiliary raw materials include refinery sludge from grease trap oil and biodiesel fuel (BDF) residue glycerin. Methane gasification can be significantly enhanced when the mixing ratios of auxiliary raw materials and food waste are properly optimized.

Nutritional supplements, including nickel (Ni), cobalt (Co), and iron (Fe), are essential for the metabolism of methanogens but are typically insufficient in most raw materials. The addition of these supplements enhances nutrient availability and accelerates the fermentation process, particularly in food waste.

For example, nutritional supplements may include compounds such as $NiCl_2 \cdot 6H_2O$, $CoCl_2 \cdot 6H_2O$, and $FeCl_2 \cdot 4H_2O$, which are already available in commercial nutrient solutions. However, the optimal amount of these supplements depends on specific conditions, and their effective application remains a challenge in current practices.

Some studies report that promoters are effective in decomposing cellulose. These promoters have the potential to enhance methane yields from cellulose-rich agricultural residues, such as resource crops like rice straw, rice husks, and dent corn. However, promoters may not significantly increase gas production from food-based waste.

Furthermore, conducting the enzyme reaction requires separate reaction tank equipment, with a reaction time ranging from several hours to one day. Examples of promoters include cellulase enzymes and rumen fluid.

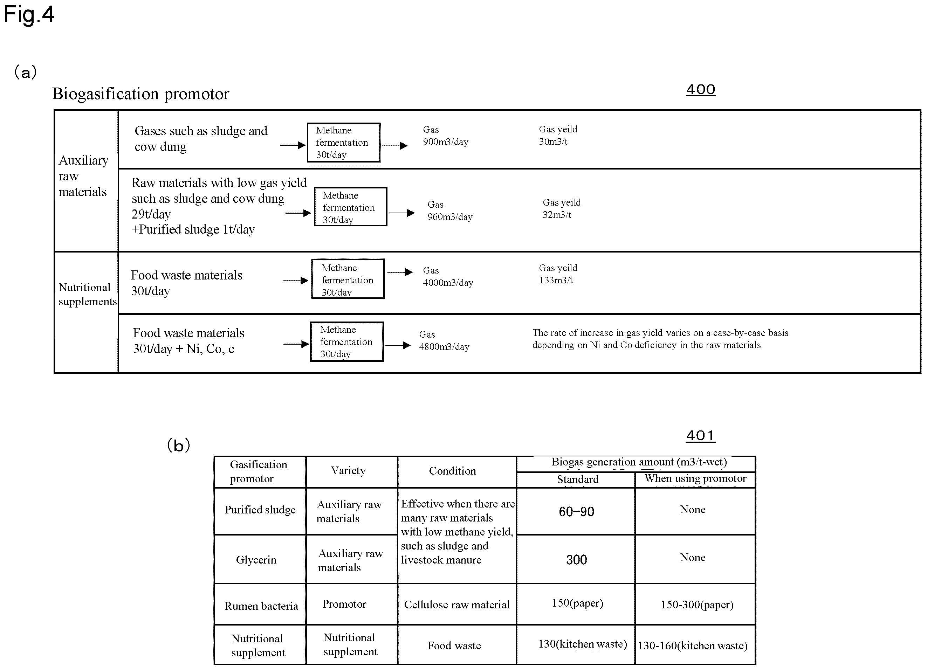

As illustrated in Table 400 of FIG. 4(a), an actual example of biogasification promotion using gasification promoters demonstrates that gas yield was improved by adding auxiliary raw materials (in this case, purified sludge) and nutritional supplements (Ni, Co, and Fe). Similarly, as shown in Table 401 of FIG. 4(b), the addition of a gasification promoter resulted in an increase in the amount of biogas generated.

After machine learning, the AI gasification promotion program (403) determines the optimal gasification promotion method based on the type, quantity, and properties of the food waste. It also calculates the raw material mixing ratio and automatically determines both the type and optimal input amount of the gasification promoter.

(4) CO2 reduction IT calculation program

The management server (50), through the implementation of the CO2 reduction IT calculation program (404), estimates the amount of methane gas generated and the corresponding CO2 reduction. Additionally, the program calculates the CO2 reduction volume and the amount that can be converted into carbon credits based on the actual utilization of biogas at biogas plants.

The management server (50) centrally manages data (via a cloud platform) related to the collection volumes of "residual sludge (including refined sludge)" and "food loss" from food waste sources, the methane gas generated from these raw materials, the electricity produced and CO2 reduced using methane gas, as well as the green hydrogen production volumes and associated CO2 reduction levels.

This series of programs, spanning from the IoT source investigation program (401) to the CO2 reduction IT calculation program (404), is collectively referred to as the methane resource recovery estimation system (400). User convenience is further enhanced by integrating these functions into a graphical user interface (GUI) accessible via a smartphone application.

The essential starting point for converting unused residual sludge and food loss into valuable resources is biogasification. In the context of biogasifying these unused materials, achieving optimal energy conversion equipment selection and maintaining a high utilization rate requires highly accurate methane yield predictions, as well as the prediction of inhibition and synergies among methane fermentation raw materials.

To address these needs, the methane resource recovery estimation system (400) leverages advanced AI and IoT technologies to scientifically and systematically integrate various independent data sources. By employing deep learning, the system enhances the accuracy of AI-driven predictions. Consequently, the methane resource recovery estimation system (400), as a digital transformation service in the resource recovery field, delivers new value to food waste sources and biogas plants.

Moreover, this system maximizes the utilization of food waste as a decarbonization resource, contributing significantly to the realization of carbon neutrality by 2050, both domestically and internationally.

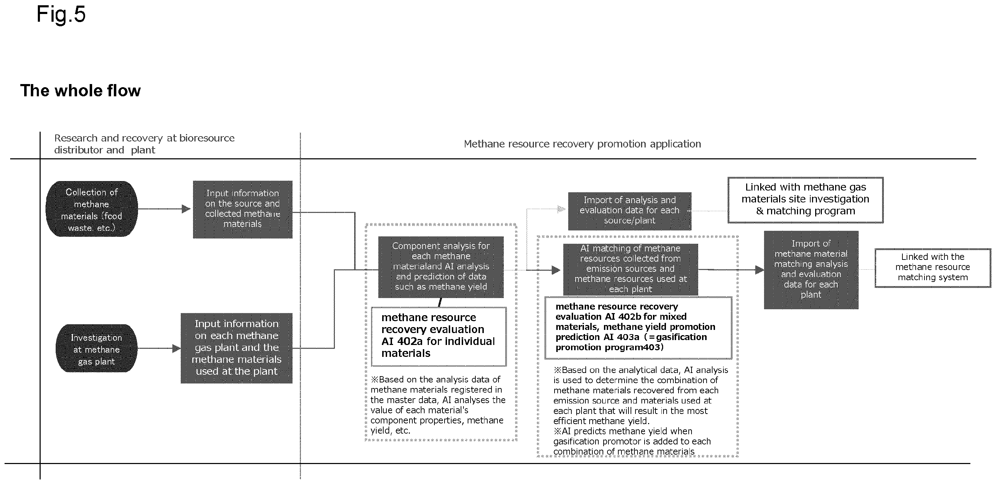

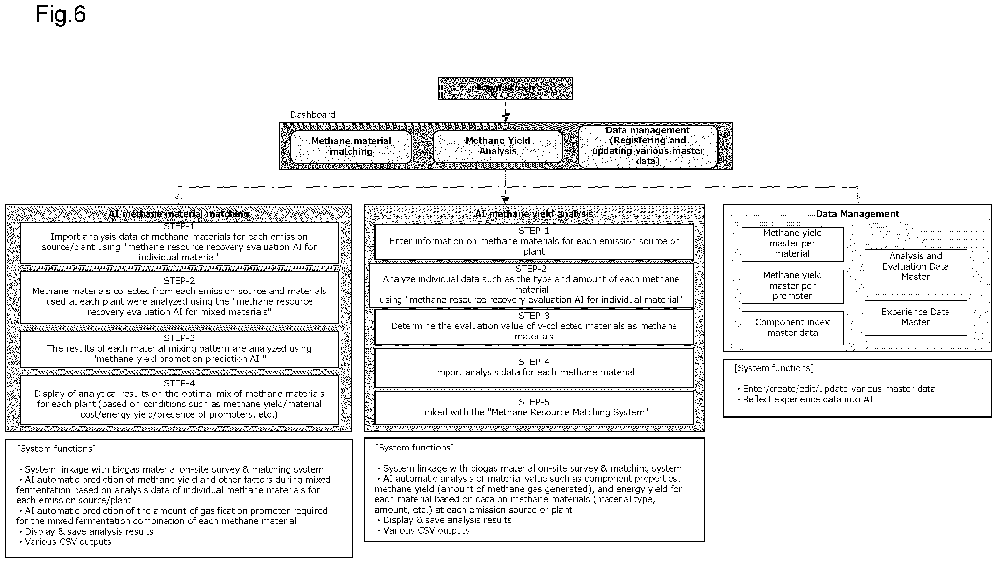

Subsequently, the specific details of the "AI Methane Resource Recovery Program (402)" and the "AI Gasification Promotion Program (403)" executed by the evaluation value calculation device for biogas materials in this embodiment will be explained with reference to Figs. 5 and 6.

The AI Methane Recovery Program (402) establishes a connection between food waste sources, including commercial facilities, restaurants, hotels, and food factories, and biogas (methane gas) plant operators. This connection is based on analysis results evaluating the compatibility of mixed fermentation between "biogas materials (food waste)" discharged from these sources and "biogas materials currently utilized in the plant" (e.g., food waste, oils and fats, sludge, livestock waste).

Through this approach, the AI Methane Recovery Program (402) aims to enhance the efficiency of biogas power generation or clean hydrogen production, while simultaneously improving the recycling rate of food waste.

The AI Gasification Promotion Program (403) predicts and analyzes the appropriate types and quantities of gasification promoters, nutritional supplements, auxiliary raw materials, and other additives. These predictions are based on analysis and evaluation data regarding the compatibility of mixed fermentation for each biogas material, as determined by the AI Methane Resource Recovery Program (402).

Building on these insights, the AI Gasification Promotion Program (403) is designed to establish optimal fermentation conditions when one or more stored biogas materials are combined and fermented.

The assumed functions of the AI Methane Resource Recovery Program (402) and the AI Gasification Promotion Program (403) are described below. As shown in FIG. 5, the AI Methane Resource Recovery Program (402) consists of two AIs: Methane Resource Recovery Evaluation AI for Individual Materials (402a) and Methane Resource Recovery Evaluation AI for Mixed Materials (402b). The basic functions of each AI are as follows.

Methane Resource Recovery Evaluation AI (402a) for Individual Materials: This AI analyzes the value of each material as a biogas resource. The analysis is based on factors such as the component properties of each material, methane yield (volume of methane gas produced), and energy yield. The evaluation covers biogas materials collected from various sources and those currently in use at each plant.

Methane Resource Recovery Evaluation AI (402b) for Mixed Materials: This AI predicts and analyzes combinations of mixed fermentation processes to identify those that achieve the highest methane yield and energy yield. The evaluation considers biogas materials collected from various sources as well as those currently utilized at each plant.

The AI Gasification Promotion Program (403) consists of the Methane Yield Promotion Prediction AI (403a), as illustrated in FIG. 5, and performs the following core functions:

Methane Yield Promotion Prediction AI (403a): This AI predicts and analyzes the types and amounts of gasification promoters required to adjust numerical indicators of analysis items to within their normal ranges. These analysis items are used to measure the value of individual materials (or their combinations) as biogas resources, as predicted and analyzed by the two AIs (402a and 402b). Gasification promoters include auxiliary raw materials (such as oil and fat refining sludge and biodiesel fuel (BDF) residue glycerin), promoters (such as cellulase enzymes, rumen fluid, and biochar), and nutritional supplements (such as $NiCl_2 \cdot 6H_2O$, $CoCl_2 \cdot 6H_2O$, and $FeCl_2 \cdot 4H_2O$).

All three AIs 402a, 402b, and 403a can be integrated into each device that constitutes the "methane resource matching system S" described above. These AIs provide specialized data related to resource recovery in an accessible and user-friendly format, tailored to the needs of users at both food waste sources and biogas plant operators. The data is presented with clear priorities and display formats, such as rankings based on methane yield, energy yield, CO2 emission reductions, material costs, and graphical visualizations (see FIG. 6 for an example).

This approach addresses the previously unresolved issue of the lack of pre-diagnosis of feedstocks and the absence of component index data necessary for stable plant operation when accepting food waste as a methane feedstock. As a result, the system facilitates the promotion of biogas resource recovery from food waste.

(2-1) Details of the Methane Resource Recovery Evaluation AI for Individual Materials

The Methane Resource Recovery Evaluation AI 402a for individual materials is one of the AIs constituting the AI Methane Resource Recovery Program 402, as described above. This AI analyzes biogas materials collected from food waste sources-such as commercial facilities, restaurants, hotels, and food factories-as well as biogas materials currently in use at biogas plants, including food waste, oils and fats, sludge, and livestock waste.

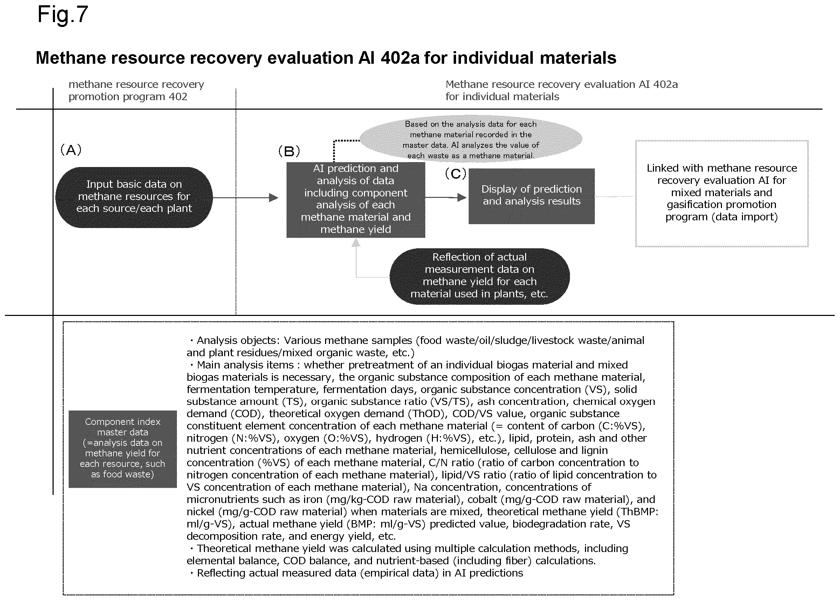

The Methane Resource Recovery Evaluation AI 402a predicts and evaluates the value of each material as a methane resource by referencing the component index master data, as illustrated in FIG. 7. The results of these predictions and analyses are not only presented to operators at the sources of the materials and at the biogas plants but are also integrated into the "(2-2) Methane Resource Recovery Evaluation AI for Mixed Materials" and "(3-1) Methane Yield Promotion Prediction AI," which are discussed later.

These results serve as reference data for compatibility analyses of mixed fermentation for each biogas material and for predicting and analyzing synergies or potential inhibition.

The assumed functions (flow) of the Methane Resource Recovery Evaluation AI 402a for individual materials are described with reference to FIG. 7.

(A) This program facilitates the registration and validation of data into the AI Methane Resource Recovery Program 402. The data includes information on biogas materials-such as food waste collected from various sources and biogas materials currently in use at biogas plants, including food waste, oils and fats, sludge, and livestock waste-as well as data on the operational status of biogas plants.

Specific examples of data on collected food waste and biogas materials currently utilized in plants include information about the characteristics of each biogas material. This data encompasses attributes such as "collection location," "type," "properties" (e.g., liquid, solid, gas, processed or unprocessed), "quantity," and inventory status. This information can be managed in real-time using technologies such as blockchain.

Additionally, data on the operating status of biogas plants encompasses various parameters. These include the type of biogas materials utilized in each plant, the presence, absence, and quantity of additives such as coagulants, the temperature of raw material storage tanks, the amount of heat required for methane fermentation tank heating, fermentation tank heating efficiency and heat loss rate, the maximum organic load per cubic meter of the methane fermentation tank, surplus heat, the amount and efficiency of hot water recovery, the organic decomposition rate within the methane fermentation tank, methane gas generation volume in the gas holder, measured methane yield (BMP), power generation amount (in the case of gas-based power generation), power generation efficiency, heat conversion values, power consumption within the facility, and the consumption ratio relative to power generation.

As with inventory data for biogas materials, this operational data can be managed in real time using technologies such as blockchain, ensuring seamless monitoring and optimization.

(B) Utilizing data on biogas materials registered and approved through the AI methane resource recovery program (402), the methane resource recovery evaluation AI (402a) analyzes and predicts the value of each material as a methane resource by referencing the component index master data.

The items verified during the prediction and analysis of the value of each material as a methane resource are assumed to include the following: the necessity of pretreatment for each methane material; organic composition;

fermentation temperature; fermentation duration; organic substance concentration (VS); solid substance amount (TS); organic substance ratio (VS/TS); ash concentration; chemical oxygen demand (COD); theoretical oxygen demand (ThOD); COD/VS value; concentrations of organic constituent elements, including carbon (C:%VS), nitrogen (N:% VS), oxygen (O:%VS), and hydrogen (H:%VS); concentrations of carbohydrates, lipids, proteins, ash, and other nutrients; hemicellulose, cellulose, and lignin concentrations (%VS); C/N ratio (ratio of carbon to nitrogen concentration); lipid/VS ratio (ratio of lipid concentration to VS concentration); sodium concentration (Na); micronutrient concentrations, such as iron (mg/kg-COD feedstock), cobalt (mg/g-COD feedstock), and nickel (mg/g-COD feedstock); theoretical methane yield (ThBMP: ml/g-VS); actual methane yield (BMP: ml/g-VS) predicted value; biodegradation rate; VS decomposition rate; and energy yield.

To estimate the theoretical methane yield, multiple calculation methods are employed. These methods include those based on conventional composition formulas (elemental balance), chemical oxygen demand (COD), including both CODCr and CODMn, and nutrient-based methods that account for components such as fiber. The accuracy of the predicted and analyzed values is further enhanced by integrating data on the actual operational status of biogas plants.

For instance, when illustrating the estimation of theoretical methane yield based on a composition formula (elemental balance), the conversion of biomass to methane through methane fermentation can be represented by the following equation [Equation 1] .

[Equation 1]

$$C_aH_bO_cN_d + (a - b/4 - c/2 + 3d/4)\,H_2O$$
$$\rightarrow (a/2 + b/8 - c/4 - 3d/8)\,CH_4 + (a/2 - b/8 + c/4 - 3d/8)\,CO_2 + dNH_3$$

Here, $C_aH_bO_cN_d$ represents the organic composition formula of the raw material. In practice, the organic composition is typically considered equivalent to volatile solids (VS), a widely used indicator. Volatile solids refer to the substances that are vaporized when the residue from fermentation ingredients is heated to approximately 600°C. The subscripts a to d contain values specific to the material. For raw materials with unknown composition formulas, an empirically determined formula (empirical formula) must be used, which can be obtained through methods such as the CHN Corder analysis.

For instance, reported empirical formulas include $C_{17}H_{29}O_{10}N$ or $C_{13}H_{21}O_7N$ for food waste, $C_{266}H_{434}O_{210}N$ for paper waste, $C_7H_{12}O_4N$ for sewage sludge, $C_{22}H_{31}O_{11}N$ for dairy cow waste, and $C_{10}H_{19}O_3N$ for wastewater sludge. Using these composition formulas, the theoretical biochemical methane potential (Theoretical Biochemical Methane Potential, ThBMP) is calculated as follows:

[Equation 2]

$$ThBMP = \frac{[a/2 + b/8 - c/4 - 3d/8] \times 22400}{12a + b + 16c + 14d} \times f_D\ (ml/g\ VS)$$

Herein, fD represents the correction coefficient, defined as the proportion of decomposable organic matter, or the fermentation decomposition rate. If the decomposition rate is unknown, it must be determined experimentally. Reported decomposition rates include 0.75 to 0.85 for food waste and food residues, 0.45 to 0.55 for pig waste, 0.25 to 0.35 for dairy cow waste, 0.34 for newspapers, 0.55 for herbaceous plants, 0.25 for pruning branches, and 0.50 for sewage sludge.

The calculation result, ThBMP, represents the volume of methane gas (in milliliters) generated per gram of volatile solids (VS) in the raw material. When employing this prediction method, it is essential to analyze the VS content of the raw material. Additionally, if the chemical formula is unknown, determining the elemental composition (CHON) of the raw material is required.

Although decomposition rates for fD have been extensively documented in various reports, when these rates are not available, they must be established through culture experiments. Such experiments involve using the raw material with methane fermentation bacteria to measure the VS decomposition rate.

To illustrate the estimation of theoretical methane yield based on nutrient composition, carbohydrates, proteins, and lipids are recognized as the three primary nutrients in food. These nutrients can be converted into methane through methane fermentation. The theoretical methane yield is calculated using [Equation 3], which incorporates the methane yields-415 ml/g VS for carbohydrates, 496 ml/g VS for proteins, and 1014 ml/g VS for lipids-calculated from the ThBMP formula in [Equation 2]. These calculations are based on the average molecular formulas C6H10O5 (carbohydrates), C5H7O2N (proteins), and C57H104O6 (lipids), as well as the weight proportions of each nutrient within the volatile solids (VS), assuming VS is normalized to a value of 1.

[Equation 3]

$$ThBMP = \left(415 \times \text{carbohydrate rate} + 496 \times \text{Protain rate} + 1014 \times \text{lipid rate}\right) \times f_D \ (ml/g\ VS)$$

When employing this prediction method, it is essential to determine the VS content of the raw material and the weight ratios of the three primary nutrients-carbohydrates, proteins, and lipids. For raw materials consisting of a single food type, these values can often be obtained from the *Standard Tables of Food Composition.* For mixed or complex materials, analytical methods should be used to determine the proportions of carbohydrates, proteins, and lipids. Similar to the prediction method based on the elemental composition formula, the decomposition rate coefficient (fD) is derived from literature sources, such as published reports or results from culture experiments.

The component index master data, utilized by the "Methane Resource Recovery Evaluation AI 402a for Individual Materials," includes analysis results for various sample methane materials, such as food waste, oils and fats, sludge, livestock waste, animal and plant residues, and mixed organic waste. These results encompass the following: necessity of pretreatment for each methane material; organic substance composition of each methane material; fermentation temperature; fermentation duration; organic substance concentration (volatile solids, VS); solid substance amount (total solids, TS); organic substance ratio (VS/TS); ash concentration; chemical oxygen demand (COD); theoretical oxygen demand (ThOD); COD/VS value; concentrations of organic constituent elements (e.g., carbon (C:%VS), nitrogen (N:%VS), oxygen (O:%VS), hydrogen (H:%VS)); nutrient concentrations (e.g., carbohydrates, lipids, proteins, ash); hemicellulose, cellulose, and lignin concentrations (%VS); C/N ratio (carbon-to-nitrogen concentration ratio); lipid/VS ratio (lipid concentration to VS concentration ratio); sodium (Na) concentration; micronutrient concentrations (e.g., iron (mg/kg-COD feedstock), cobalt (mg/g-COD feedstock), nickel (mg/g-COD feedstock)); theoretical methane yield (ThBMP: ml/g-VS); actual methane yield (BMP: ml/g-VS); biodegradation rate; VS decomposition rate; energy yield; and more.

In addition to the analysis results for each of these items, data on the actual operating conditions at individual biogas plants is sampled for each methane material utilized and incorporated as learning data for the Methane Resource Recovery Evaluation AI 402a for Individual Materials.

(C) The prediction and analysis results generated by the Methane Resource Recovery Evaluation AI 402a for Individual Materials are presented within the Methane Resource Matching System to both the distributors of each material and the biogas plant operators. Simultaneously, these prediction and analysis results are imported into subsequent processes, including the "Methane Resource Recovery Evaluation AI 402b for Mixed Materials" and the "AI Gasification Promotion Program 403." These data serve as reference points for predicting and analyzing the compatibility of mixed fermentation for various biogas materials, as well as their synergistic (or inhibitory) effects.

(2-2) Details of Methane Resource Recovery Evaluation AI 402b for Mixed Materials

The Methane Resource Recovery Evaluation AI 402b for Mixed Materials is one of the core components of the "AI Methane Resource Recovery Program 402." This AI performs compatibility analysis and evaluation for mixed fermentation of one or more stocked biogas materials. It relies on the evaluation, prediction, and analysis data related to methane resource recovery generated by the aforementioned "Methane Resource Recovery Evaluation AI 402a for Individual Materials."

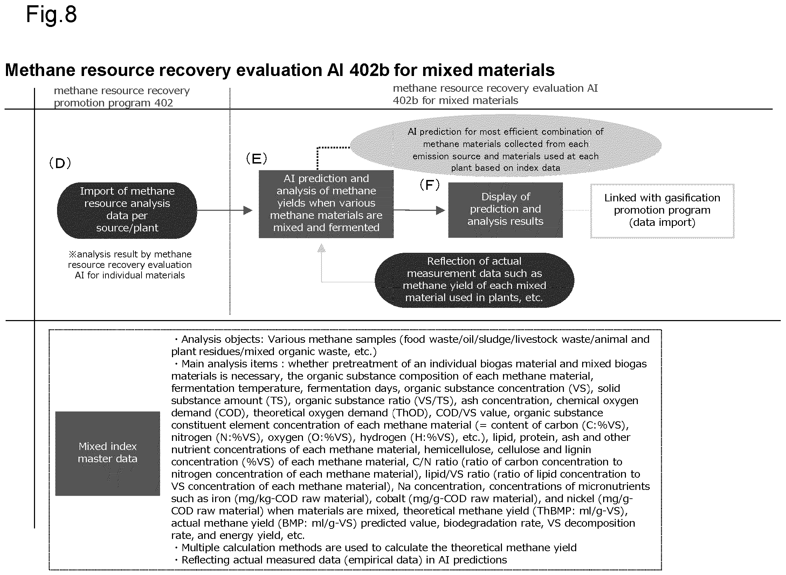

The evaluation considers biogas materials collected from various sources (e.g., commercial facilities, restaurants, hotels, food factories) and biogas materials currently used in biogas plants, including food waste, oils and fats, sludge, and livestock waste. By referring to the mixed index master data shown in FIG. 8, the AI conducts comprehensive compatibility assessments to determine the potential for successful mixed fermentation.

By simultaneously referencing data on the operational status of each biogas plant, the Methane Resource Recovery Evaluation AI 402b for Mixed Materials can analyze and identify the biogas material most compatible for mixed fermentation at each biogas plant. Furthermore, it can determine, in real time, the distribution source capable of providing the optimal biogas material for mixed fermentation.

In this context, data related to each biomass material, its source of origin, the biogas plant, and the biogas materials currently in use at the plant are always interconnected and maintained as a unified dataset.

The operational flow for the Methane Resource Recovery Evaluation AI 402b for Mixed Materials, as illustrated in FIG. 8, is outlined below. The evaluation, prediction, and analysis data related to methane resource recovery, generated by the Methane Resource Recovery Evaluation AI 402a for Individual Materials, for biogas materials (e.g., food waste) collected from various sources and biogas materials currently utilized in biogas plants (e.g., food waste, oils and fats, sludge, livestock waste), are imported into the Methane Resource Recovery Evaluation AI 402b for Mixed Materials through system integration.

(E) Building on the prediction and analysis results for individual biogas materials imported from the Methane Resource Recovery Evaluation AI 402a, the Methane Resource Recovery Evaluation AI 402b for Mixed Materials evaluates the compatibility of methane fermentation for mixed biogas materials. This is achieved by referencing the Mixture Index Master Data to predict and analyze the effects of combining and fermenting the materials.

The following items are assumed as prediction and analysis parameters when evaluating the compatibility of mixed fermentation for stocked biogas materials: the necessity of pretreatment for each raw material during mixing and fermentation; the organic composition of each methane material; fermentation temperature and days; concentrations of organic substances (VS) and solids (TS); the VS/TS ratio; ash concentration; chemical oxygen demand (COD); theoretical oxygen demand (ThOD); the COD/VS ratio; elemental composition of organic substances, including carbon (C:%VS), nitrogen (N:%VS), oxygen (O:%VS), and hydrogen (H:%VS); concentrations of nutrients such as carbohydrates, lipids, proteins, and ash; hemicellulose, cellulose, and lignin concentrations (%VS); the C/N ratio (carbon-to-nitrogen concentration ratio); the lipid/VS ratio; Na concentration; micronutrient concentrations such as iron (mg/kg-COD raw material), cobalt (mg/g-COD raw material), and nickel (mg/g-COD raw material); theoretical methane yield (ThBMP: ml/g-VS); actual methane yield (BMP: ml/g-VS); biodegradation rate; VS decomposition rate; and energy yield and more.

To estimate the theoretical methane yield, multiple calculation methods are employed. These methods include composition formula-based calculations (elemental balance), chemical oxygen demand (COD) -based calculations (including CODCr and CODMn), and nutrient-based calculations (which incorporate fiber content). Additionally, the accuracy of prediction and analysis values is enhanced by integrating data on the actual operating conditions of biogas plants.

The mixed index master data, which the "methane resource recovery evaluation AI 402b for mixed materials" utilizes for learning and analysis, includes data for various sample methane materials, such as food waste, oils and fats, sludge, livestock waste, animal and plant residues, and mixed organic waste. The data encompasses the following attributes: whether pretreatment of each methane material is necessary; the organic substance composition of each methane material; fermentation temperature; fermentation days; organic substance concentration (VS); solid substance amount (TS); organic substance ratio (VS/TS); ash concentration; chemical oxygen demand (COD); theoretical oxygen demand (ThOD); COD/VS value; organic substance constituent element concentrations, such as carbon (C:%VS), nitrogen (N:%VS), oxygen (O:%VS), and hydrogen (H:%VS); carbohydrate, lipid, protein, ash, and other nutrient concentrations; hemicellulose, cellulose, and lignin concentrations (%VS); the C/N ratio (carbon-to-nitrogen concentration ratio); lipid/VS ratio (lipid concentration to VS concentration ratio); Na concentration; micronutrient concentrations such as iron (mg/kg-COD raw material), cobalt (mg/g-COD raw material), and nickel (mg/g-COD raw material); theoretical methane yield (ThBMP: ml/g-VS); actual methane yield (BMP: ml/g-VS) predicted value; biodegradation rate; VS decomposition rate; and energy yield.

The analysis results for each of these attributes are incorporated into the master data. Additionally, data on the actual operating conditions at biogas plants is sampled based on the methane materials used and is employed as learning data for the AI.

The evaluation value calculation device described in this embodiment utilizes specific indices and logic to determine the compatibility of each biogas material in stock during mixed fermentation. For example, the following items, in the order a through g, are assumed: (a) C/N ratio: whether the ratio of carbon concentration to nitrogen concentration in the raw material is 25 or more; values below 25 indicate a risk of inhibition;

(b) Lipid/VS ratio: whether the ratio of lipid concentration to VS concentration in the raw material is 35 or less; values above 35 pose a risk of inhibition;
(c) Lipid/carbohydrate ratio: whether the ratio of lipid concentration to carbohydrate concentration in the raw material is 13 or more;
(d) Protein/carbohydrate ratio: whether the ratio of protein concentration to carbohydrate concentration in the raw material is 26 or more;
(e) Micronutrients: for example, whether the raw material contains at least 165 mg/kg-COD of iron, 14 mg/g-COD of cobalt, and 5 mg/g-COD of nickel;
(f) Na concentration: whether the sodium concentration in the raw material is less than 5.6 g/L;
(g) Provided that conditions a through f are satisfied (with a, b, e, and f being essential conditions and the order of evaluation non-specific), the higher the methane yield during mixed fermentation-measured as the methane production per gram of volatile solids (VS) in the raw material (unit: mL/g-VS)-the better the compatibility of the materials.

Additionally, if the methane yield is less than 100 mL/g-VS, the combination of biogas materials is considered unsuitable for simple mixed fermentation from an economic perspective.

Furthermore, the prediction and analysis results (data) generated by the methane resource recovery evaluation AI 402b for mixed materials are presented to both the sources of the materials and biogas plant operators through the methane resource matching system S. Simultaneously, these results are imported into the subsequent "AI Gasification Promotion Program 403" to serve as reference data for predicting and analyzing the synergy and inhibitory effects of each biogas material during mixed fermentation.

(F) The results (data) of the predictions and analyses conducted by the methane resource recovery evaluation AI 402b for mixed materials are presented to the sources of each material and to biogas plant operators via the methane resource matching system S. Simultaneously, these prediction and analysis results are imported into the subsequent "AI Gasification Promotion Program 403" and serve as one of the reference data sets for predicting and analyzing the synergy and inhibitory effects of each biogas material during mixed fermentation.

(3) Details of the AI Gasification Promotion Program

The AI gasification promotion program 403 comprises a methane yield promotion prediction AI 403a; it forms part of the methane resource matching system S, together with the previously mentioned AI methane resource recovery program 402.

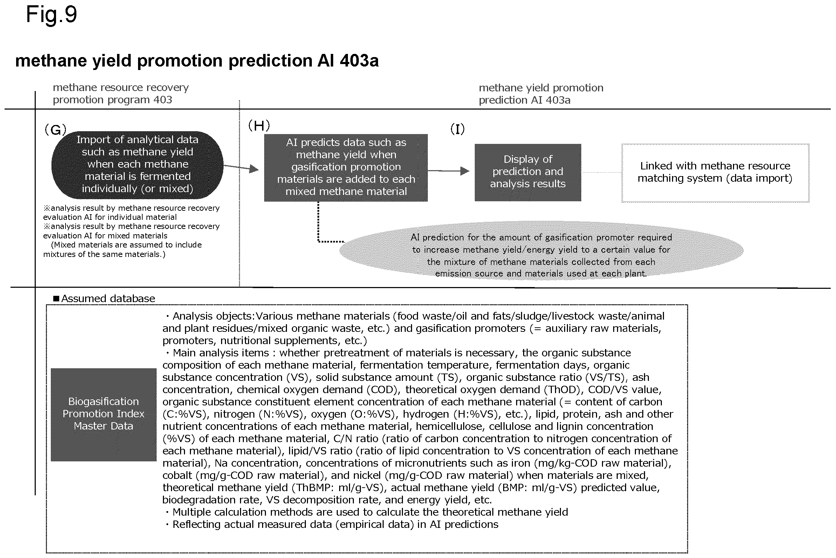

The methane yield promotion prediction AI 403a predicts and analyzes the types and amounts of "gasification promoters," "nutritional supplements," and "auxiliary raw materials" required to create optimal fermentation conditions when mixing and fermenting one or more biogas materials in stock. These predictions are based on evaluation and analysis data from the "Methane resource recovery evaluation AI 402b for mixed materials," which examines biogas materials collected from sources such as commercial facilities, restaurants, hotels, and food factories, alongside materials currently used in biogas plants, including food waste, oils and fats, sludge, and livestock waste. Furthermore, compatibility analysis and evaluation data for mixed fermentation of in-stock biogas materials, also provided by the AI, are incorporated into this process. The analysis is further refined by referencing the bio-gasification promotion index master data illustrated in FIG.9.

The methane yield promotion prediction AI 403a predicts and analyzes the type and amount of gasification promoters required to adjust the numerical indicators of each analysis item that influence methane gas production. These adjustments ensure that items with insufficient or excessive values are brought within their normal ranges. The indicators include, but are not limited to, the conditions a to g listed for determining the compatibility of the aforementioned mixed fermentation.

The methane yield promotion prediction AI 403a predicts and analyzes the potential synergy associated with the mixing of materials that do not require the addition of gasification promoters or other additives (i.e., those that satisfy all numerical indicators for biogas generation). This analysis is conducted by referencing the bio-gasification promotion indicator master data, as shown in FIG. 9.

Additionally, for combinations of materials identified through the prediction and analysis results of the "Methane resource recovery evaluation AI 402b for mixed materials" as having an estimated methane yield of less than 100 mL/g-VS after mixed fermentation, the addition of "gasification promoters," "nutritional supplements," or "auxiliary raw materials" may enable the combination to meet favorable conditions for methane gas production.

The functionality and process flow assumed for the methane yield promotion prediction AI 403a are described below with reference to the flow diagram presented in FIG.9.

(G) Initially, the evaluation, prediction, and analysis data generated by the "methane resource recovery evaluation AI 402a for individual materials" and the "methane resource recovery evaluation AI 402b for mixed materials" are integrated into the AI gasification promotion program 403 (methane yield promotion prediction AI 403a) through system integration.

(H) Based on the prediction and analysis data imported from the "Methane Resource Recovery Evaluation AI 402a for Individual Materials" and the "Methane Resource Recovery Evaluation AI 402b for Mixed Materials," the Methane Yield Promotion Prediction AI 403a predicts and analyzes the types and quantities of "gasification promoters," "nutritional supplements," and "auxiliary raw materials" necessary to optimize fermentation conditions for the individual fermentation of each biogas material. This process is carried out by referencing the bio-gasification promotion index master data.

Furthermore, the Methane Yield Promotion Prediction AI 403a evaluates the types and quantities of "gasification promoters," "nutritional supplements," and "auxiliary raw materials" required to optimize fermentation conditions for various combinations of one or more stocked biogas materials. These combinations are derived from the "Methane Resource Recovery Evaluation AI 402b for Mixed Materials," and the analysis is also conducted by referencing the bio-gasification promotion index master data.

Herein, the types of "gasification promoters," "nutritional supplements," and "auxiliary raw materials" considered include the following examples: gasification promoters, such as cellulase enzymes, rumen fluid, and biochar; nutritional supplements, such as $NiCl_2 \cdot 6H_2O$, $CoCl_2 \cdot 6H_2O$, and $FeCl_2 \cdot 4H_2O$; and auxiliary raw materials, such as oil and fat refining sludge and BDF residue glycerin.

For example, during the individual or mixed fermentation of each biogas material, the methane yield promotion prediction AI 403a predicts and analyzes the types and quantities of "gasification promoters," "nutritional supplements," and "auxiliary raw materials" required to ensure that the values of the following analytical items fall within the optimal range for favorable fermentation conditions: whether pretreatment of raw materials is necessary; the organic composition of each methane material; fermentation temperature; fermentation duration; organic substance concentration (VS); solid substance amount (TS); organic substance ratio (VS/TS); ash concentration; chemical oxygen demand (COD); theoretical oxygen demand (ThOD); COD/VS value; concentrations of organic elemental constituents in each methane material, including carbon (C:%VS), nitrogen (N:%VS), oxygen (O:%VS), and hydrogen (H:%VS); carbohydrate, lipid, protein, ash, and other nutrient concentrations; hemicellulose, cellulose, and lignin concentrations (%VS); C/N ratio (carbon-to-nitrogen concentration ratio); lipid/VS ratio (lipid-to-VS concentration ratio); Na concentration; concentrations of micronutrients such as iron (mg/kg-COD raw material), cobalt (mg/g-COD raw material), and nickel (mg/g-COD raw material) during material mixing; theoretical methane yield (ThBMP: ml/g-VS); predicted actual methane yield (BMP: ml/g-VS); biodegradation rate; VS decomposition rate; and energy yield.

The numerical indicators for each analysis item include the following criteria:

(a) C/N Ratio: Whether the ratio of carbon concentration to nitrogen concentration of the raw material is 25 or greater (ratios below 25 may present a risk of inhibition);
(b) Lipid/VS Ratio: Whether the ratio of lipid concentration to the volatile solids (VS) concentration of the raw material is 35 or less (ratios of 35 or more may present a risk of inhibition);
(c) Lipid/Carbohydrate Ratio: Whether the ratio of lipid concentration to carbohydrate concentration of the raw material is 13 or greater;
(d) Protein/Carbohydrate Ratio: Whether the ratio of protein concentration to carbohydrate concentration of the raw material is 26 or greater;
(e) Micronutrients: For example, whether the raw material contains iron at 165 mg/kg-COD or more, cobalt at 14 mg/g-COD or more, and nickel at 5 mg/g-COD or more;
(f) Na Concentration: Whether the sodium concentration of the raw material is less than 5.6 g/L;
(g) Methane Yield: Provided that conditions (a) through (f) are met, whether the methane yield, defined as the volume of methane produced per gram of raw material VS (unit: mL/g-VS), is 100 mL/g-VS or more during individual or mixed fermentation.

In addition to the primary indicators, further analysis items and numerical indicators used to assess synergy include the following criteria:

(a) Whether the C/N ratio, defined as the ratio of carbon concentration to nitrogen concentration in the raw material, falls between 25 and 27;
(b) Whether the protein/carbohydrate ratio, defined as the ratio of protein concentration to carbohydrate concentration in the raw material, is 26 or greater;
(c) Whether the lipid/VS ratio, defined as the ratio of lipid concentration to the volatile solids (VS) concentration in the raw material, is 35 or less, while also ensuring that the lipid/carbohydrate ratio, defined as the ratio of lipid concentration to carbohydrate concentration in the raw material, is 13 or greater.

For individual materials (data from the methane resource recovery evaluation AI 402a), the synergy resulting from mixing the material with a "gasification promoter" or similar additives is predicted and analyzed. For mixed materials (data from the methane resource recovery evaluation AI 402b), the synergy from simple mixing of materials, both with and without the addition of "gasification promoters" or similar additives, is also predicted and analyzed.

(I) Finally, the methane yield promotion prediction AI 403a outputs the results of its predictions and analyses, which are then presented to the sources of the respective biogas materials and the operators of biogas plants through the methane resource matching system S.

<Specific Configuration of the Evaluation Value Calculation Device for Biogas Materials>

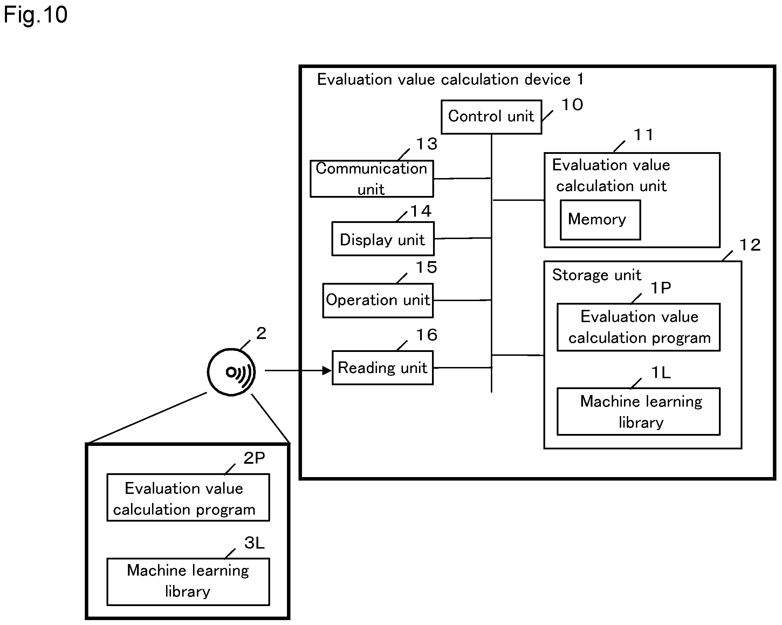

The processing units included in the evaluation value calculation device 1 for biogas materials, as implemented in this embodiment, are described with reference to FIG. 10. The evaluation value calculation device 1 functions as at least one of the following: a learning device that trains machine learning models using library data specific to biogas materials to determine model parameters, and an estimation device that calculates evaluation values related to methane fermentation and methane gasification (resource recovery) by applying the trained machine learning model to target biogas materials, including both individual materials and mixed materials.

First, the processing units integrated within the methane resource recovery device 1 will be described with reference to FIG. 10. As illustrated in FIG. 10, the methane resource recovery device 1 comprises the following components: a control unit (10); an evaluation value calculation unit (11); a storage unit (12); a communication unit (13); a display unit (14); an operation unit (15); and a reading unit (16). While the operation of the evaluation value calculation device 1 is explained below as being performed on a single server computer, the device may alternatively be configured to distribute processing across multiple computers or operate as a cloud-based system.

The control unit (10) utilizes a processor, such as a CPU, and memory to manage the device's components and execute various functions.

The evaluation value calculation unit (11) employs a processor and memory to estimate evaluation values related to the resource recovery of the target biogas material, based on control instructions received from the control unit (10). The control unit (10) and the evaluation value calculation unit (11) may be implemented as a single hardware unit, such as a System on a Chip (SoC), integrating a processor (e.g., a CPU), memory, the storage unit (12), and the communication unit (13).

The storage unit (12) utilizes a hard disk or flash memory to store various essential components, including the evaluation value calculation program (1P) and a machine learning library (1L) that serves as the machine learning model. Additionally, the storage unit (12) retains definition data for the machine learning model, parameters such as the trained model's setting values, and other related information. The programs contained within the evaluation value calculation program (1P) specifically include the AI methane resource recovery program (402) and the AI gasification promotion program (403) previously described.

The communication unit (13) functions as a communication module enabling connectivity to communication networks, such as the Internet. It facilitates the transmission of biogas material-related information, including a machine learning library and evaluation values associated with the resource recovery of biogas materials, to other terminal devices. The communication unit (13) may incorporate components such as a network card, a wireless communication device, or a carrier communication module.

The display unit (14) utilizes components such as a liquid crystal panel to provide visual output. The operation unit (15) may include user interface devices such as a keyboard or mouse. It transmits user-input operation information to the control unit (10). This user-provided information may include property data (e.g., type, collection location, characteristics, and quantity of biogas materials, both individual and mixed) as well as operational status data of the biogas plant.

The reading unit (16) may utilize a disk drive or similar device to access the optimization calculation program and machine learning library stored on a recording medium (e.g., an optical disk). The evaluation value calculation program (1P) and machine learning library (1L) stored in the storage unit (12) can originate from the evaluation value calculation program (2P) and machine learning library (3L) read by the reading unit (16) from the recording medium. These programs and libraries may then be copied to the storage unit (12) under the control of the control unit (10).

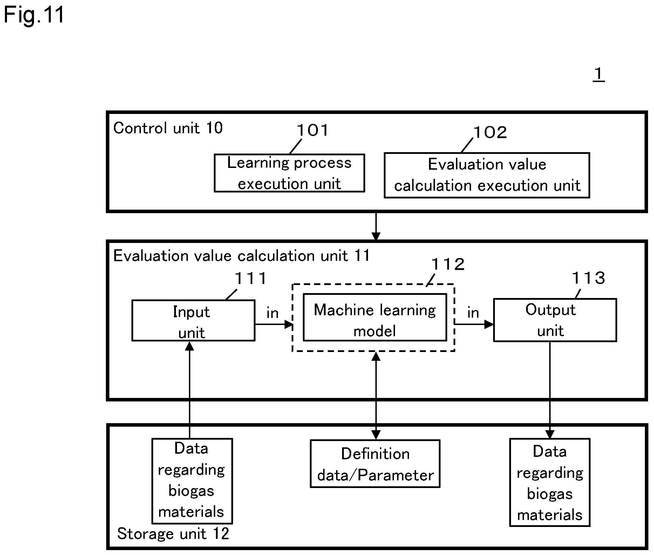

Next, the functionality of the evaluation value calculation device (1) is described with reference to FIG. 11. The control unit (10) of the evaluation value calculation device (1) includes a learning process execution unit (101), which utilizes artificial intelligence (AI), and an evaluation value calculation execution unit (102).

The learning process execution unit (101) functions as a machine learning model (machine learning engine) based on the machine learning library (1L), definition data, and parameter information stored in the storage unit (12). In essence, the learning process execution unit (101) executes the process of automatically learning the setting values (e.g., parameters) of the machine learning model to be trained, using training data while utilizing the model itself during the learning process.

During the learning process, the machine learning libraries analyze various parameters related to biogas materials. These parameters include whether pretreatment is required for different biogas materials; the organic substance composition of each methane material; fermentation conditions such as temperature and days; organic substance concentration (VS: Volatile Solids) and solid substance amount (TS: Total Solids); the ratio of organic substances (VS/TS); ash concentration; chemical oxygen demand (COD); theoretical oxygen demand (ThOD); COD/VS value; the concentration of organic constituent elements (e.g., carbon (C:%VS), nitrogen (N:%VS), oxygen (O:%VS), hydrogen (H:%VS)); the concentrations of carbohydrates, lipids, proteins, ash, and other nutrients; hemicellulose, cellulose, and lignin concentrations (%VS); the C/N ratio (carbon to nitrogen concentration ratio); lipid/VS ratio (lipid to VS concentration ratio); Na concentration; and micronutrient concentrations such as iron (mg/kg-COD raw material), cobalt (mg/g-COD raw material), and nickel (mg/g-COD raw material). Additional factors include theoretical methane yield (ThBMP: ml/g-VS), predicted actual methane yield (BMP: ml/g-VS), biodegradation rate, VS decomposition rate, and energy yield.

For example, the learning process execution unit 101 can perform a process to minimize the error between the output data generated by inputting training data into the complete machine learning model 112 and the corresponding known training data. As a result, the unit updates the model's parameters (weights). The parameters obtained through this learning process are stored in the storage unit 12.

The evaluation value calculation execution unit 102 estimates evaluation values based on the evaluation value calculation program 1P stored in the storage unit 12. Specifically, it uses a machine learning model to estimate evaluation values for the input biogas materials (individual or mixed). Additionally, the evaluation value calculation execution unit 102 processes input data, which includes biogas material-related information (e.g., machine learning libraries, property data such as the type of biomass material, collection location, characteristics, quantity, and operational status data of the biogas plant). This input is provided to the input unit 111 based on user operations performed through the operation unit 15.

As described above, the operational status data for the biogas plant includes information such as the type of biogas material used at the plant; the fermentation method; the presence and quantity of additives, such as coagulants; the volatile solids (VS) volume load per cubic meter of the methane fermentation tank; the organic decomposition rate within the methane fermentation tank; the volume of methane gas generated in the gas holder; and the measured methane yield (Biochemical Methane Potential, BMP).

The input unit 111 of the evaluation value calculation unit 11 processes input related to a machine learning library associated with biogas materials, as well as biogas material-related data. The learning process execution unit 101 transmits the machine learning library to the machine learning model 112. Additionally, the evaluation value calculation execution unit 102 provides property data of the biogas material, which is the subject of the evaluation value estimation, along with operational status data of the biogas plant, to the machine learning model 112.

The specific type of biogas material-related data, including property data of the biogas material under evaluation and operational status data of the biogas plant, may be identified, for example, through information or an identifier located in the header section.

The development program for the machine learning model 112 may utilize widely adopted programming languages such as Python or MATLAB. These tools are commonly employed for tasks such as reading various data files, performing deep learning, conducting data analysis, constructing model formulas, and implementing programming logic.

If a convolutional neural network (CNN) is employed, which utilizes multiple convolutional layers and pooling layers to extract features from input data during inference, the machine learning model 112 may incorporate several stages of convolutional and pooling layers as defined by the corresponding definition data. Additionally, the model may include a fully connected layer to extract input data features and execute estimation processing based on these extracted features.

When utilizing a trained model, the machine learning model 112 estimates evaluation values related to the resource recovery of biogas materials, based on pre-trained parameters. These evaluations are derived from property data, such as the type, collection location, characteristics, and quantity of biogas materials (both individual and mixed materials), as well as data on the operational status of the biogas plant. The evaluation values include determinations such as whether pretreatment is required; the organic substance composition of each methane material; fermentation temperature; fermentation duration; organic substance concentration (VS); solid substance amount (TS); organic substance ratio (VS/TS); ash concentration; chemical oxygen demand (COD); theoretical oxygen demand (ThOD); COD/VS ratio; concentrations of organic substance constituent elements (e.g., carbon (C:%VS), nitrogen (N:%VS), oxygen (O:%VS), hydrogen (H:%VS)); concentrations of nutrients such as carbohydrates, lipids, proteins, and ash; hemicellulose, cellulose, and lignin concentrations (%VS); C/N ratio (carbon to nitrogen concentration ratio); lipid/VS ratio (lipid to VS concentration ratio); sodium (Na) concentration; and micronutrient concentrations such as iron (mg/kg-COD feedstock), cobalt (mg/g-COD feedstock), and nickel (mg/g-COD feedstock). Additionally, theoretical methane yield (ThBMP: ml/g-VS), actual methane yield (BMP: ml/g-VS) predicted values, biodegradation rate, VS decomposition rate, energy yield, and other related metrics are estimated.

Particularly, when estimating the evaluation value of mixed materials, the following criteria are assessed:

(a) C/N Ratio: Whether the ratio of carbon concentration to nitrogen concentration in the raw material is 25 or greater. Ratios below 25 pose a risk of fermentation inhibition.

(b) Lipid/VS Ratio: Whether the ratio of lipid concentration to volatile solids (VS) concentration in the raw material is 35 or less. Ratios exceeding 35 pose a risk of inhibition.

(c) Lipid/Carbohydrate Ratio: Whether the ratio of lipid concentration to carbohydrate concentration in the raw material is 13 or greater.

(d) Protein/Carbohydrate Ratio: Whether the ratio of protein concentration to carbohydrate concentration in the raw material is 26 or greater.

(e) Micronutrients: Whether the raw material contains sufficient levels of essential micronutrients, such as iron (≥165 mg/kg-COD raw material), cobalt (≥14 mg/g-COD raw material), and nickel (≥5 mg/g-COD raw material).

(f) Na Concentration: Whether the sodium concentration in the raw material is less than 5.6 g/L.

(g) Methane Yield: Provided the above conditions (a to f) are met (with a, b, e, and f being essential), whether the methane yield during mixed fermentation-defined as the amount of methane produced per gram of VS (unit: mL/g-VS)-is sufficiently high to indicate good compatibility for mixed fermentation.

If the methane yield is less than 100 mL/g-VS, the combination of materials is deemed unsuitable for simple mixed fermentation from an economic perspective.

Herein, the machine learning model 112 assesses the appropriate types and quantities of gasification promoters, nutritional supplements, auxiliary raw materials, and related components, based on the evaluation values of the mixed fermentation of each biogas material. Consequently, it becomes possible to optimize fermentation conditions for the processing of one or more stored biogas materials.

In this context, the evaluation values used to determine the synergy with the "gasification promoter," "nutritional supplement," and "auxiliary raw material" are specified as follows:

(a) C/N ratio: whether the ratio of carbon concentration to nitrogen concentration in the raw material falls between 25 and 27;
(b) Protein/Carbohydrate ratio: whether the ratio of protein concentration to carbohydrate concentration in the raw material is 26 or higher;
(c) Lipid/VS and Lipid/Carbohydrate ratios: whether the ratio of lipid concentration to volatile solids (VS) in the raw material is 35 or higher, and whether the ratio of lipid concentration to carbohydrate concentration is 13 or higher.

The evaluation value generated by the machine learning model 112 is subsequently sent to the output unit 113 and can be stored in the memory unit 12 as biogas material-related data.

As described above, the evaluation value calculation device 1 links biogas materials discharged from their sources (e.g., commercial facilities, restaurants, hotels, food factories) with the biogas materials currently utilized in biogas plants, using the evaluation values. This linkage enables the preparation of a machine learning model designed for more efficient biogas production. Consequently, the evaluation value calculation device 1 employs the AI-driven machine learning model 112 to learn and estimate evaluation values related to the resource recovery of biogas materials. This capability facilitates precise mixing of methane gas materials and contributes to achieving carbon neutrality.

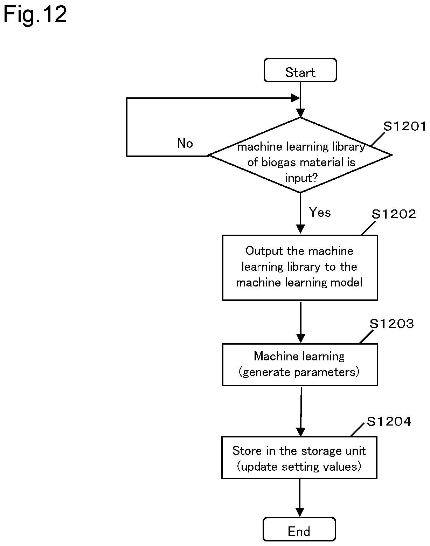

In the next step, the operation procedure for the evaluation value calculation device 1, functioning as a learning device in this embodiment, will be described with reference to FIG. 12. First, when the machine learning library 1L is input into the input unit 111 (Yes in S1201), the input unit 111 transmits the library to the machine learning model 112 (S1202). The machine learning model 112 then performs parameter updating (i.e., updating of setting values) based on the machine learning library 1L (S1203). In the context of machine learning using a neural network, this parameter update involves minimizing the difference between the input data and the answer data, for example, through the mini-batch gradient descent method. Finally, the updated parameters are stored in the storage unit 12 (S1204).

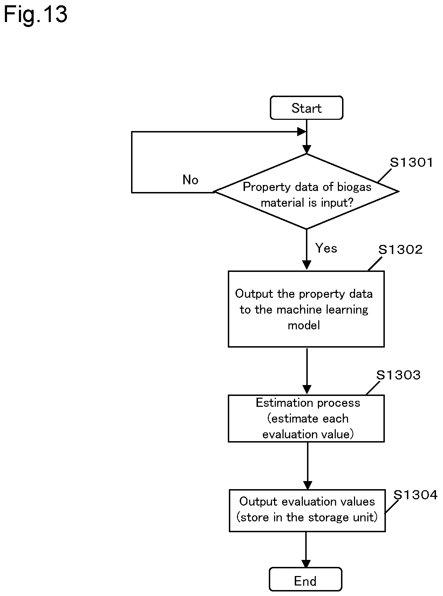

The operation procedure for the evaluation value calculation device 1, when functioning as an estimation device in this embodiment, is described below with reference to FIG. 13. First, if property data-such as the type, collection location, properties, and quantity of biogas material-is input into the input unit 111 (Yes in S1301), the input unit 111 transmits this property data to the machine learning model 112 (S1302). Next, the machine learning model 112 executes an estimation process based on the input property data and generates estimation data, including evaluation values and assessments corresponding to conditions (a) through (g) as previously described (S1303). Finally, the evaluation value resulting from this estimation process is stored in the storage unit 12 (S1304).

<Experimental Example>

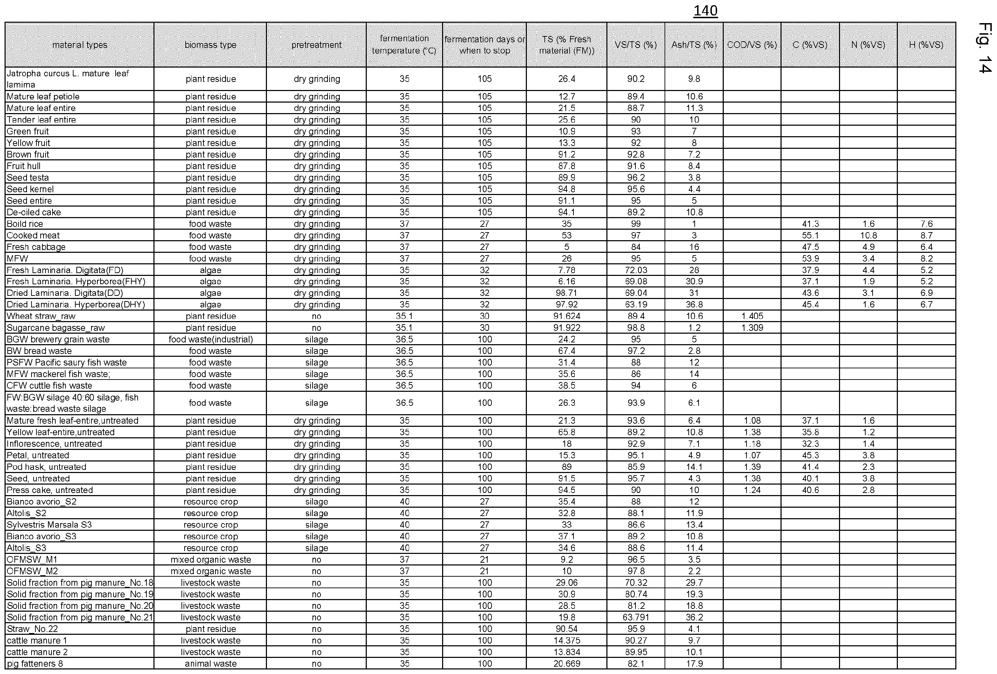

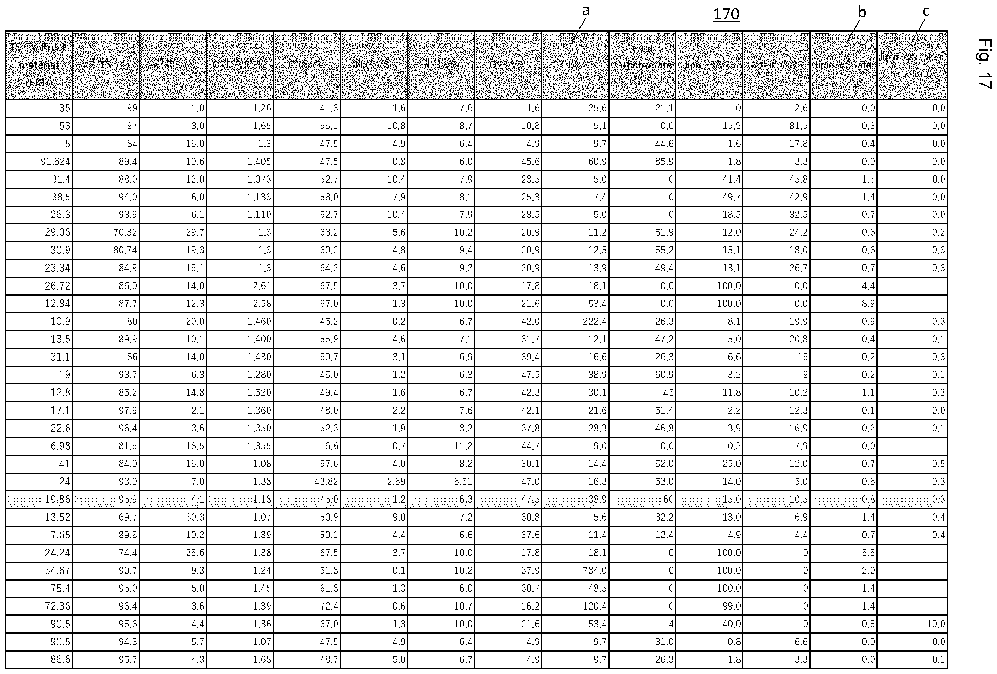

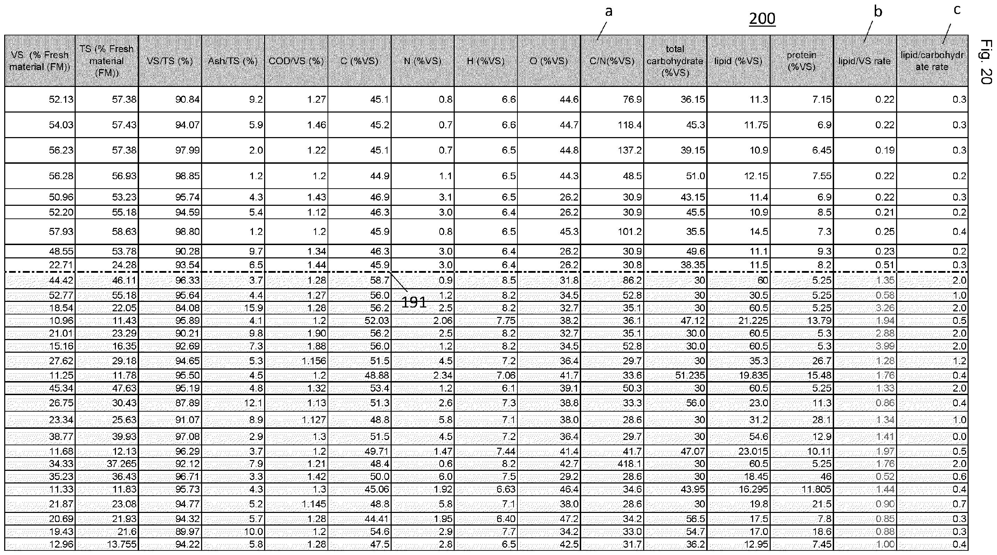

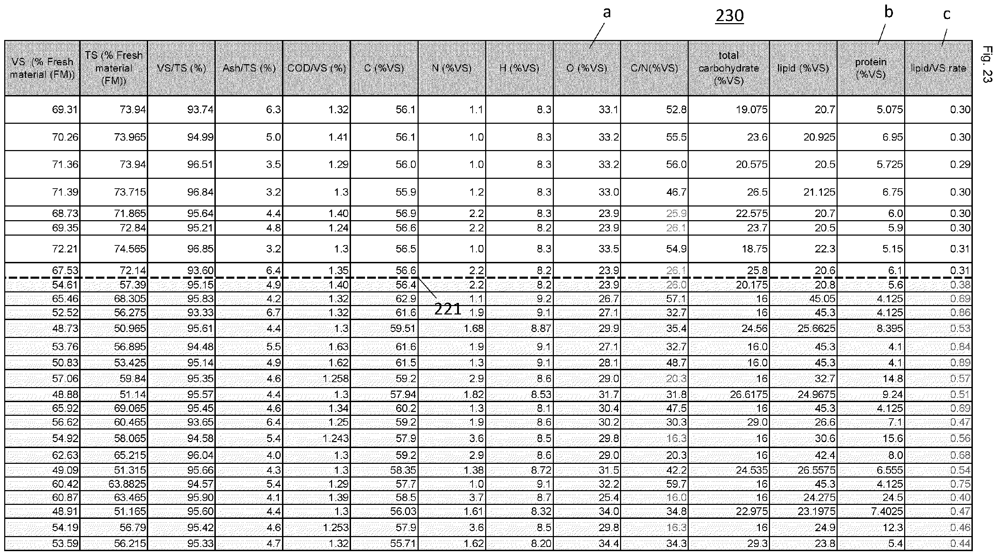

The following section presents an experimental example of machine learning using the evaluation value calculation device 1 for methane materials, as described in this embodiment, with reference to FIGS. 14 to 23. The purpose of this experiment is to develop an AI model capable of generating evaluation values to optimize the production of biogas fuel, utilizing biogas materials such as food waste as the primary raw material.

The AI model developed in this experiment is designed to predict evaluation values such as methane yield in biogas materials. For the purpose of this experiment, "MATLAB" (produced by MathWorks) was selected as the AI model development platform due to its robust mathematical processing capabilities, integration of advanced neural networks, extensive machine learning functions, and its ability to operate without requiring vast amounts of training data or complex programming. Alternative platforms, such as "Python" or "Mathematica" (produced by Wolfram), may also be utilized for similar purposes.

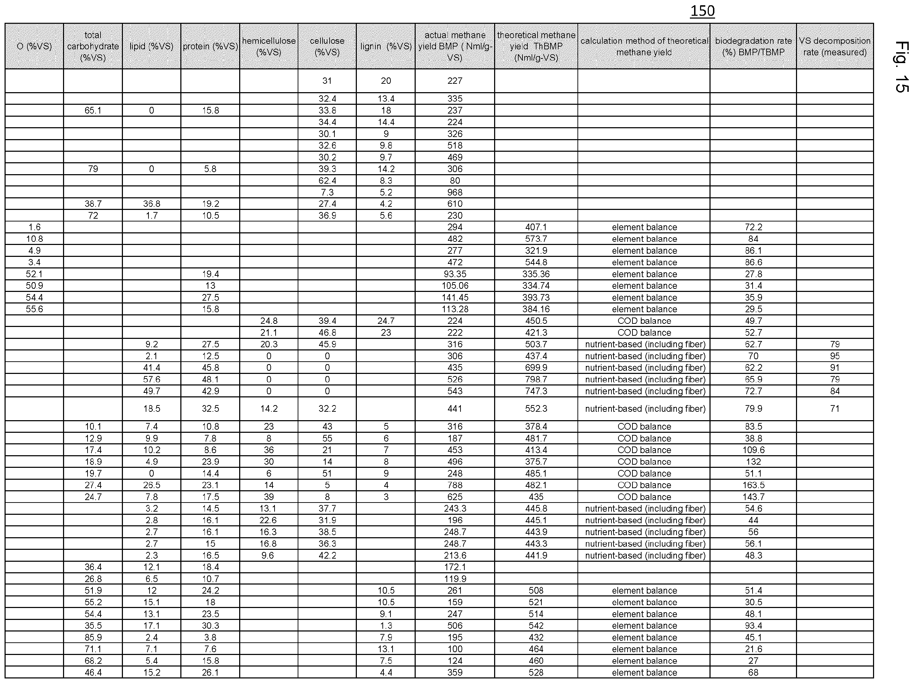

The specific AI model developed in this experiment is described below. An example of the machine learning library utilized in this AI model is presented in Tables 140 and 150, shown in FIGS. 14 and 15 (displayed as adjacent tables in the actual figures). As outlined in Tables 140 and 150, the machine learning library includes data on various aspects of biogas materials: types of raw materials for biogas production and their corresponding biomass species; whether pretreatment of each raw material is necessary; methane gas fermentation methods, fermentation temperatures, fermentation durations, or target dates for fermentation cessation; organic substance concentration (VS); solid substance amount (TS); organic substance ratio (VS/TS); ash concentration (Ash/TS); chemical oxygen demand (COD); COD/VS value; constituent element concentrations of organic substances in each methane material (e.g., carbon (C:%VS), nitrogen (N:%VS), oxygen (O:%VS), hydrogen (H:%VS)); nutrient concentrations such as carbohydrates, lipids, proteins, ash, and other components; hemicellulose, cellulose, and lignin concentrations (%VS); C/N ratio (carbon-to-nitrogen concentration ratio of each methane material); lipid/VS ratio (lipid concentration relative to VS concentration in each methane material); sodium (Na) concentration; micronutrient concentrations for each feedstock, including iron (mg/kg-COD feedstock), cobalt (mg/g-COD feedstock), and nickel (mg/g-COD feedstock); theoretical methane yield (ThBMP:

ml/g-VS); actual methane yield (BMP: ml/g-VS) predicted values; biodegradation rates; VS decomposition rates; and energy yields.

It is possible to include various types of AI models in this context, such as a machine learning model that trains a high-dimensional dataset as a multivariate normal distribution, and a deep learning model utilizing an autoencoder, which is a specific type of deep learning. However, other learning models may also be implemented, depending on the requirements and available data.

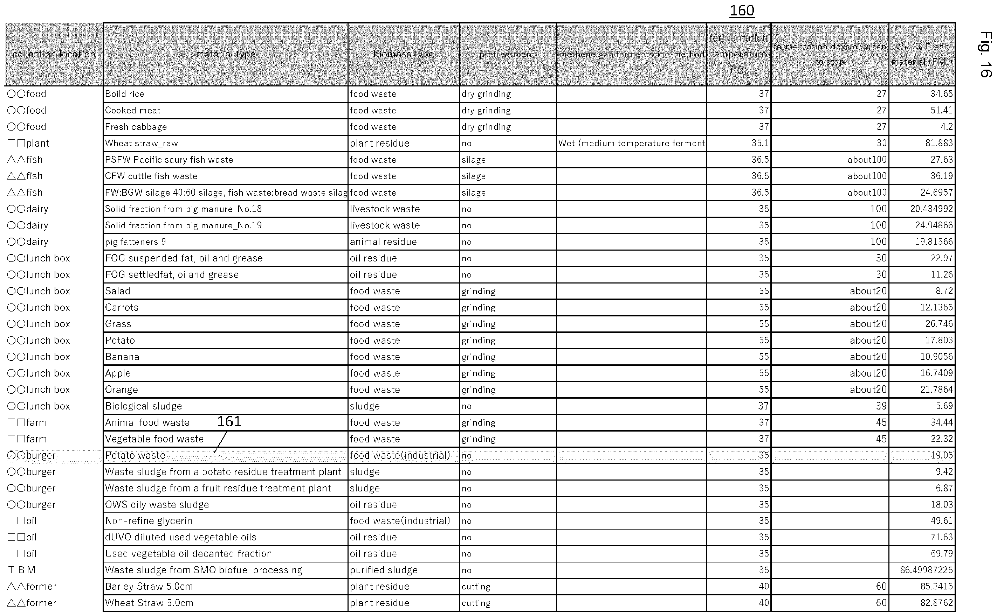

The estimated results of the evaluation values for resource recovery in this experimental example are described with reference to FIGS. 16 through 24. Using the machine learning model developed as outlined above, the evaluation values for resource recovery are first estimated based on property data, including the collection location and type of raw material for individual biogas materials. The results of these estimations are presented in Tables 160, 170, and 180, shown in FIGS. 16 through 18 (these tables are displayed side by side in the actual figures).

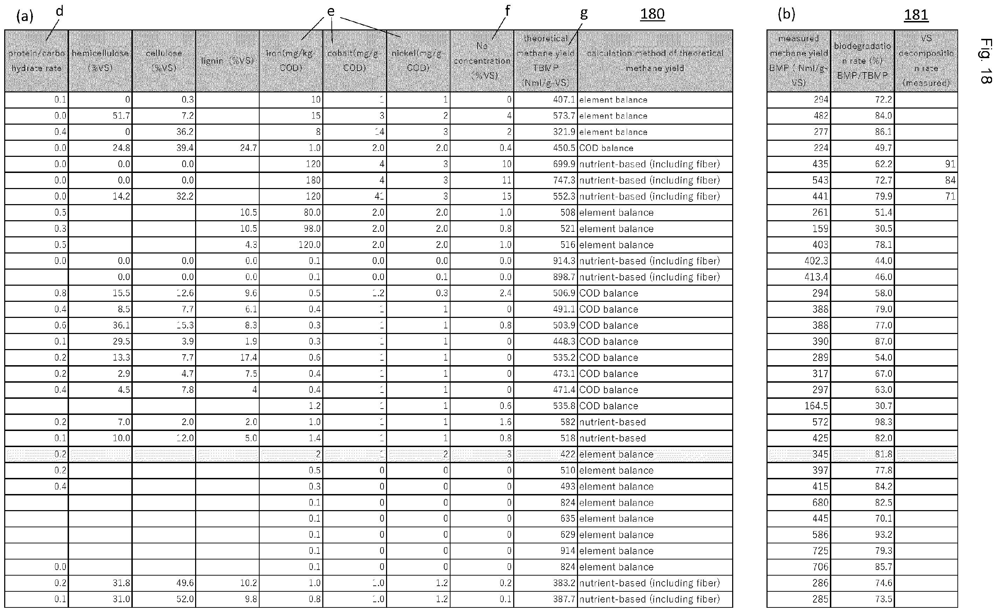

In Table 181 of FIG. 18(b), measured data such as methane yield and VS decomposition rate, which are not estimated, are recorded. To enhance the accuracy of estimations, including theoretical methane yield, it is essential to increase the quantity of actual measured data available for comparison with the estimated data and incorporate this data into the machine learning library used by the learning model. Furthermore, operational improvements are necessary, such as developing a program that integrates additional measured values as training data (library data) into the learning model and implementing a user-friendly interface to simplify the creation of learning models.

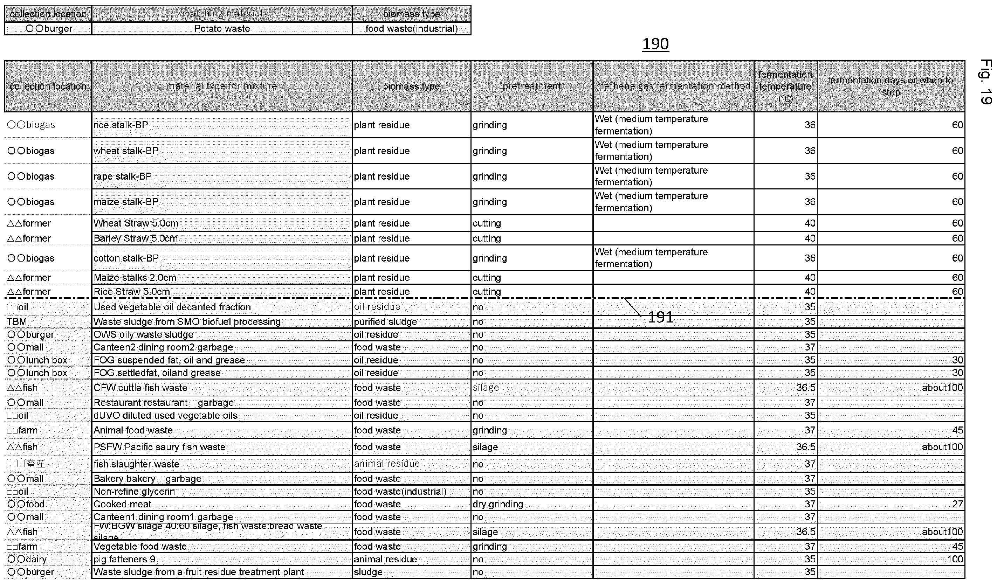

Using the created machine learning model, evaluation values for each mixed biogas material were estimated. In this scenario, one material (potato waste 161) from the individually estimated biogas materials was mixed at a 50:50 ratio with each of the other individual biogas materials listed in FIGS. 16 to 18 (specifically, all materials listed below boiled rice in FIG. 16). The resulting estimated evaluation values are presented in Tables 190, 200, and 210 in FIGS. 19 to 21 (which are arranged side by side).

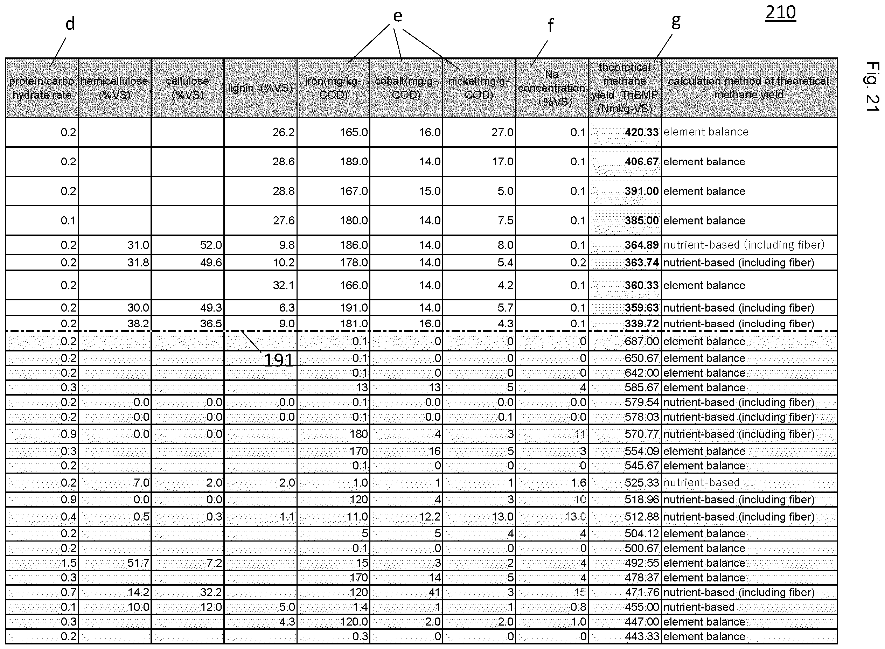

In this analysis, the mixed biogas materials that meet the minimum required conditions (a, b, e, and f) are listed above the dashed line in the tables, with combinations arranged in descending order of methane yield. Materials listed below the dashed line 191 are considered inappropriate for mixing, even if they result in a higher methane yield, because they fail to meet one or more of the required conditions (a, b, e, or f). Specifically, as illustrated in FIG. 21, for the individual biogas material (potato waste) in this experimental example, nine raw materials satisfying the above conditions (a to f) were identified. These materials, ranked in descending order of methane yield, are as follows: rice stalk-BP, wheat stalk-BP, rape stalk-BP, maize stalk-BP, wheat straw (5.0 cm), barley straw (5.0 cm), cotton stalk-BP, maize stalks (2.0 cm), and rice straw (5.0 cm). Among these, "rice stalk-BP" was determined to have particularly high compatibility for biogas production.

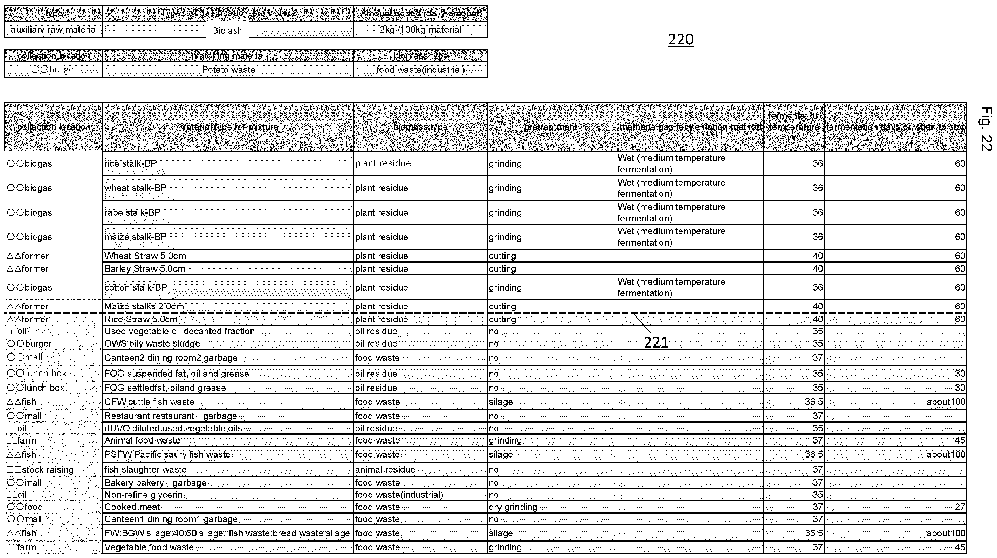

Furthermore, even if certain mixed biogas materials do not initially satisfy all of the required conditions (a to f), it is possible for them to meet these conditions with the addition of auxiliary agents such as "gasification promoters," "nutritional supplements," or "auxiliary raw materials." To evaluate this possibility, further estimations were performed by adding a gasification promoter (bioash, in this case, at a ratio of 2 kg per 100 kg of raw material) to the previously estimated evaluation values of the mixed biogas materials shown in FIGS. 19 through 21. The updated estimated evaluation values are presented in tables 220, 230, and 240 in FIGS. 22 through 24, respectively (FIGS. 22 through 24 are displayed as side-by-side tables).

In this case, the mixed biogas materials that meet all the conditions (a to f) are displayed above the dashed line, and the combinations yielding the highest methane production are listed in descending order. It was determined that the materials listed below dashed line 221 do not form a suitable mixture, as they fail to satisfy any of the conditions a through f, even if the methane yield is high.

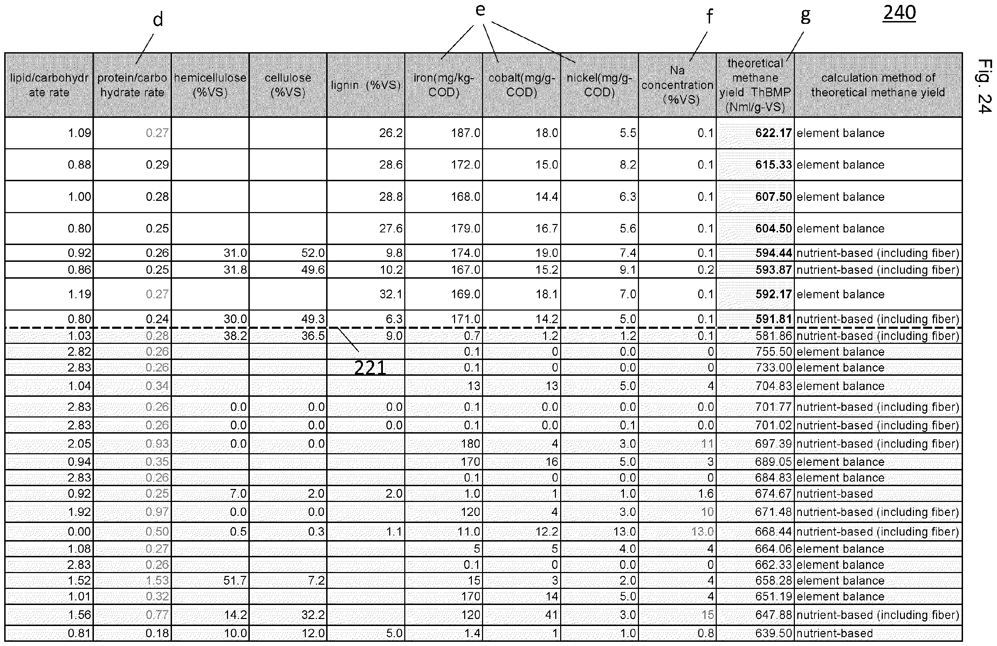

As illustrated in FIG. 24, for the individual biogas material (potato waste) and the gasification promoter (bioash, added at a ratio of 2 kg per 100 kg of raw material) used in this experimental example, eight raw materials satisfied all the conditions (a to f). These materials are rice stalk-BP, wheat stalk-BP, rape stalk-BP, maize stalk-BP, wheat straw (5.0 cm), barley straw (5.0 cm), cotton stalk-BP, and maize stalks (2.0 cm). Among these, "rice stalk-BP" was identified as particularly compatible with biogas generation.

Furthermore, it was estimated that the methane yield would exceed the previously estimated value when no gasification promoter was used (methane yield without gasification promoter: 420.33; methane yield with gasification promoter: 622.17). Based on this analysis, for the individual biogas material "potato waste," the optimal condition for enhanced biogas production was determined to involve mixing it with rice stalk-BP and incorporating a gasification promoter.

As described above, this invention pertains to an evaluation value calculation device (Device 1) for biogas materials that calculates evaluation values related to resource recovery when biogas is produced using at least one or more types of biogas materials. The device includes the following components: an input unit (111), which receives input data including a machine learning library (1L) related to biogas materials, property data concerning biogas materials, or operational status data from a biogas plant; a machine learning model

(112), designed to accept inputs from the input unit, such as the machine learning library (1L), property data, or operational status data, and to utilize this information for learning and estimation processes; a learning process execution unit (101), which trains and adjusts the parameters of the machine learning model (112) based on the machine learning library (1L); and an evaluation value calculation execution unit (102), which utilizes the machine learning model (112) to estimate evaluation values related to the resource recovery of biogas materials based on property data or operational status data.

The property data includes at least one of the following: type; collection location; properties; or quantity of biomass materials.

The evaluation values include at least one of the following indicators related to mixed biogas materials when individual biogas materials or combinations thereof are mixed: (a) C/N ratio (the ratio of carbon concentration to nitrogen concentration); (b) Lipid/VS ratio (the ratio of lipid concentration to volatile solids concentration); (c) Lipid/Carbohydrate ratio; (d) Protein/Carbohydrate ratio; (e) Micronutrient concentrations, such as iron (mg/kg-COD feedstock), cobalt (mg/g-COD feedstock), and nickel (mg/g-COD feedstock); (f) Sodium (Na) concentration; (g) Theoretical methane yield (ThBMP: ml/g-VS).

With this configuration, the evaluation value calculation device for biogas materials enables the active utilization of biogas resources by providing specific evaluation values. This invention addresses key challenges, such as the lack of prior diagnostics for biogas materials and the absence of reliable evaluation values for ensuring stable operation of biogas plants-both of which have hindered the use of food waste and other materials as biogas feedstocks.

Furthermore, by leveraging the estimated evaluation values, the invention facilitates optimal matching of food waste, residual sludge, food loss, and similar resources with biogas plants that perform methane gasification. Through the methane resource matching system accessible via the Internet, the invention enhances the operating rate of biogas plants and contributes significantly to achieving a decarbonized society.

With this configuration, the evaluation value calculation device 1 for biogas materials facilitates the more effective utilization of biogas resources by providing specific and actionable evaluation values. Consequently, this invention addresses key challenges, including the absence of prior diagnostic tools for biogas materials and the lack of reliable evaluation metrics to ensure the stable operation of power plants that utilize such materials. These challenges have historically hindered the adoption of food waste and similar materials as viable sources for biogas production.

Moreover, leveraging the estimated evaluation values, the invention enables the optimal pairing of food sources: such as residual sludge; food loss; and other waste materials, with biogas plants that employ methane gasification processes for food waste. This matching process is facilitated through the methane resource matching system, accessible via the Internet. By improving the operational efficiency of biogas plants, this innovation contributes significantly to advancing the transition toward a decarbonized society.

It is important to note that to achieve the objectives of the evaluation value calculation device, the present invention may also be implemented as a determination method comprising steps corresponding to the functional units included in the evaluation value calculation device. Additionally, the invention can be embodied as a program that enables a computer to execute these steps. This program may be distributed via a physical recording medium, such as a USB drive, or through a transmission medium, such as the Internet.

**Reference Signs List**

> 1 evaluation value calculation device
> 1P evaluation value calculation program
> 1L machine learning library
> 10 control unit
> 11 evaluation value calculation unit
> 12 storage unit
> 13 communication unit
> 101 learning process execution unit
> 102 evaluation value calculation execution unit
> 111 input unit
> 112 machine learning model
> 113 output unit

**Claims**

1. An evaluation value calculation device for calculating an evaluation value regarding resource recovery of biogas materials, the device comprising:

an input unit configured to receive input data regarding a machine learning library for biogas materials or property data related to biogas materials;
a machine learning model configured to receive the machine learning library or property data from the input unit;
a learning process execution unit configured to learn setting values within the machine learning model based on the machine learning library; and
an evaluation value calculation execution unit configured to estimate an evaluation value for resource recovery of biogas materials by performing an estimation process based on the property data using the machine learning model,
wherein the property data includes at least one of the following: type, collection location, property, and quantity of biomass materials.

2. The evaluation value calculation device for biogas materials according to claim 1,

wherein data regarding the operational status of a biogas plant is configured to be input via the input unit;
wherein the data regarding the operational status of the biogas plant is further processed by the machine learning model;
wherein the evaluation value calculation execution unit is configured to estimate an evaluation value regarding resource recovery of biogas materials by performing an estimation process based on both the property data and the data regarding the operational status of the biogas plant while utilizing the machine learning model; and
wherein the data regarding the operational status of the biogas plant includes at least one of the following: the type of biogas material used at the plant; the fermentation method; the presence and amount of additives, such as coagulants; the VS volume load per cubic meter of a methane fermentation tank; the organic decomposition rate in the methane fermentation tank; the methane gas generation amount in a gas holder; and the measured methane yield (BMP).

3. The evaluation value calculation device for biogas materials according to claim 1 or 2,
wherein the evaluation value estimated by the evaluation value calculation execution unit includes at least one of the following evaluation values regarding mixed biogas material when an individual biogas material or a plurality of individual materials are mixed:

(a) the C/N ratio, which represents the ratio of carbon concentration to nitrogen concentration;
(b) the lipid/VS ratio, which represents the ratio of lipid concentration to VS (Volatile Solids) concentration;
(c) the lipid/carbohydrate ratio;
(d) the protein/carbohydrate ratio;
(e) micronutrient concentrations, including iron (mg/kg-COD feedstock), cobalt (mg/g-COD feedstock), and nickel (mg/g-COD feedstock);
(f) the Na concentration; and
(g) the theoretical methane yield (ThBMP: ml/g-VS).

4. The evaluation value calculation device for biogas materials according to claim 3,
wherein the evaluation value calculation execution unit estimates at least one of the following evaluation values regarding mixed biogas materials:

(a) the C/N ratio being 25 or higher;
(b) the lipid/VS ratio being 35 or lower;
(c) the lipid/carbohydrate ratio being 13 or higher;
(d) the protein/carbohydrate ratio being 26 or higher;
(e) among micronutrients, whether iron is 165 mg/kg-COD raw material or higher, cobalt is 14 mg/g-COD raw material or higher, and nickel is 5 mg/g-COD raw material or higher;
(f) the Na concentration being less than 5.6 g/L; and
(g) the methane yield during mixed fermentation.

5. The evaluation value calculation device for biogas materials according to claim 4,
wherein the evaluation value calculation execution unit determines that the compatibility of mixed fermentation for mixed biogas materials improves as the methane yield described in (g) increases, provided that the conditions described in (a) to (f) are satisfied.

**6.** The evaluation value calculation device for biogas materials according to claim 4 or 5,
wherein the evaluation value calculation execution unit determines that when the methane yield is less than 100 mL/g-VS, the combination of biogas materials is unsuitable for mixed fermentation.

**7.** The evaluation value calculation device for biogas materials according to claim 4,
wherein the evaluation value calculation execution unit further evaluates the following conditions:

(a) A C/N ratio ranging from 25 to 27;
(b) A lipid/VS ratio of 35 or less, and simultaneously,
(c) a lipid/carbohydrate ratio of 13 or more;
(d) A protein/carbohydrate ratio of 26 or higher.

**8.** The evaluation value calculation device for biogas materials according to claim 3,
wherein the evaluation value calculation execution unit further predicts and determines appropriate types and quantities of gasification promoters, nutritional supplements, and auxiliary raw materials.

**9.** The evaluation value calculation device for biogas materials according to claim 3,
wherein the evaluation value calculation execution unit further estimates the following as part of the evaluation value: whether pretreatment of each biogas material is necessary; the organic substance composition of each methane material; fermentation temperature; fermentation duration; organic substance concentration (VS) of each methane material; solid substance amount (TS); organic substance ratio (VS/TS); ash concentration; chemical oxygen demand (COD); theoretical oxygen demand (ThOD); COD/VS value; organic substance constituent element concentrations of each methane material, including carbon (C:%VS), nitrogen (N:%VS), oxygen (O:%VS), and hydrogen (H:%VS); and hemicellulose, cellulose, and lignin concentrations (%VS) of each methane material.

**10.** The evaluation value calculation device for biogas materials according to claim 1,
wherein the machine learning library includes at least one of the following: whether pretreatment of individual biogas materials and mixed biogas materials is necessary; the organic substance composition of each methane material; fermentation temperature; fermentation duration; organic substance concentration (VS); solid substance amount (TS); organic substance ratio (VS/TS); ash concentration; chemical oxygen demand (COD); theoretical oxygen demand (ThOD); COD/VS value; organic substance constituent element concentrations, including carbon (C:%VS), nitrogen (N:%VS), oxygen (O:%VS), and hydrogen (H:%VS); carbohydrate, lipid, protein, ash, and other nutrient concentrations of each methane material; hemicellulose, cellulose, and lignin concentrations (%VS) of each methane material; C/N ratio (ratio of carbon concentration to nitrogen concentration of each methane material); lipid/VS ratio (ratio of lipid concentration to VS concentration of each methane material); Na concentration; concentrations of micronutrients such as iron (mg/kg-COD raw material), cobalt (mg/g-COD raw material), and nickel (mg/g-COD raw material) when materials are mixed; theoretical methane yield (ThBMP: ml/g-VS); actual methane yield (BMP: ml/g-VS) predicted value; biodegradation rate; VS decomposition rate; and energy yield.

**11.** The evaluation value calculation device for biogas materials according to claim 1, further comprising:

a communication unit configured to establish a communication connection to a communication network,
wherein the evaluation value regarding the resource recovery of biogas materials, estimated by the machine learning library and the evaluation value calculation execution unit, is transmitted via the communication unit.

**12.** A program for use with an evaluation value calculation device that calculates an evaluation value regarding the resource recovery of biogas materials, comprising:

an input step of receiving input regarding a machine learning library for biogas materials or property data related to biogas materials, and inputting the machine learning library or property data into a machine learning model;
a learning process execution step of learning setting values in the machine learning model based on the machine learning library; and
an evaluation value calculation execution step of estimating an evaluation value regarding the resource recovery of biogas materials by performing an estimation process based on the property data while using the machine learning model,
wherein the property data includes at least one of the following: type, collection location, property, and quantity of biomass materials.

**13.** The program according to claim 12,

wherein data regarding the operational status of a biogas plant is received in the input step;
wherein the data regarding the operational status of the biogas plant is input to the machine learning model;
wherein an evaluation value regarding resource recovery of biogas materials is estimated by performing an estimation process based on the property data and the data regarding the operational status of the biogas plant using the machine learning model in the evaluation value calculation execution step; and
wherein the data regarding the operational status of the biogas plant includes at least one of the following: the type of biogas material used at the plant; the fermentation method; the presence and amount of additives such as coagulants; the VS volume load per cubic meter of a methane fermentation tank; the organic decomposition rate in the methane fermentation tank; the amount of methane gas generated in a gas holder; and the measured methane yield (BMP).

**14.** An evaluation value calculation method for calculating an evaluation value regarding the resource recovery of biogas materials, comprising:

an input step for receiving a machine learning library or property data related to biogas materials, and inputting the received data into a machine learning model;
a learning process execution step for training the machine learning model using the machine learning library; and
an evaluation value calculation execution step for estimating an evaluation value regarding the resource recovery of biogas materials by performing an estimation process based on the property data using the machine learning model,
wherein the property data includes at least one of the following: type, collection location, properties, and quantity of biomass materials.

**15.** The evaluation value calculation method according to claim 14,

wherein data regarding the operational status of a biogas plant is input during the input step;
wherein the operational status data of the biogas plant is provided to the machine learning model;
wherein, during the evaluation value calculation execution step, an evaluation value regarding the resource recovery of biogas materials is estimated by performing an estimation process based on the property data and the operational status data of the biogas plant using the machine learning model; and
wherein the operational status data of the biogas plant includes at least one of the following: the type of biogas material used at the plant; the fermentation method; the presence and amount of additives such as coagulants; the VS volume load per cubic meter of the methane fermentation tank; the organic decomposition rate within the methane fermentation tank; the amount of methane gas generated in a gas holder; and the measured methane yield (BMP).

## Fig. 1

Food waste collecting process | Matching process | Methane gas production/consumption process

Fig. 2

EP 4 521 334 A1

EP 4 521 334 A1

## Fig. 3

Methane resource recovery estimation system — 400

IoT source investigation program
• source of food waste
• methane power plant — 401

AI methane resource recovery program
• individual material diagnosis
• Mixing & compatibility diagnosis — 402

AI gasification promotion program
optimal dosing calculation of promotor
Utilization of purified sludge/glycerin/rumen
bacteria, etc. — 403

CO2 reduction IT calculation program
• CO2 reduction amount
• credit calculation — 404

# Fig.4

(a)

## Biogasification promotor

<u>400</u>

| Auxiliary raw materials | Gases such as sludge and cow dung → Methane fermentation 30t/day → Gas 900m3/day | Gas yeild 30m3/t |
| | Raw materials with low gas yield such as sludge and cow dung 29t/day +Purified sludge 1t/day → Methane fermentation 30t/day → Gas 960m3/day | Gas yeild 32m3/t |
| Nutritional supplements | Food waste materials 30t/day → Methane fermentation 30t/day → Gas 4000m3/day | Gas yeild 133m3/t |
| | Food waste materials 30t/day + Ni, Co, e → Methane fermentation 30t/day → Gas 4800m3/day | The rate of increase in gas yield varies on a case-by-case basis depending on Ni and Co deficiency in the raw materials. |

(b)

<u>401</u>

| Gasification promotor | Variety | Condition | Biogas generation amount (m3/t-wet) | |
| --- | --- | --- | --- | --- |
| | | | Standard | When using promotor |
| Purified sludge | Auxiliary raw materials | Effective when there are many raw materials with low methane yield, such as sludge and livestock manure | 60-90 | None |
| Glycerin | Auxiliary raw materials | | 300 | None |
| Rumen bacteria | Promotor | Cellulose raw material | 150(paper) | 150-300(paper) |
| Nutritional supplement | Nutritional supplement | Food waste | 130(kitchen waste) | 130-160(kitchen waste) |

# Fig.5

## The whole flow

| Research and recovery at bioresource distributor and plant | Methane resource recovery promotion application |

Collection of methane materials (food waste, etc.)

Input information on the source and collected methane materials

Import of analysis and evaluation data for each source/plant

Linked with methane gas materials site investigation & matching program

Component analysis for each methane material and AI analysis and prediction of data such as methane yield

AI matching of methane resources collected from emission sources and methane resources used at each plant

Import of methane material matching analysis and evaluation data for each plant

Linked with the methane resource matching system

Investigation at methane gas plant

Input information on each methane gas plant and the methane materials used at the plant

**methane resource recovery evaluation AI 402a for individual materials**

※Based on the analysis data of methane materials registered in the master data, AI analyses the value of each material's component properties, methane yield, etc.

**methane resource recovery evaluation AI 402b for mixed materials, methane yield promotion prediction AI 403a (=gasification promotion program403)**

※Based on the analytical data, AI analysis is used to determine the combination of methane materials recovered from each emission source and materials used at each plant that will result in the most efficient methane yield.
※AI predicts methane yield when gasification promotor is added to each combination of methane materials

Fig.6

Login screen

Dashboard

Methane material matching | Methane Yield Analysis | Data management (Registering and updating various master data)

**AI methane material matching**

STEP-1
Import analysis data of methane materials for each emission source/plant using "methane resource recovery evaluation AI for individual material"

STEP-2
Methane materials collected from each emission source and materials used at each plant were analyzed using the "methane resource recovery evaluation AI for mixed materials"

STEP-3
The results of each material mixing pattern are analyzed using "methane yield promotion prediction AI "

STEP-4
Display of analytical results on the optimal mix of methane materials for each plant (based on conditions such as methane yield/material cost/energy yield/presence of promoters, etc.)

[System functions]

· System linkage with biogas material on-site survey & matching system
· AI automatic prediction of methane yield and other factors during mixed fermentation based on analysis data of individual methane materials for each emission source/plant
· AI automatic prediction of the amount of gasification promoter required for the mixed fermentation combination of each methane material
· Display & save analysis results
· Various CSV outputs

**AI methane yield analysis**

STEP-1
Enter information on methane materials for each emission source or plant

STEP-2
Analyze individual data such as the type and amount of each methane material
using "methane resource recovery evaluation AI for individual material"

STEP-3
Determine the evaluation value of v-collected materials as methane materials

STEP-4
Import analysis data for each methane material

STEP-5
Linked with the "Methane Resource Matching System"

[System functions]

· System linkage with biogas material on-site survey & matching system
· AI automatic analysis of material value such as component properties, methane yield (amount of methane gas generated), and energy yield for each material based on data on methane materials (material type, amount, etc.) at each emission source or plant
· Display & save analysis results
· Various CSV outputs

**Data Management**

Methane yield master per material

Methane yield master per promoter

Component index master data

Analysis and Evaluation Data Master

Experience Data Master

[System functions]

· Enter/create/edit/update various master data
· Reflect experience data into AI

EP 4 521 334 A1

30

## Fig.7

## Methane resource recovery evaluation AI 402a for individual materials

methane resource recovery
promotion program 402

Methane resource recovery evaluation AI 402a
for individual materials

(A)

Input basic data on
methane resources for
each source/each plant

(B)

Based on the analysis data for each
methane material recorded in the
master data, AI analyzes the value of
each waste as a methane material.

AI prediction and
analysis of data
including component
analysis of each
methane material and
methane yield

(C)

Display of prediction
and analysis results

Linked with methane resource
recovery evaluation AI for
mixed materials and
gasification promotion
program (data import)

Reflection of actual
measurement data on
methane yield for each
material used in plants, etc.

Component index
master data
(=analysis data on
methane yield for
each resource, such
as food waste)

· Analysis objects: Various methane samples (food waste/oil/sludge/livestock waste/animal and plant residues/mixed organic waste, etc.)
· Main analysis items : whether pretreatment of an individual biogas material and mixed biogas materials is necessary, the organic substance composition of each methane material, fermentation temperature, fermentation days, organic substance concentration (VS), solid substance amount (TS), organic substance ratio (VS/TS), ash concentration, chemical oxygen demand (COD), theoretical oxygen demand (ThOD), COD/VS value, organic substance constituent element concentration of each methane material (= content of carbon (C:%VS), nitrogen (N:%VS), oxygen (O:%VS), hydrogen (H:%VS), etc.), lipid, protein, ash and other nutrient concentrations of each methane material, hemicellulose, cellulose and lignin concentration (%VS) of each methane material, C/N ratio (ratio of carbon concentration to nitrogen concentration of each methane material), lipid/VS ratio (ratio of lipid concentration to VS concentration of each methane material), Na concentration, concentrations of micronutrients such as iron (mg/kg-COD raw material), cobalt (mg/g-COD raw material), and nickel (mg/g-COD raw material) when materials are mixed, theoretical methane yield (ThBMP: ml/g-VS), actual methane yield (BMP: ml/g-VS) predicted value, biodegradation rate, VS decomposition rate, and energy yield, etc.
· Theoretical methane yield was calculated using multiple calculation methods, including elemental balance, COD balance, and nutrient-based (including fiber) calculations.
· Reflecting actual measured data (empirical data) in AI predictions

# Fig.8

## Methane resource recovery evaluation AI 402b for mixed materials

| methane resource recovery promotion program 402 | methane resource recovery evaluation AI 402b for mixed materials |
|---|---|

AI prediction for most efficient combination of methane materials collected from each emission source and materials used at each plant based on index data

(D)

(E)

Import of methane resource analysis data per source/plant

AI prediction and analysis of methane yields when various methane materials are mixed and fermented

(F)

Display of prediction and analysis results

Linked with gasification promotion program (data import)

※analysis result by methane resource recovery evaluation AI for individual materials

Reflection of actual measurement data such as methane yield of each mixed material used in plants, etc.

Mixed index master data

· Analysis objects: Various methane samples (food waste/oil/sludge/livestock waste/animal and plant residues/mixed organic waste, etc.)
· Main analysis items : whether pretreatment of an individual biogas material and mixed biogas materials is necessary, the organic substance composition of each methane material, fermentation temperature, fermentation days, organic substance concentration (VS), solid substance amount (TS), organic substance ratio (VS/TS), ash concentration, chemical oxygen demand (COD), theoretical oxygen demand (ThOD), COD/VS value, organic substance constituent element concentration of each methane material (= content of carbon (C:%VS), nitrogen (N:%VS), oxygen (O:%VS), hydrogen (H:%VS), etc.), lipid, protein, ash and other nutrient concentrations of each methane material, hemicellulose, cellulose and lignin concentration (%VS) of each methane material, C/N ratio (ratio of carbon concentration to nitrogen concentration of each methane material), lipid/VS ratio (ratio of lipid concentration to VS concentration of each methane material), Na concentration, concentrations of micronutrients such as iron (mg/kg-COD raw material), cobalt (mg/g-COD raw material), and nickel (mg/g-COD raw material) when materials are mixed, theoretical methane yield (ThBMP: ml/g-VS), actual methane yield (BMP: ml/g-VS) predicted value, biodegradation rate, VS decomposition rate, and energy yield, etc.
· Multiple calculation methods are used to calculate the theoretical methane yield
· Reflecting actual measured data (empirical data) in AI predictions

# Fig.9

## methane yield promotion prediction AI 403a

| methane resource recovery promotion program 403 | methane yield promotion prediction AI 403a |

(G) Import of analytical data such as methane yield when each methane material is fermented individually (or mixed)

※analysis result by methane resource recovery evaluation AI for individual material
※analysis result by methane resource recovery evaluation AI for mixed materials
(Mixed materials are assumed to include mixtures of the same materials.)

(H) AI predicts data such as methane yield when gasification promotion materials are added to each mixed methane material

(I) Display of prediction and analysis results

Linked with methane resource matching system (data import)

AI prediction for the amount of gasification promoter required to increase methane yield/energy yield to a certain value for the mixture of methane materials collected from each emission source and materials used at each plant.

■Assumed database

Biogasification Promotion Index Master Data

· Analysis objects:Various methane materials (food waste/oil and fats/sludge/livestock waste/animal and plant residues/mixed organic waste, etc.) and gasification promoters (= auxiliary raw materials, promoters, nutritional supplements, etc.)
· Main analysis items : whether pretreatment of materials is necessary, the organic substance composition of each methane material, fermentation temperature, fermentation days, organic substance concentration (VS), solid substance amount (TS), organic substance ratio (VS/TS), ash concentration, chemical oxygen demand (COD), theoretical oxygen demand (ThOD), COD/VS value, organic substance constituent element concentration of each methane material (= content of carbon (C:%VS), nitrogen (N:%VS), oxygen (O:%VS), hydrogen (H:%VS), etc.), lipid, protein, ash and other nutrient concentrations of each methane material, hemicellulose, cellulose and lignin concentration (%VS) of each methane material, C/N ratio (ratio of carbon concentration to nitrogen concentration of each methane material), lipid/VS ratio (ratio of lipid concentration to VS concentration of each methane material), Na concentration, concentrations of micronutrients such as iron (mg/kg-COD raw material), cobalt (mg/g-COD raw material), and nickel (mg/g-COD raw material) when materials are mixed, theoretical methane yield (ThBMP: ml/g-VS), actual methane yield (BMP: ml/g-VS) predicted value, biodegradation rate, VS decomposition rate, and energy yield, etc.
· Multiple calculation methods are used to calculate the theoretical methane yield
· Reflecting actual measured data (empirical data) in AI predictions

Fig.10

Evaluation value calculation device 1

Control unit 10

Evaluation value calculation unit 11

Memory

Storage unit 12
1P
Evaluation value calculation program

1L
Machine learning library

Communication unit 13

Display unit 14

Operation unit 15

Reading unit 16

2

2P
Evaluation value calculation program

3L
Machine learning library

Fig.11

EP 4 521 334 A1

Fig.12

```
                              ┌─────────┐
                              │  Start  │
                              └─────────┘
                                   │
                                   ▼
          ┌──────────────►      ╱────────╲           S1201
          │       No      ╱ machine learning ╲
          │        ◄─────◄   library            ►
          │               ╲ of biogas material is ╱
          │                ╲     input?      ╱
          │                   ╲────────╱
          │                        │ Yes           S1202
          │                        ▼
          │              ┌────────────────────┐
          │              │ Output the machine │
          │              │ learning library to│
          │              │ the machine        │
          │              │ learning model     │
          │              └────────────────────┘
          │                        │              S1203
          │                        ▼
          │              ┌────────────────────┐
          │              │ Machine learning   │
          │              │ (generate          │
          │              │  parameters)       │
          │              └────────────────────┘
          │                        │              S1204
          │                        ▼
          │              ┌────────────────────┐
          │              │ Store in the       │
          │              │ storage unit       │
          │              │ (update setting    │
          │              │  values)           │
          │              └────────────────────┘
                                   │
                                   ▼
                              ┌─────────┐
                              │   End   │
                              └─────────┘
```

EP 4 521 334 A1

Fig.13

```
                    ┌──────────┐
                    │  Start   │
                    └────┬─────┘
      ┌──────────────────▼──────┐
      │            ◇            │ S1301
      │  ◇ Property data of ◇   │
 No   │  ◇  biogas material  ◇  │
◄─────┤  ◇    is input?      ◇  │
      └─────────────┬───────────┘
                    │ Yes    S1302
                    ▼
          ┌──────────────────┐
          │ Output the       │
          │ property data    │
          │ to the machine   │
          │ learning model   │
          └────────┬─────────┘
                   │        S1303
                   ▼
          ┌──────────────────┐
          │ Estimation       │
          │ process          │
          │ (estimate each   │
          │ evaluation value)│
          └────────┬─────────┘
                   │        S1304
                   ▼
          ┌──────────────────┐
          │ Output evaluation│
          │ values           │
          │ (store in the    │
          │ storage unit)    │
          └────────┬─────────┘
                   ▼
             ┌──────────┐
             │   End    │
             └──────────┘
```

| material types | biomass type | pretreatment | fermentation temperature (°C) | fermentation days or when to stop | TS (% Fresh material (FM)) | VS/TS (%) | Ash/TS (%) | COD/VS (%) | C (%VS) | N (%VS) | H (%VS) |
|---|---|---|---|---|---|---|---|---|---|---|---|
| Jatropha curcus L. mature leaf lamima | plant residue | dry grinding | 35 | 105 | 26.4 | 90.2 | 9.8 | | | | |
| Mature leaf petiole | plant residue | dry grinding | 35 | 105 | 12.7 | 89.4 | 10.6 | | | | |
| Mature leaf entire | plant residue | dry grinding | 35 | 105 | 21.5 | 88.7 | 11.3 | | | | |
| Tender leaf entire | plant residue | dry grinding | 35 | 105 | 25.6 | 90 | 10 | | | | |
| Green fruit | plant residue | dry grinding | 35 | 105 | 10.9 | 93 | 7 | | | | |
| Yellow fruit | plant residue | dry grinding | 35 | 105 | 13.3 | 92 | 8 | | | | |
| Brown fruit | plant residue | dry grinding | 35 | 105 | 91.2 | 92.8 | 7.2 | | | | |
| Fruit hull | plant residue | dry grinding | 35 | 105 | 87.8 | 91.6 | 8.4 | | | | |
| Seed testa | plant residue | dry grinding | 35 | 105 | 89.9 | 96.2 | 3.8 | | | | |
| Seed kernel | plant residue | dry grinding | 35 | 105 | 94.8 | 95.6 | 4.4 | | | | |
| Seed entire | plant residue | dry grinding | 35 | 105 | 91.1 | 95 | 5 | | | | |
| De-oiled cake | plant residue | dry grinding | 35 | 105 | 94.1 | 89.2 | 10.8 | | | | |
| Boild rice | food waste | dry grinding | 37 | 27 | 35 | 99 | 1 | | 41.3 | 1.6 | 7.6 |
| Cooked meat | food waste | dry grinding | 37 | 27 | 53 | 97 | 3 | | 55.1 | 10.8 | 8.7 |
| Fresh cabbage | food waste | dry grinding | 37 | 27 | 5 | 84 | 16 | | 47.5 | 4.9 | 6.4 |
| MFW | food waste | dry grinding | 37 | 27 | 26 | 95 | 5 | | 53.9 | 3.4 | 8.2 |
| Fresh Laminaria. Digitata(FD) | algae | dry grinding | 35 | 32 | 7.78 | 72.03 | 28 | | 37.9 | 4.4 | 5.2 |
| Fresh Laminaria. Hyperborea(FHY) | algae | dry grinding | 35 | 32 | 6.16 | 69.08 | 30.9 | | 37.1 | 1.9 | 5.2 |
| Dried Laminaria. Digitata(DD) | algae | dry grinding | 35 | 32 | 98.71 | 69.04 | 31 | | 43.6 | 3.1 | 6.9 |
| Dried Laminaria. Hyperborea(DHY) | algae | dry grinding | 35 | 32 | 97.92 | 63.19 | 36.8 | | 45.4 | 1.6 | 6.7 |
| Wheat straw_raw | plant residue | no | 35.1 | 30 | 91.624 | 89.4 | 10.6 | 1.405 | | | |
| Sugarcane bagasse_raw | plant residue | no | 35.1 | 30 | 91.922 | 98.8 | 1.2 | 1.309 | | | |
| BGW brewery grain waste | food waste(industrial) | silage | 36.5 | 100 | 24.2 | 95 | 5 | | | | |
| BW bread waste | food waste | silage | 36.5 | 100 | 67.4 | 97.2 | 2.8 | | | | |
| PSFW Pacific saury fish waste | food waste | silage | 36.5 | 100 | 31.4 | 88 | 12 | | | | |
| MFW mackerel fish waste; | food waste | silage | 36.5 | 100 | 35.6 | 86 | 14 | | | | |
| CFW cuttle fish waste | food waste | silage | 36.5 | 100 | 38.5 | 94 | 6 | | | | |
| FW:BGW silage 40:60 silage, fish waste:bread waste silage | food waste | silage | 36.5 | 100 | 26.3 | 93.9 | 6.1 | | | | |
| Mature fresh leaf-entire,untreated | plant residue | dry grinding | 35 | 100 | 21.3 | 93.6 | 6.4 | 1.08 | 37.1 | 1.6 | |
| Yellow leaf-entire,untreated | plant residue | dry grinding | 35 | 100 | 65.8 | 89.2 | 10.8 | 1.38 | 35.8 | 1.2 | |
| Inflorescence, untreated | plant residue | dry grinding | 35 | 100 | 18 | 92.9 | 7.1 | 1.18 | 32.3 | 1.4 | |
| Petal, untreated | plant residue | dry grinding | 35 | 100 | 15.3 | 95.1 | 4.9 | 1.07 | 45.3 | 3.8 | |
| Pod hask, untreated | plant residue | dry grinding | 35 | 100 | 89 | 85.9 | 14.1 | 1.39 | 41.4 | 2.3 | |
| Seed, untreated | plant residue | dry grinding | 35 | 100 | 91.5 | 95.7 | 4.3 | 1.38 | 40.1 | 3.8 | |
| Press cake, untreated | plant residue | dry grinding | 35 | 100 | 94.5 | 90 | 10 | 1.24 | 40.6 | 2.8 | |
| Bianco avorio_S2 | resource crop | silage | 40 | 27 | 35.4 | 88 | 12 | | | | |
| Altolis_S2 | resource crop | silage | 40 | 27 | 32.8 | 88.1 | 11.9 | | | | |
| Sylvestris Marsala S3 | resource crop | silage | 40 | 27 | 33 | 86.6 | 13.4 | | | | |
| Bianco avorio_S3 | resource crop | silage | 40 | 27 | 37.1 | 89.2 | 10.8 | | | | |
| Altolis_S3 | resource crop | silage | 40 | 27 | 34.6 | 88.6 | 11.4 | | | | |
| OFMSW_M1 | mixed organic waste | no | 37 | 21 | 9.2 | 96.5 | 3.5 | | | | |
| OFMSW_M2 | mixed organic waste | no | 37 | 21 | 10 | 97.8 | 2.2 | | | | |
| Solid fraction from pig manure_No.18 | livestock waste | no | 35 | 100 | 29.06 | 70.32 | 29.7 | | | | |
| Solid fraction from pig manure_No.19 | livestock waste | no | 35 | 100 | 30.9 | 80.74 | 19.3 | | | | |
| Solid fraction from pig manure_No.20 | livestock waste | no | 35 | 100 | 28.5 | 81.2 | 18.8 | | | | |
| Solid fraction from pig manure_No.21 | livestock waste | no | 35 | 100 | 19.8 | 63.791 | 36.2 | | | | |
| Straw_No.22 | plant residue | no | 35 | 100 | 90.54 | 95.9 | 4.1 | | | | |
| cattle manure 1 | livestock waste | no | 35 | 100 | 14.375 | 90.27 | 9.7 | | | | |
| cattle manure 2 | livestock waste | no | 35 | 100 | 13.834 | 89.95 | 10.1 | | | | |
| pig fatteners 8 | animal waste | no | 35 | 100 | 20.669 | 82.1 | 17.9 | | | | |

Fig. 14

EP 4 521 334 A1

Fig. 15

150

| O (%VS) | total carbohydrate (%VS) | lipid (%VS) | protein (%VS) | hemicellulose (%VS) | cellulose (%VS) | lignin (%VS) | actual methane yield BMP (Nml/g-VS) | theoretical methane yield ThBMP (Nml/g-VS) | calculation method of theoretical methane yield | biodegradation rate (%) BMP/TBMP | VS decomposition rate (measured) |
|---|---|---|---|---|---|---|---|---|---|---|---|
|  |  |  |  |  | 31 | 20 | 227 |  |  |  |  |
|  |  |  |  |  | 32.4 | 13.4 | 335 |  |  |  |  |
|  | 65.1 | 0 | 15.8 |  | 33.8 | 18 | 237 |  |  |  |  |
|  |  |  |  |  | 34.4 | 14.4 | 224 |  |  |  |  |
|  |  |  |  |  | 30.1 | 9 | 326 |  |  |  |  |
|  |  |  |  |  | 32.6 | 9.8 | 518 |  |  |  |  |
|  | 79 | 0 | 5.8 |  | 30.2 | 9.7 | 469 |  |  |  |  |
|  |  |  |  |  | 39.3 | 14.2 | 306 |  |  |  |  |
|  |  |  |  |  | 62.4 | 8.3 | 80 |  |  |  |  |
|  | 38.7 | 36.8 | 19.2 |  | 7.3 | 5.2 | 968 |  |  |  |  |
|  | 72 | 1.7 | 10.5 |  | 27.4 | 4.2 | 610 |  |  |  |  |
|  |  |  |  |  | 36.9 | 5.6 | 230 |  |  |  |  |
| 1.6 |  |  |  |  |  |  | 294 | 407.1 | element balance | 72.2 |  |
| 10.8 |  |  |  |  |  |  | 482 | 573.7 | element balance | 84 |  |
| 4.9 |  |  |  |  |  |  | 277 | 321.9 | element balance | 86.1 |  |
| 3.4 |  |  |  |  |  |  | 472 | 544.8 | element balance | 86.6 |  |
| 52.1 |  |  | 19.4 |  |  |  | 93.35 | 335.36 | element balance | 27.8 |  |
| 50.9 |  |  | 13 |  |  |  | 105.06 | 334.74 | element balance | 31.4 |  |
| 54.4 |  |  | 27.5 |  |  |  | 141.45 | 393.73 | element balance | 35.9 |  |
| 55.6 |  |  | 15.8 |  |  |  | 113.28 | 384.16 | element balance | 29.5 |  |
|  |  |  |  | 24.8 | 39.4 | 24.7 | 224 | 450.5 | COD balance | 49.7 |  |
|  |  |  |  | 21.1 | 46.8 | 23 | 222 | 421.3 | COD balance | 52.7 |  |
|  |  | 9.2 | 27.5 | 20.3 | 45.9 |  | 316 | 503.7 | nutrient-based (including fiber) | 62.7 | 79 |
|  |  | 2.1 | 12.5 | 0 | 0 |  | 306 | 437.4 | nutrient-based (including fiber) | 70 | 95 |
|  |  | 41.4 | 45.8 | 0 | 0 |  | 435 | 699.9 | nutrient-based (including fiber) | 62.2 | 91 |
|  |  | 57.6 | 48.1 | 0 | 0 |  | 526 | 798.7 | nutrient-based (including fiber) | 65.9 | 79 |
|  |  | 49.7 | 42.9 | 0 | 0 |  | 543 | 747.3 | nutrient-based (including fiber) | 72.7 | 84 |
|  |  | 18.5 | 32.5 | 14.2 | 32.2 |  | 441 | 552.3 | nutrient-based (including fiber) | 79.9 | 71 |
|  | 10.1 | 7.4 | 10.8 | 23 | 43 | 5 | 316 | 378.4 | COD balance | 83.5 |  |
|  | 12.9 | 9.9 | 7.8 | 8 | 55 | 6 | 187 | 481.7 | COD balance | 38.8 |  |
|  | 17.4 | 10.2 | 8.6 | 36 | 21 | 7 | 453 | 413.4 | COD balance | 109.6 |  |
|  | 18.9 | 4.9 | 23.9 | 30 | 14 | 8 | 496 | 375.7 | COD balance | 132 |  |
|  | 19.7 | 0 | 14.4 | 6 | 51 | 9 | 248 | 485.1 | COD balance | 51.1 |  |
|  | 27.4 | 26.5 | 23.1 | 14 | 5 | 4 | 788 | 482.1 | COD balance | 163.5 |  |
|  | 24.7 | 7.8 | 17.5 | 39 | 8 | 3 | 625 | 435 | COD balance | 143.7 |  |
|  |  | 3.2 | 14.5 | 13.1 | 37.7 |  | 243.3 | 445.8 | nutrient-based (including fiber) | 54.6 |  |
|  |  | 2.8 | 16.1 | 22.6 | 31.9 |  | 196 | 445.1 | nutrient-based (including fiber) | 44 |  |
|  |  | 2.7 | 16.1 | 16.3 | 38.5 |  | 248.7 | 443.9 | nutrient-based (including fiber) | 56 |  |
|  |  | 2.7 | 15 | 16.8 | 36.3 |  | 248.7 | 443.3 | nutrient-based (including fiber) | 56.1 |  |
|  |  | 2.3 | 16.5 | 9.6 | 42.2 |  | 213.6 | 441.9 | nutrient-based (including fiber) | 48.3 |  |
|  | 36.4 | 12.1 | 18.4 |  |  |  | 172.1 |  |  |  |  |
|  | 26.8 | 6.5 | 10.7 |  |  |  | 119.9 |  |  |  |  |
|  | 51.9 | 12 | 24.2 |  |  | 10.5 | 261 | 508 | element balance | 51.4 |  |
|  | 55.2 | 15.1 | 18 |  |  | 10.5 | 159 | 521 | element balance | 30.5 |  |
|  | 54.4 | 13.1 | 23.5 |  |  | 9.1 | 247 | 514 | element balance | 48.1 |  |
|  | 35.5 | 17.1 | 30.3 |  |  | 1.3 | 506 | 542 | element balance | 93.4 |  |
|  | 85.9 | 2.4 | 3.8 |  |  | 7.9 | 195 | 432 | element balance | 45.1 |  |
|  | 71.1 | 7.1 | 7.6 |  |  | 13.1 | 100 | 464 | element balance | 21.6 |  |
|  | 68.2 | 5.4 | 15.8 |  |  | 7.5 | 124 | 460 | element balance | 27 |  |
|  | 46.4 | 15.2 | 26.1 |  |  | 4.4 | 359 | 528 | element balance | 68 |  |

Fig. 16

160

| collection location | material type | biomass type | pretreatment | methene gas fermentation method | fermentation temperature (°C) | fermentation days or when to stop | VS (% Fresh material (FM)) |
|---|---|---|---|---|---|---|---|
| ○○food | Boild rice | food waste | dry grinding | | 37 | 27 | 34.65 |
| ○○food | Cooked meat | food waste | dry grinding | | 37 | 27 | 51.41 |
| ○○food | Fresh cabbage | food waste | dry grinding | | 37 | 27 | 4.2 |
| □□plant | Wheat straw_raw | plant residue | no | Wet (medium temperature ferment | 35.1 | 30 | 81.883 |
| △△fish | PSFW Pacific saury fish waste | food waste | silage | | 36.5 | about100 | 27.63 |
| △△fish | CFW cuttle fish waste | food waste | silage | | 36.5 | about100 | 36.19 |
| △△fish | FW:BGW silage 40:60 silage, fish waste:bread waste silag | food waste | silage | | 36.5 | about100 | 24.6957 |
| ○○dairy | Solid fraction from pig manure_No.18 | livestock waste | no | | 35 | 100 | 20.434992 |
| ○○dairy | Solid fraction from pig manure_No.19 | livestock waste | no | | 35 | 100 | 24.94866 |
| ○○dairy | pig fatteners 9 | animal residue | no | | 35 | 100 | 19.81566 |
| ○○lunch box | FOG suspended fat, oil and grease | oil residue | no | | 35 | 30 | 22.97 |
| ○○lunch box | FOG settledfat, oiland grease | oil residue | no | | 35 | 30 | 11.26 |
| ○○lunch box | Salad | food waste | grinding | | 55 | about20 | 8.72 |
| ○○lunch box | Carrots | food waste | grinding | | 55 | about20 | 12.1365 |
| ○○lunch box | Grass | food waste | grinding | | 55 | about20 | 26.746 |
| ○○lunch box | Potato | food waste | grinding | | 55 | about20 | 17.803 |
| ○○lunch box | Banana | food waste | grinding | | 55 | about20 | 10.9056 |
| ○○lunch box | Apple | food waste | grinding | | 55 | about20 | 16.7409 |
| ○○lunch box | Orange | food waste | grinding | | 55 | about20 | 21.7864 |
| ○○lunch box | Biological sludge | sludge | no | | 37 | 39 | 5.69 |
| □□farm | Animal food waste    161 | food waste | grinding | | 37 | 45 | 34.44 |
| □□farm | Vegetable food waste | food waste | grinding | | 37 | 45 | 22.32 |
| ○○burger | Potato waste | food waste(industrial) | no | | 35 | | 19.05 |
| ○○burger | Waste sludge from a potato residue treatment plant | sludge | no | | 35 | | 9.42 |
| ○○burger | Waste sludge from a fruit residue treatment plant | sludge | no | | 35 | | 6.87 |
| ○○burger | OWS oily waste sludge | oil residue | no | | 35 | | 18.03 |
| □□oil | Non-refine glycerin | food waste(industrial) | no | | 35 | | 49.61 |
| □□oil | dUVO diluted used vegetable oils | oil residue | no | | 35 | | 71.63 |
| □□oil | Used vegetable oil decanted fraction | oil residue | no | | 35 | | 69.79 |
| T B M | Waste sludge from SMO biofuel processing | purified sludge | no | | 35 | | 86.49987225 |
| △△former | Barley Straw 5.0cm | plant residue | cutting | | 40 | 60 | 85.3415 |
| △△former | Wheat Straw 5.0cm | plant residue | cutting | | 40 | 60 | 82.8762 |

Fig. 17

170

| TS (% Fresh material (FM)) | VS/TS (%) | Ash/TS (%) | COD/VS (%) | C (%VS) | N (%VS) | H (%VS) | O (%VS) | C/N (%VS) (a) | total carbohydrate (%VS) | lipid (%VS) | protein (%VS) | lipid/VS rate (b) | lipid/carbohydrate rate (c) |
|---|---|---|---|---|---|---|---|---|---|---|---|---|---|
| 35 | 99 | 1.0 | 1.26 | 41.3 | 1.6 | 7.6 | 1.6 | 25.6 | 21.1 | 0 | 2.6 | 0.0 | 0.0 |
| 53 | 97 | 3.0 | 1.65 | 55.1 | 10.8 | 8.7 | 10.8 | 5.1 | 0.0 | 15.9 | 81.5 | 0.3 | 0.0 |
| 5 | 84 | 16.0 | 1.3 | 47.5 | 4.9 | 6.4 | 4.9 | 9.7 | 44.6 | 1.6 | 17.8 | 0.4 | 0.0 |
| 91.624 | 89.4 | 10.6 | 1.405 | 47.5 | 0.8 | 6.0 | 45.6 | 60.9 | 85.9 | 1.8 | 3.3 | 0.0 | 0.0 |
| 31.4 | 88.0 | 12.0 | 1.073 | 52.7 | 10.4 | 7.9 | 28.5 | 5.0 | 0 | 41.4 | 45.8 | 1.5 | 0.0 |
| 38.5 | 94.0 | 6.0 | 1.133 | 58.0 | 7.9 | 8.1 | 25.3 | 7.4 | 0 | 49.7 | 42.9 | 1.4 | 0.0 |
| 26.3 | 93.9 | 6.1 | 1.110 | 52.7 | 10.4 | 7.9 | 28.5 | 5.0 | 0 | 18.5 | 32.5 | 0.7 | 0.2 |
| 29.06 | 70.32 | 29.7 | 1.3 | 63.2 | 5.6 | 10.2 | 20.9 | 11.2 | 51.9 | 12.0 | 24.2 | 0.6 | 0.3 |
| 30.9 | 80.74 | 19.3 | 1.3 | 60.2 | 4.8 | 9.4 | 20.9 | 12.5 | 55.2 | 15.1 | 18.0 | 0.6 | 0.3 |
| 23.34 | 84.9 | 15.1 | 1.3 | 64.2 | 4.6 | 9.2 | 20.9 | 13.9 | 49.4 | 13.1 | 26.7 | 0.7 | 0.3 |
| 26.72 | 86.0 | 14.0 | 2.61 | 67.5 | 3.7 | 10.0 | 17.8 | 18.1 | 0.0 | 100.0 | 0.0 | 4.4 |  |
| 12.84 | 87.7 | 12.3 | 2.58 | 67.0 | 1.3 | 10.0 | 21.6 | 53.4 | 0.0 | 100.0 | 0.0 | 8.9 |  |
| 10.9 | 80 | 20.0 | 1.460 | 45.2 | 0.2 | 6.7 | 42.0 | 222.4 | 26.3 | 8.1 | 19.9 | 0.9 | 0.3 |
| 13.5 | 89.9 | 10.1 | 1.400 | 55.9 | 4.6 | 7.1 | 31.7 | 12.1 | 47.2 | 5.0 | 20.8 | 0.4 | 0.1 |
| 31.1 | 86 | 14.0 | 1.430 | 50.7 | 3.1 | 6.9 | 39.4 | 16.6 | 26.3 | 6.6 | 15 | 0.2 | 0.3 |
| 19 | 93.7 | 6.3 | 1.280 | 45.0 | 1.2 | 6.3 | 47.5 | 38.9 | 60.9 | 3.2 | 9 | 0.2 | 0.1 |
| 12.8 | 85.2 | 14.8 | 1.520 | 49.4 | 1.6 | 6.7 | 42.3 | 30.1 | 45 | 11.8 | 10.2 | 1.1 | 0.3 |
| 17.1 | 97.9 | 2.1 | 1.360 | 48.0 | 2.2 | 7.6 | 42.1 | 21.6 | 51.4 | 2.2 | 12.3 | 0.1 | 0.0 |
| 22.6 | 96.4 | 3.6 | 1.350 | 52.3 | 1.9 | 8.2 | 37.8 | 28.3 | 46.8 | 3.9 | 16.9 | 0.2 | 0.1 |
| 6.98 | 81.5 | 18.5 | 1.355 | 6.6 | 0.7 | 11.2 | 44.7 | 9.0 | 0.0 | 0.2 | 7.9 | 0.0 | 0.5 |
| 41 | 84.0 | 16.0 | 1.08 | 57.6 | 4.0 | 8.2 | 30.1 | 14.4 | 52.0 | 25.0 | 12.0 | 0.7 | 0.3 |
| 24 | 93.0 | 7.0 | 1.38 | 43.82 | 2.69 | 6.51 | 47.0 | 16.3 | 53.0 | 14.0 | 5.0 | 0.6 | 0.3 |
| 19.86 | 95.9 | 4.1 | 1.18 | 45.0 | 1.2 | 6.3 | 47.5 | 38.9 | 60 | 15.0 | 10.5 | 0.8 | 0.3 |
| 13.52 | 69.7 | 30.3 | 1.07 | 50.9 | 9.0 | 7.2 | 30.8 | 5.6 | 32.2 | 13.0 | 6.9 | 1.4 | 0.4 |
| 7.65 | 89.8 | 10.2 | 1.39 | 50.1 | 4.4 | 6.6 | 37.6 | 11.4 | 12.4 | 4.9 | 4.4 | 0.7 | 0.4 |
| 24.24 | 74.4 | 25.6 | 1.38 | 67.5 | 3.7 | 10.0 | 17.8 | 18.1 | 0 | 100.0 | 0 | 5.5 |  |
| 54.67 | 90.7 | 9.3 | 1.24 | 51.8 | 0.1 | 10.2 | 37.9 | 784.0 | 0 | 100.0 | 0 | 2.0 |  |
| 75.4 | 95.0 | 5.0 | 1.45 | 61.8 | 1.3 | 6.0 | 30.7 | 48.5 | 0 | 100.0 | 0 | 1.4 |  |
| 72.36 | 96.4 | 3.6 | 1.39 | 72.4 | 0.6 | 10.7 | 16.2 | 120.4 | 0 | 99.0 | 0 | 1.4 |  |
| 90.5 | 95.6 | 4.4 | 1.36 | 67.0 | 1.3 | 10.0 | 21.6 | 53.4 | 4 | 40.0 | 0 | 0.5 | 10.0 |
| 90.5 | 94.3 | 5.7 | 1.07 | 47.5 | 4.9 | 6.4 | 4.9 | 9.7 | 31.0 | 0.8 | 6.6 | 0.0 | 0.0 |
| 86.6 | 95.7 | 4.3 | 1.68 | 48.7 | 5.0 | 6.7 | 4.9 | 9.7 | 26.3 | 1.8 | 3.3 | 0.0 | 0.1 |

Fig. 18

EP 4 521 334 A1

(a)

| protein/carbo hydrate rate | hemicellulose (%VS) | cellulose (%VS) | lignin (%VS) | iron(mg/kg-COD) | cobalt(mg/g-COD) | nickel(mg/g-COD) | Na concentration (%VS) | theoretical methane yield TBMP (Nml/g-VS) | calculation method of theoretical methane yield |
|---|---|---|---|---|---|---|---|---|---|
| 0.1 | 0 | 0.3 | | 10 | 1 | 1 | 0 | 407.1 | element balance |
| 0.0 | 51.7 | 7.2 | | 15 | 3 | 2 | 4 | 573.7 | element balance |
| 0.4 | 0 | 36.2 | | 8 | 14 | 3 | 2 | 321.9 | element balance |
| 0.0 | 24.8 | 39.4 | 24.7 | 1.0 | 2.0 | 2.0 | 0.4 | 450.5 | COD balance |
| 0.0 | 0.0 | 0.0 | | 120 | 4 | 3 | 10 | 699.9 | nutrient-based (including fiber) |
| 0.0 | 0.0 | 0.0 | | 180 | 4 | 3 | 11 | 747.3 | nutrient-based (including fiber) |
| 0.0 | 14.2 | 32.2 | | 120 | 41 | 3 | 15 | 552.3 | nutrient-based (including fiber) |
| 0.5 | | | 10.5 | 80.0 | 2.0 | 2.0 | 1.0 | 508 | element balance |
| 0.3 | | | 10.5 | 98.0 | 2.0 | 2.0 | 0.8 | 521 | element balance |
| 0.5 | | | 4.3 | 120.0 | 2.0 | 2.0 | 1.0 | 516 | element balance |
| 0.0 | 0.0 | 0.0 | 0.0 | 0.1 | 0.0 | 0.0 | 0.0 | 914.3 | nutrient-based (including fiber) |
| | 0.0 | 0.0 | 0.0 | 0.1 | 0.0 | 0.1 | 0.0 | 898.7 | nutrient-based (including fiber) |
| 0.8 | 15.5 | 12.6 | 9.6 | 0.5 | 1.2 | 0.3 | 2.4 | 506.9 | COD balance |
| 0.4 | 8.5 | 7.7 | 6.1 | 0.4 | 1 | 1 | 0 | 491.1 | COD balance |
| 0.6 | 36.1 | 15.3 | 8.3 | 0.3 | 1 | 1 | 0.8 | 503.9 | COD balance |
| 0.1 | 29.5 | 3.9 | 1.9 | 0.3 | 1 | 1 | 0 | 448.3 | COD balance |
| 0.2 | 13.3 | 7.7 | 17.4 | 0.6 | 1 | 1 | 0 | 535.2 | COD balance |
| 0.2 | 2.9 | 4.7 | 7.5 | 0.4 | 1 | 1 | 0 | 473.1 | COD balance |
| 0.4 | 4.5 | 7.8 | 4 | 0.4 | 1 | 1 | 0 | 471.4 | COD balance |
| | | | | 1.2 | 1 | 1 | 0.6 | 535.8 | COD balance |
| 0.2 | 7.0 | 2.0 | 2.0 | 1.0 | 1 | 1 | 1.6 | 582 | nutrient-based |
| 0.1 | 10.0 | 12.0 | 5.0 | 1.4 | 1 | 1 | 0.8 | 518 | nutrient-based |
| 0.2 | | | | 2 | 1 | 2 | 3 | 422 | element balance |
| 0.2 | | | | 0.5 | 0 | 0 | 0 | 510 | element balance |
| 0.4 | | | | 0.3 | 0 | 0 | 0 | 493 | element balance |
| | | | | 0.1 | 0 | 0 | 0 | 824 | element balance |
| | | | | 0.1 | 0 | 0 | 0 | 635 | element balance |
| | | | | 0.1 | 0 | 0 | 0 | 629 | element balance |
| | | | | 0.1 | 0 | 0 | 0 | 914 | element balance |
| 0.0 | | | | 0.1 | 0 | 0 | 0 | 824 | element balance |
| 0.2 | 31.8 | 49.6 | 10.2 | 1.0 | 1.0 | 1.2 | 0.2 | 383.2 | nutrient-based (including fiber) |
| 0.1 | 31.0 | 52.0 | 9.8 | 0.8 | 1.0 | 1.2 | 0.1 | 387.7 | nutrient-based (including fiber) |

(b)

| measured methane yield BMP (Nml/g-VS) | biodegradation rate (%) BMP/TBMP | VS decomposition rate (measured) |
|---|---|---|
| 294 | 72.2 | |
| 482 | 84.0 | |
| 277 | 86.1 | |
| 224 | 49.7 | |
| 435 | 62.2 | 91 |
| 543 | 72.7 | 84 |
| 441 | 79.9 | 71 |
| 261 | 51.4 | |
| 159 | 30.5 | |
| 403 | 78.1 | |
| 402.3 | 44.0 | |
| 413.4 | 46.0 | |
| 294 | 58.0 | |
| 388 | 79.0 | |
| 388 | 77.0 | |
| 390 | 87.0 | |
| 289 | 54.0 | |
| 317 | 67.0 | |
| 297 | 63.0 | |
| 164.5 | 30.7 | |
| 572 | 98.3 | |
| 425 | 82.0 | |
| 345 | 81.8 | |
| 397 | 77.8 | |
| 415 | 84.2 | |
| 680 | 82.5 | |
| 445 | 70.1 | |
| 586 | 93.2 | |
| 725 | 79.3 | |
| 706 | 85.7 | |
| 286 | 74.6 | |
| 285 | 73.5 | |

Fig. 19

| collection location | matching material | biomass type |
|---|---|---|
| ○○burger | Potato waste | food waste(industrial) |

190

| collection location | material type for mixture | biomass type | pretreatment | methene gas fermentation method | fermentation temperature (℃) | fermentation days or when to stop |
|---|---|---|---|---|---|---|
| ○○biogas | rice stalk-BP | plant residue | grinding | Wet (medium temperature fermentation) | 36 | 60 |
| ○○biogas | wheat stalk-BP | plant residue | grinding | Wet (medium temperature fermentation) | 36 | 60 |
| ○○biogas | rape stalk-BP | plant residue | grinding | Wet (medium temperature fermentation) | 36 | 60 |
| ○○biogas | maize stalk-BP | plant residue | grinding | Wet (medium temperature fermentation) | 36 | 60 |
| △△former | Wheat Straw 5.0cm | plant residue | cutting | | 40 | 60 |
| △△former | Barley Straw 5.0cm | plant residue | cutting | | 40 | 60 |
| ○○biogas | cotton stalk-BP | plant residue | grinding | Wet (medium temperature fermentation) | 36 | 60 |
| △△former | Maize stalks 2.0cm | plant residue | cutting | | 40 | 60 |
| △△former | Rice Straw 5.0cm | plant residue | cutting | | 40 | 60 |
| □□oil | Used vegetable oil decanted fraction | oil residue | no | 191 | 35 | |
| TBM | Waste sludge from SMO biofuel processing | purified sludge | no | | 35 | |
| ○○burger | OWS oily waste sludge | oil residue | no | | 35 | |
| ○○mall | Canteen2 dining room2 garbage | food waste | no | | 37 | |
| ○○lunch box | FOG suspended fat, oil and grease | oil residue | no | | 35 | 30 |
| ○○lunch box | FOG settledfat, oiland grease | oil residue | no | | 35 | 30 |
| △△fish | CFW cuttle fish waste | food waste | silage | | 36.5 | about100 |
| ○○mall | Restaurant restaurant garbage | food waste | no | | 37 | |
| □□oil | dUVO diluted used vegetable oils | oil residue | no | | 35 | |
| □□farm | Animal food waste | food waste | grinding | | 37 | 45 |
| △△fish | PSFW Pacific saury fish waste | food waste | silage | | 36.5 | about100 |
| □□畜産 | fish slaughter waste | animal residue | no | | 37 | |
| ○○mall | Bakery bakery garbage | food waste | no | | 37 | |
| □□oil | Non-refine glycerin | food waste(industrial) | no | | 35 | |
| ○○food | Cooked meat | food waste | dry grinding | | 37 | 27 |
| ○○mall | Canteen1 dining room1 garbage | food waste | no | | 37 | |
| △△fish | FW:BGW silage 40:60 silage, fish waste:bread waste silage | food waste | silage | | 36.5 | about100 |
| □□farm | Vegetable food waste | food waste | grinding | | 37 | 45 |
| ○○dairy | pig fatteners 9 | animal residue | no | | 35 | 100 |
| ○○burger | Waste sludge from a fruit residue treatment plant | sludge | no | | 35 | |

EP 4 521 334 A1

Fig. 20

a   200   b   c

| VS (% Fresh material (FM)) | TS (% Fresh material (FM)) | VS/TS (%) | Ash/TS (%) | COD/VS (%) | C (%VS) | N (%VS) | H (%VS) | O (%VS) | C/N(%VS) | total carbohydrate (%VS) | lipid (%VS) | protein (%VS) | lipid/VS rate | lipid/carbohydrate rate |
|---|---|---|---|---|---|---|---|---|---|---|---|---|---|---|
| 52.13 | 57.38 | 90.84 | 9.2 | 1.27 | 45.1 | 0.8 | 6.6 | 44.6 | 76.9 | 36.15 | 11.3 | 7.15 | 0.22 | 0.3 |
| 54.03 | 57.43 | 94.07 | 5.9 | 1.46 | 45.2 | 0.7 | 6.6 | 44.7 | 118.4 | 45.3 | 11.75 | 6.9 | 0.22 | 0.3 |
| 56.23 | 57.38 | 97.99 | 2.0 | 1.22 | 45.1 | 0.7 | 6.5 | 44.8 | 137.2 | 39.15 | 10.9 | 6.45 | 0.19 | 0.3 |
| 56.28 | 56.93 | 98.85 | 1.2 | 1.2 | 44.9 | 1.1 | 6.5 | 44.3 | 48.5 | 51.0 | 12.15 | 7.55 | 0.22 | 0.2 |
| 50.96 | 53.23 | 95.74 | 4.3 | 1.43 | 46.9 | 3.1 | 6.5 | 26.2 | 30.9 | 43.15 | 11.4 | 6.9 | 0.22 | 0.3 |
| 52.20 | 55.18 | 94.59 | 5.4 | 1.12 | 46.3 | 3.0 | 6.4 | 26.2 | 30.9 | 45.5 | 10.9 | 8.5 | 0.21 | 0.2 |
| 57.93 | 58.63 | 98.80 | 1.2 | 1.2 | 45.9 | 0.8 | 6.5 | 45.3 | 101.2 | 35.5 | 14.5 | 7.3 | 0.25 | 0.4 |
| 48.55 | 53.78 | 90.28 | 9.7 | 1.34 | 46.3 | 3.0 | 6.4 | 26.2 | 30.9 | 49.6 | 11.1 | 9.3 | 0.23 | 0.2 |
| 22.71 | 24.28 | 93.54 | 6.5 | 1.44 | 45.9 | 3.0 | 6.4 | 26.2 | 30.8 | 38.35 | 11.5 | 8.2 | 0.51 | 0.3 |
| 44.42 | 46.11 | 96.33 | 3.7 | 1.28 | 58.7 | 0.9 | 8.5 | 31.8 | 86.2 | 30 | 60 | 5.25 | 1.35 | 2.0 |
| 52.77 | 55.18 | 95.64 | 4.4 | 1.27 | 56.0 | 1.2 | 8.2 | 34.5 | 52.8 | 30 | 30.5 | 5.25 | 0.58 | 1.0 |
| 18.54 | 22.05 | 84.08 | 15.9 | 1.28 | 56.2 | 2.5 | 8.2 | 32.7 | 35.1 | 30 | 60.5 | 5.25 | 3.26 | 2.0 |
| 10.96 | 11.43 | 95.89 | 4.1 | 1.2 | 52.03 | 2.06 | 7.75 | 38.2 | 36.1 | 47.12 | 21.225 | 13.79 | 1.94 | 0.5 |
| 21.01 | 23.29 | 90.21 | 9.8 | 1.90 | 56.2 | 2.5 | 8.2 | 32.7 | 35.1 | 30.0 | 60.5 | 5.3 | 2.88 | 2.0 |
| 15.16 | 16.35 | 92.69 | 7.3 | 1.88 | 56.0 | 1.2 | 8.2 | 34.5 | 52.8 | 30.0 | 60.5 | 5.3 | 3.99 | 2.0 |
| 27.62 | 29.18 | 94.65 | 5.3 | 1.156 | 51.5 | 4.5 | 7.2 | 36.4 | 29.7 | 30 | 35.3 | 26.7 | 1.28 | 1.2 |
| 11.25 | 11.78 | 95.50 | 4.5 | 1.2 | 48.88 | 2.34 | 7.06 | 41.7 | 33.6 | 51.235 | 19.835 | 15.48 | 1.76 | 0.4 |
| 45.34 | 47.63 | 95.19 | 4.8 | 1.32 | 53.4 | 1.2 | 6.1 | 39.1 | 50.3 | 30 | 60.5 | 5.25 | 1.33 | 2.0 |
| 26.75 | 30.43 | 87.89 | 12.1 | 1.13 | 51.3 | 2.6 | 7.3 | 38.8 | 33.3 | 56.0 | 23.0 | 11.3 | 0.86 | 0.4 |
| 23.34 | 25.63 | 91.07 | 8.9 | 1.127 | 48.8 | 5.8 | 7.1 | 38.0 | 28.6 | 30 | 31.2 | 28.1 | 1.34 | 1.0 |
| 38.77 | 39.93 | 97.08 | 2.9 | 1.3 | 51.5 | 4.5 | 7.2 | 36.4 | 29.7 | 30 | 54.6 | 12.9 | 1.41 | 0.0 |
| 11.68 | 12.13 | 96.29 | 3.7 | 1.2 | 49.71 | 1.47 | 7.44 | 41.4 | 41.7 | 47.07 | 23.015 | 10.11 | 1.97 | 0.5 |
| 34.33 | 37.265 | 92.12 | 7.9 | 1.21 | 48.4 | 0.6 | 8.2 | 42.7 | 418.1 | 30 | 60.5 | 5.25 | 1.76 | 2.0 |
| 35.23 | 36.43 | 96.71 | 3.3 | 1.42 | 50.0 | 6.0 | 7.5 | 29.2 | 28.6 | 30 | 18.45 | 46 | 0.52 | 0.6 |
| 11.33 | 11.83 | 95.73 | 4.3 | 1.3 | 45.06 | 1.92 | 6.63 | 46.4 | 34.6 | 43.95 | 16.295 | 11.805 | 1.44 | 0.4 |
| 21.87 | 23.08 | 94.77 | 5.2 | 1.145 | 48.8 | 5.8 | 7.1 | 38.0 | 28.6 | 30 | 19.8 | 21.5 | 0.90 | 0.7 |
| 20.69 | 21.93 | 94.32 | 5.7 | 1.28 | 44.41 | 1.95 | 6.40 | 47.2 | 34.2 | 56.5 | 17.5 | 7.8 | 0.85 | 0.3 |
| 19.43 | 21.6 | 89.97 | 10.0 | 1.2 | 54.6 | 2.9 | 7.7 | 34.2 | 33.0 | 54.7 | 17.0 | 18.6 | 0.88 | 0.3 |
| 12.96 | 13.755 | 94.22 | 5.8 | 1.28 | 47.5 | 2.8 | 6.5 | 42.5 | 31.7 | 36.2 | 12.95 | 7.45 | 1.00 | 0.4 |

191

d    e    f    g    <u>210</u>

| protein/carbo hydrate rate | hemicellulose (%VS) | cellulose (%VS) | lignin (%VS) | iron(mg/kg-COD) | cobalt(mg/g-COD) | nickel(mg/g-COD) | Na concentration (%VS) | theoretical methane yield ThBMP (Nml/g-VS) | calculation method of theoretical methane yield |
|---|---|---|---|---|---|---|---|---|---|
| 0.2 | | | 26.2 | 165.0 | 16.0 | 27.0 | 0.1 | 420.33 | element balance |
| 0.2 | | | 28.6 | 189.0 | 14.0 | 17.0 | 0.1 | 406.67 | element balance |
| 0.2 | | | 28.8 | 167.0 | 15.0 | 5.0 | 0.1 | 391.00 | element balance |
| 0.1 | | | 27.6 | 180.0 | 14.0 | 7.5 | 0.1 | 385.00 | element balance |
| 0.2 | 31.0 | 52.0 | 9.8 | 186.0 | 14.0 | 8.0 | 0.1 | 364.89 | nutrient-based (including fiber) |
| 0.2 | 31.8 | 49.6 | 10.2 | 178.0 | 14.0 | 5.4 | 0.2 | 363.74 | nutrient-based (including fiber) |
| 0.2 | | | 32.1 | 166.0 | 14.0 | 4.2 | 0.1 | 360.33 | element balance |
| 0.2 | 30.0 | 49.3 | 6.3 | 191.0 | 14.0 | 5.7 | 0.1 | 359.63 | nutrient-based (including fiber) |
| 0.2 | 38.2 | 36.5 | 9.0 | 181.0 | 16.0 | 4.3 | 0.1 | 339.72 | nutrient-based (including fiber) |
| 0.2 | | | 191 | 0.1 | 0 | 0 | 0 | 687.00 | element balance |
| 0.2 | | | | 0.1 | 0 | 0 | 0 | 650.67 | element balance |
| 0.2 | | | | 0.1 | 0 | 0 | 0 | 642.00 | element balance |
| 0.3 | | | | 13 | 13 | 5 | 4 | 585.67 | element balance |
| 0.2 | 0.0 | 0.0 | 0.0 | 0.1 | 0.0 | 0.0 | 0.0 | 579.54 | nutrient-based (including fiber) |
| 0.2 | 0.0 | 0.0 | 0.0 | 0.1 | 0.0 | 0.1 | 0.0 | 578.03 | nutrient-based (including fiber) |
| 0.9 | 0.0 | 0.0 | | 180 | 4 | 3 | 11 | 570.77 | nutrient-based (including fiber) |
| 0.3 | | | | 170 | 16 | 5 | 3 | 554.09 | element balance |
| 0.2 | | | | 0.1 | 0 | 0 | 0 | 545.67 | element balance |
| 0.2 | 7.0 | 2.0 | 2.0 | 1.0 | 1 | 1 | 1.6 | 525.33 | nutrient-based |
| 0.9 | 0.0 | 0.0 | | 120 | 4 | 3 | 10 | 518.96 | nutrient-based (including fiber) |
| 0.4 | 0.5 | 0.3 | 1.1 | 11.0 | 12.2 | 13.0 | 13.0 | 512.88 | nutrient-based (including fiber) |
| 0.2 | | | | 5 | 5 | 4 | 4 | 504.12 | element balance |
| 0.2 | | | | 0.1 | 0 | 0 | 0 | 500.67 | element balance |
| 1.5 | 51.7 | 7.2 | | 15 | 3 | 2 | 4 | 492.55 | element balance |
| 0.3 | | | | 170 | 14 | 5 | 4 | 478.37 | element balance |
| 0.7 | 14.2 | 32.2 | | 120 | 41 | 3 | 15 | 471.76 | nutrient-based (including fiber) |
| 0.1 | 10.0 | 12.0 | 5.0 | 1.4 | 1 | 1 | 0.8 | 455.00 | nutrient-based |
| 0.3 | | | 4.3 | 120.0 | 2.0 | 2.0 | 1.0 | 447.00 | element balance |
| 0.2 | | | | 0.3 | 0 | 0 | 0 | 443.33 | element balance |

Fig. 21

EP 4 521 334 A1

Fig. 22

EP 4 521 334 A1

| type | Types of gasification promoters | Amount added (daily amount) |
|---|---|---|
| auxiliary raw material | Bio ash | 2kg /100kg-material |

220

| collection location | matching material | biomass type |
|---|---|---|
| ○○burger | Potato waste | food waste(industrial) |

| collection location | material type for mixture | biomass type | pretreatment | methene gas fermentation method | fermentation temperature (°C) | fermentation days or when to stop |
|---|---|---|---|---|---|---|
| ○○biogas | rice stalk-BP | plant residue | grinding | Wet (medium temperature fermentation) | 36 | 60 |
| ○○biogas | wheat stalk-BP | plant residue | grinding | Wet (medium temperature fermentation) | 36 | 60 |
| ○○biogas | rape stalk-BP | plant residue | grinding | Wet (medium temperature fermentation) | 36 | 60 |
| ○○biogas | maize stalk-BP | plant residue | grinding | Wet (medium temperature fermentation) | 36 | 60 |
| △△former | Wheat Straw 5.0cm | plant residue | cutting | | 40 | 60 |
| △△former | Barley Straw 5.0cm | plant residue | cutting | | 40 | 60 |
| ○○biogas | cotton stalk-BP | plant residue | grinding | Wet (medium temperature fermentation) | 36 | 60 |
| △△former | Maize stalks 2.0cm | plant residue | cutting | | 40 | 60 |
| △△former | Rice Straw 5.0cm | plant residue | cutting | | 40 | 60 |
| □□oil | Used vegetable oil decanted fraction | oil residue | no | | 35 | |
| ○○burger | OWS oily waste sludge | oil residue | no | 221 | 35 | |
| ○○mall | Canteen2 dining room2 garbage | food waste | no | | 37 | |
| ○○lunch box | FOG suspended fat, oil and grease | oil residue | no | | 35 | 30 |
| ○○lunch box | FOG settledfat, oiland grease | oil residue | no | | 35 | 30 |
| △△fish | CFW cuttle fish waste | food waste | silage | | 36.5 | about100 |
| ○○mall | Restaurant restaurant garbage | food waste | no | | 37 | |
| □□oil | dUVO diluted used vegetable oils | oil residue | no | | 35 | |
| □□farm | Animal food waste | food waste | grinding | | 37 | 45 |
| △△fish | PSFW Pacific saury fish waste | food waste | silage | | 36.5 | about100 |
| □□stock raising | fish slaughter waste | animal residue | no | | 37 | |
| ○○mall | Bakery bakery garbage | food waste | no | | 37 | |
| □□oil | Non-refine glycerin | food waste(industrial) | no | | 35 | |
| ○○food | Cooked meat | food waste | dry grinding | | 37 | 27 |
| ○○mall | Canteen1 dining room1 garbage | food waste | no | | 37 | |
| △△fish | FW:BGW silage 40:60 silage, fish waste:bread waste silage | food waste | silage | | 36.5 | about100 |
| □□farm | Vegetable food waste | food waste | grinding | | 37 | 45 |

Fig. 23

EP 4 521 334 A1

| VS (% Fresh material (FM)) | TS (% Fresh material (FM)) | VS/TS (%) | Ash/TS (%) | COD/VS (%) | C (%VS) | N (%VS) | H (%VS) | O (%VS) | C/N(%VS) | total carbohydrate (%VS) | lipid (%VS) | protein (%VS) | lipid/VS rate |
|---|---|---|---|---|---|---|---|---|---|---|---|---|---|
| 69.31 | 73.94 | 93.74 | 6.3 | 1.32 | 56.1 | 1.1 | 8.3 | 33.1 | 52.8 | 19.075 | 20.7 | 5.075 | 0.30 |
| 70.26 | 73.965 | 94.99 | 5.0 | 1.41 | 56.1 | 1.0 | 8.3 | 33.2 | 55.5 | 23.6 | 20.925 | 6.95 | 0.30 |
| 71.36 | 73.94 | 96.51 | 3.5 | 1.29 | 56.0 | 1.0 | 8.3 | 33.2 | 56.0 | 20.575 | 20.5 | 5.725 | 0.29 |
| 71.39 | 73.715 | 96.84 | 3.2 | 1.3 | 55.9 | 1.2 | 8.3 | 33.0 | 46.7 | 26.5 | 21.125 | 6.75 | 0.30 |
| 68.73 | 71.865 | 95.64 | 4.4 | 1.40 | 56.9 | 2.2 | 8.3 | 23.9 | 25.9 | 22.575 | 20.7 | 6.0 | 0.30 |
| 69.35 | 72.84 | 95.21 | 4.8 | 1.24 | 56.6 | 2.2 | 8.2 | 23.9 | 26.1 | 23.7 | 20.5 | 5.9 | 0.30 |
| 72.21 | 74.565 | 96.85 | 3.2 | 1.3 | 56.5 | 1.0 | 8.3 | 33.5 | 54.9 | 18.75 | 22.3 | 5.15 | 0.31 |
| 67.53 | 72.14 | 93.60 | 6.4 | 1.35 | 56.6 | 2.2 | 8.2 | 23.9 | 26.1 | 25.8 | 20.6 | 6.1 | 0.31 |
| 54.61 | 57.39 | 95.15 | 4.9 | 1.40 | 56.4 | 2.2 | 8.2 | 23.9 | 26.0 | 20.175 | 20.8 | 5.6 | 0.38 |
| 65.46 | 68.305 | 95.83 | 4.2 | 1.32 | 62.9 | 1.1 | 9.2 | 26.7 | 57.1 | 16 | 45.05 | 4.125 | 0.69 |
| 52.52 | 56.275 | 93.33 | 6.7 | 1.32 | 61.6 | 1.9 | 9.1 | 27.1 | 32.7 | 16 | 45.3 | 4.125 | 0.86 |
| 48.73 | 50.965 | 95.61 | 4.4 | 1.3 | 59.51 | 1.68 | 8.87 | 29.9 | 35.4 | 24.56 | 25.6625 | 8.395 | 0.53 |
| 53.76 | 56.895 | 94.48 | 5.5 | 1.63 | 61.6 | 1.9 | 9.1 | 27.1 | 32.7 | 16.0 | 45.3 | 4.1 | 0.84 |
| 50.83 | 53.425 | 95.14 | 4.9 | 1.62 | 61.5 | 1.3 | 9.1 | 28.1 | 48.7 | 16.0 | 45.3 | 4.1 | 0.89 |
| 57.06 | 59.84 | 95.35 | 4.6 | 1.258 | 59.2 | 2.9 | 8.6 | 29.0 | 20.3 | 16 | 32.7 | 14.8 | 0.57 |
| 48.88 | 51.14 | 95.57 | 4.4 | 1.3 | 57.94 | 1.82 | 8.53 | 31.7 | 31.8 | 26.6175 | 24.9675 | 9.24 | 0.51 |
| 65.92 | 69.065 | 95.45 | 4.6 | 1.34 | 60.2 | 1.3 | 8.1 | 30.4 | 47.5 | 16 | 45.3 | 4.125 | 0.69 |
| 56.62 | 60.465 | 93.65 | 6.4 | 1.25 | 59.2 | 1.9 | 8.6 | 30.2 | 30.3 | 29.0 | 26.6 | 7.1 | 0.47 |
| 54.92 | 58.065 | 94.58 | 5.4 | 1.243 | 57.9 | 3.6 | 8.5 | 29.8 | 16.3 | 16 | 30.6 | 15.6 | 0.56 |
| 62.63 | 65.215 | 96.04 | 4.0 | 1.3 | 59.2 | 2.9 | 8.6 | 29.0 | 20.3 | 16 | 42.4 | 8.0 | 0.68 |
| 49.09 | 51.315 | 95.66 | 4.3 | 1.3 | 58.35 | 1.38 | 8.72 | 31.5 | 42.2 | 24.535 | 26.5575 | 6.555 | 0.54 |
| 60.42 | 63.8825 | 94.57 | 5.4 | 1.29 | 57.7 | 1.0 | 9.1 | 32.2 | 59.7 | 16 | 45.3 | 4.125 | 0.75 |
| 60.87 | 63.465 | 95.90 | 4.1 | 1.39 | 58.5 | 3.7 | 8.7 | 25.4 | 16.0 | 16 | 24.275 | 24.5 | 0.40 |
| 48.91 | 51.165 | 95.60 | 4.4 | 1.3 | 56.03 | 1.61 | 8.32 | 34.0 | 34.8 | 22.975 | 23.1975 | 7.4025 | 0.47 |
| 54.19 | 56.79 | 95.42 | 4.6 | 1.253 | 57.9 | 3.6 | 8.5 | 29.8 | 16.3 | 16 | 24.9 | 12.3 | 0.46 |
| 53.59 | 56.215 | 95.33 | 4.7 | 1.32 | 55.71 | 1.62 | 8.20 | 34.4 | 34.3 | 29.3 | 23.8 | 5.4 | 0.44 |

a    230    b    c

221

Fig. 24

240

| lipid/carbohydrate rate | protein/carbohydrate rate | hemicellulose (%VS) | cellulose (%VS) | lignin (%VS) | iron (mg/kg-COD) | cobalt (mg/g-COD) | nickel (mg/g-COD) | Na concentration (%VS) | theoretical methane yield ThBMP (Nml/g-VS) | calculation method of theoretical methane yield |
|---|---|---|---|---|---|---|---|---|---|---|
| 1.09 | 0.27 | | | 26.2 | 187.0 | 18.0 | 5.5 | 0.1 | 622.17 | element balance |
| 0.88 | 0.29 | | | 28.6 | 172.0 | 15.0 | 8.2 | 0.1 | 615.33 | element balance |
| 1.00 | 0.28 | | | 28.8 | 168.0 | 14.4 | 6.3 | 0.1 | 607.50 | element balance |
| 0.80 | 0.25 | | | 27.6 | 179.0 | 16.7 | 5.6 | 0.1 | 604.50 | element balance |
| 0.92 | 0.26 | 31.0 | 52.0 | 9.8 | 174.0 | 19.0 | 7.4 | 0.1 | 594.44 | nutrient-based (including fiber) |
| 0.86 | 0.25 | 31.8 | 49.6 | 10.2 | 167.0 | 15.2 | 9.1 | 0.2 | 593.87 | nutrient-based (including fiber) |
| 1.19 | 0.27 | | | 32.1 | 169.0 | 18.1 | 7.0 | 0.1 | 592.17 | element balance |
| 0.80 | 0.24 | 30.0 | 49.3 | 6.3 | 171.0 | 14.2 | 5.0 | 0.1 | 591.81 | nutrient-based (including fiber) |
| 1.03 | 0.28 | 38.2 | 36.5 | 9.0 | 0.7 | 1.2 | 1.2 | 0.1 | 581.86 | nutrient-based (including fiber) |
| 2.82 | 0.26 | | | | 0.1 | 0 | 0.0 | 0 | 755.50 | element balance |
| 2.83 | 0.26 | | | | 0.1 | 0 | 0.0 | 0 | 733.00 | element balance |
| 1.04 | 0.34 | | | | 13 | 13 | 5.0 | 4 | 704.83 | element balance |
| 2.83 | 0.26 | 0.0 | 0.0 | 0.0 | 0.1 | 0.0 | 0.0 | 0.0 | 701.77 | nutrient-based (including fiber) |
| 2.83 | 0.26 | 0.0 | 0.0 | 0.0 | 0.1 | 0.0 | 0.1 | 0.0 | 701.02 | nutrient-based (including fiber) |
| 2.05 | 0.93 | 0.0 | 0.0 | | 180 | 4 | 3.0 | 11 | 697.39 | nutrient-based (including fiber) |
| 0.94 | 0.35 | | | | 170 | 16 | 5.0 | 3 | 689.05 | element balance |
| 2.83 | 0.26 | | | | 0.1 | 0 | 0.0 | 0 | 684.83 | element balance |
| 0.92 | 0.25 | 7.0 | 2.0 | 2.0 | 1.0 | 1 | 1.0 | 1.6 | 674.67 | nutrient-based |
| 1.92 | 0.97 | 0.0 | 0.0 | | 120 | 4 | 3.0 | 10 | 671.48 | nutrient-based (including fiber) |
| 0.00 | 0.50 | 0.5 | 0.3 | 1.1 | 11.0 | 12.2 | 13.0 | 13.0 | 668.44 | nutrient-based (including fiber) |
| 1.08 | 0.27 | | | | 5 | 5 | 4.0 | 4 | 664.06 | element balance |
| 2.83 | 0.26 | | | | 0.1 | 0 | 0.0 | 0 | 662.33 | element balance |
| 1.52 | 1.53 | 51.7 | 7.2 | | 15 | 3 | 2.0 | 4 | 658.28 | element balance |
| 1.01 | 0.32 | | | | 170 | 14 | 5.0 | 4 | 651.19 | element balance |
| 1.56 | 0.77 | 14.2 | 32.2 | | 120 | 41 | 3.0 | 15 | 647.88 | nutrient-based (including fiber) |
| 0.81 | 0.18 | 10.0 | 12.0 | 5.0 | 1.4 | 1 | 1.0 | 0.8 | 639.50 | nutrient-based |

d  e  f  g

221

Fig. 25

Fig. 25 diagram:

- Used waste cooking oil → Feed for poultry farming, etc. (Waste with established resource circulation)

Dashed box containing:
- Oil → Waste oils → Biofuel conversion
  - ·For power generation
  - ·Heavy oil substitute
- 1003 (pointing to Oil)

Business related food waste

<Sources of waste>
food chain
food factory
Commercial facility
Supermarket
convenience store
School/Hospital
Employee cafeteria etc.

Residual sludge — 1001
·precipitation residue

Food loss — 1002
·Unsold items
·Expired
·Remaining materials
·others

Purified sludge

Biogasification — 1004
·Methane power generation
·Clean hydrogen

EP 4 521 334 A1

Fig. 26

1100

**60% of stores are concentrated in the top 10 prefectures**

Restaurant

Supermarket, convenience store

Commercial facility

Shopping street

**Business food waste including grease traps (GT) ⇒ Urban biomass**

TBM prepared from the National Federation of Oils and Fats Business Cooperative Associations, Ministry of Agriculture, Forestry and Fisheries' "2019 Recycling Rate of Food Circulating Resources" and Ministry of the Environment's "Survey Report on the Actual Status of Industrial Waste Treatment"

|  | Production amount | Recycle rate | |
|---|---|---|---|
| Waste cooking oil | 180,000 tons | **90%** | Recycled into material materials, industrial materials, BDF, etc. |
| Grease trap / Waste oil | 300,000 tons | **1%** | Developing biofuel technology at TBM Service provided by major companies mainly in the Tokyo metropolitan area |
| Residual sludge | 600,000 tons | **10%** | Mainly incinerated or landfilled as industrial waste sludge Reuse of composting and methanation combined is less than 10% |
| Food loss | 3,090,000 tons | **20%** | High-quality food residue can be turned into feed or compost On the other hand, most low-quality food residue is incinerated. |

Approximately 3 million tons of residual sludge + food loss are unused ⇒ Potential of 600 million KWh if methane power is generated

## INTERNATIONAL SEARCH REPORT

| International application No. |
| --- |
| **PCT/JP2023/004304** |

**A. CLASSIFICATION OF SUBJECT MATTER**

*G06Q 50/10*(2012.01)i
FI: G06Q50/10

According to International Patent Classification (IPC) or to both national classification and IPC

**B. FIELDS SEARCHED**

Minimum documentation searched (classification system followed by classification symbols)
G06Q50/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| P, X | WO 2022/230035 A1 (TBM CORP.) 03 November 2022 (2022-11-03) paragraphs [0012]-[0073] | 1-15 |
| A | JP 2009-223386 A (TOSHIBA CORP.) 01 October 2009 (2009-10-01) entire text, all drawings | 1-15 |
| A | JP 2019-131631 A (MITSUBISHI HITACHI POWER SYSTEMS, LTD.) 08 August 2019 (2019-08-08) entire text, all drawings | 1-15 |
| A | WO 2019/167743 A1 (MITSUBISHI HEAVY INDUSTRIES, LTD.) 06 September 2019 (2019-09-06) entire text, all drawings | 1-15 |

☐ Further documents are listed in the continuation of Box C.     ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **30 March 2023** | **11 April 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/004304**

| Patent document cited in search report | | | Publication date (day/month/year) | Patent family member(s) | | | Publication date (day/month/year) |
|---|---|---|---|---|---|---|---|
| WO | 2022/230035 | A1 | 03 November 2022 | (Family: none) | | | |
| JP | 2009-223386 | A | 01 October 2009 | (Family: none) | | | |
| JP | 2019-131631 | A | 08 August 2019 | (Family: none) | | | |
| WO | 2019/167743 | A1 | 06 September 2019 | US | 2020/0378949 | A1 | |
| | | | | entire text, all drawings | | | |
| | | | | JP | 2019-147106 | A | |
| | | | | EP | 3741831 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014217804 A
- JP 5452814 B

- JP 3216173 U